# EUROPEAN PATENT SPECIFICATION

(11) **EP 2 203 160 B1**
(45) Date of publication and mention of the grant of the patent: **17.07.2013**
(21) Application number: 08799307.7
(22) Date of filing: 08.09.2008
(51) Int. Cl.: A61K 31/00, A61K 31/47, A61K 31/472, A61P 27/06

(54) **PHARMACOLOGICAL ADJUNCTIVE TREATMENT ASSOCIATED WITH GLAUCOMA FILTRATION SURGERY**
PHARMAKOLOGISCHE HILFSBEHANDLUNG IM ZUSAMMENHANG MIT GLAUKOM-FILTRATIONSCHIRURGIE
TRAITEMENT PHARMACOLOGIQUE D'APPOINT ASSOCIÉ À UNE CHIRURGIE FILTRANTE DU GLAUCOME

(30) Priority: 27.09.2007 US 975620 P
(43) Date of publication of application: 07.07.2010
(73) Proprietor: Bausch & Lomb Incorporated, Rochester, NY 14604-2701 (US)
(72) Inventor: BARTELS, Stephen, P., Pittsford NY 14534 (US)
(74) Representative: Glas, Holger
(86) International application number: PCT/US2008/075579
(87) International publication number: WO 2009/042377

(56) References cited:
- WO-A-2006/050998
- WO-A-2008/027796
- PLEYER U ET AL: "Effects of a selective glucocorticoid receptor agonist on experimental keratoplasty" GRAEFE'S ARCHIVE FOR CLINICAL AND EXPERIMENTAL OPHTHALMOLOGY 200505 DE, vol. 243, no. 5, May 2005 (2005-05), pages 450-455, XP002508371 ISSN: 0721-832X
- SCHAECKE H ET AL: "DISSOCIATED GLUCOCORTICOID RECEPTOR LIGANDS: COMPOUNDS WITH AN IMPROVED THERAPEUTIC INDEX" CURRENT OPINION IN INVESTIGATIONAL DRUGS, PHARMAPRESS, US, vol. 6, no. 5, 1 May 2005 (2005-05-01), pages 503-507, XP009069961 ISSN: 1472-4472
- LAMA P J ET AL: "Antifibrotics and wound healing in glaucoma surgery" SURVEY OF OPHTHALMOLOGY 200305 US, vol. 48, no. 3, May 2003 (2003-05), pages 314-346, XP002508372 ISSN: 0039-6257
- MINER J N ET AL: "New and improved glucocorticoid receptor ligands" EXPERT OPINION ON INVESTIGATIONAL DRUGS 200512 GB, vol. 14, no. 12, December 2005 (2005-12), pages 1527-1545, XP002508373 ISSN: 1354-3784
- REUTER K C ET AL: "M1682 Compound a (CpdA), a Selective Glucocorticoid Receptor Agonist (SEGRA), Positively Affects Wound Healing While Reducing NF-KappaB Activity in Intestinal Epithelial Cells" GASTROENTEROLOGY, ELSEVIER, PHILADELPHIA, PA, vol. 134, no. 4, 1 April 2008 (2008-04-01), pages A-396, XP023433649 ISSN: 0016-5085 [retrieved on 2008-04-01]
- BETAGERI R ET AL: "Trifluoromethyl group as a pharmacophore: Effect of replacing a CF3 group on binding and agonist activity of a glucocorticoid receptor ligand", BIOORGANIC & MEDICINAL CHEMISTRY LETTERS, PERGAMON, ELSEVIER SCIENCE, GB, vol. 15, no. 21, 1 November 2005 (2005-11-01), pages 4761-4769, XP025313923, ISSN: 0960-894X, DOI: 10.1016/J.BMCL.2005.07.025 [retrieved on 2005-11-01]

## Description

### BACKGROUND

The present invention relates to compositions and methods for pharmacological adjunctive treatment associated with glaucoma filtration surgery. In particular, the present invention relates to such compositions comprising dissociated glucocorticoid receptor agonists ("DIGRAs") and such methods for reducing the risk of poorly functioning filtering bleb following glaucoma filtration surgery.

Glaucoma is a group of diseases that are characterized by the death of retinal ganglion cells ("RGCs"), specific visual field loss, and optic nerve atrophy. Glaucoma is the second leading cause of blindness worldwide. Although there are many individuals with high intraocular pressure ("IOP") who do not have glaucomatous loss of vision, an IOP that is high compared to the population mean is a risk factor for the development of glaucoma. Conversely, there are glaucoma patients with normal IOP (normal-tension glaucoma). Lowering IOP via pharmaceuticals or surgery is currently the mainstay of glaucoma treatment. Even in normal-tension glaucoma patients, it can significantly delay glaucomatous changes.

In a normal subject's eye, a fluid, known as the aqueous humor, circulates freely through the anterior chamber of the eye. This fluid, which is continuously produced by the eye's ciliary body, is drained from the eye, through the trabecular meshwork, back into the bloodstream. When the fluid drains properly, an appropriate fluid pressure is maintained in the anterior chamber, maintaining the shape of the cornea. Elevated IOP develops when the filtration mechanism of the trabecular meshwork is no longer adequate, leading to an increase in fluid within the anterior chamber. This increase in fluid, in turn, leads to an increase in the IOP. It is this increase in IOP that subsequently causes pressure on the optic nerve at the level of the optic nerve head. This causes damage to the optic nerve, which, in turn, may lead to blindness.

Presently, glaucoma is not curable by any available treatment method. However, a variety of different treatment options are available to control, and, perhaps, to slow, the progression of the disease. Possible treatment options include pharmaceutical therapy, laser eye surgery, and/or conventional surgical methods. Although many different surgical techniques have been employed in the past, glaucoma filtration surgery (or also known as filtering surgery or trabeculectomy), is principally performed today for the surgical (i.e., non-laser) management of glaucoma.

Filtration surgery lowers the IOP by creating a fistula between the anterior chamber and the subconjunctival space, creating a filtering bleb. The aqueous humor that percolates through the bleb can then either be absorbed through veins or conjunctival lymphatics or, in some cases where the conjunctiva is thin, pass directly into the tear film. A major determinant of glaucoma filtration surgery ("GFS") success is the conjunctival wound healing response, with excessive post-operative scarring leading to filtration failure.

The filtration surgery technique is designed to provide an alternative pathway for the aqueous humor fluid to leave the eye. More specifically, during filtration surgery the filtering or conjunctival bleb is created which serves as an auxiliary "drain" on the outside of the eyeball, and which has direct communication to the inside of the eyeball. This alternate pathway has a lower resistance to fluid outflow than that of the failing, or irreversibly obstructed, trabecular meshwork found in the glaucoma patient. The desired result of this technique is to achieve a lower IOP that is compatible with normal optic nerve function.

Because the basic early events of GFS wound healing have not been well described in humans, animal models have been used to delineate the initial stages of wound healing process. Despite a number of studies using animal models and human tissues, however, the early events of wound healing after GFS are still not clearly understood. In the context of general wound healing, observations in animal models and after GFS in humans suggest a sequence of events in GFS wound healing that occur in early bleb failure due to non- or poorly functioning bleb. Wound healing has been shown to involve an orderly series of events, including extracellular matrix ("ECM") degradation, cell migration, matrix synthesis, and tissue remodeling. After surgical trauma to the conjunctiva, episclera, and iris, blood vessels constrict, and leakage of plasma proteins (including fibrinogen, fibronectin, and plasminogen) and blood cells occurs. Fibroblasts proliferate and migrate from the wound edges after GFS and subsequently synthesize fibronectin, interstitial collagens, and glycosaminoglycans to form fibrovascular tissue, and later this tissue is remodeled to form a dense collagenous subconjunctival scar with scattered fibroblasts and blood vessels leading to wound scarring and filtration failure. Even if the surgery is initially successful, scarring may close the drainage channels at the bleb surface layers in the course of months or years.

GFS differs from most surgical procedures in that inhibition of wound healing (in the sense of excessive scarring tissue formation) is desirable to achieve surgical success. Successful filtration surgery is generally characterized by formation of a filtering bleb, which is a subconjunctival accumulation of the aqueous humor. Histopathological examinations of functioning blebs demonstrate the appearance of loosely arranged connective tissue beneath the conjunctival epithelium. Early failed blebs demonstrate abnormally thickened, dense collagenous connective tissue beneath the conjunctival epithelium. In contrast, late failing blebs are often thin, and avascular. Thus, the long-term maintenance of a well-functioning filtering bleb is critical for the success of the procedure.

Although administration of anti-scarring agents such as mitomycin-C ("MMC") and 5-fluorouracil ("5-FU"), during or after surgery, help prevent postsurgical scarring and improve glaucoma surgical outcome, they do so by causing widespread fibroblast cell death and apoptosis. A small fraction (on the order of a few percent) of the cases with postsurgical complication, manifested as late-onset bleb leakage, is associated with the use of antiscarring agents. See; e.g., D.S. Greenfield et al., Arch. Ophthalmol., Vol. 116, 443 (1998).

Glucocorticosteroids (also referred to herein as "corticosteroids" or "GCs") have been administered postoperatively, topically and/or orally, to prevent excessive scarring and have shown beneficial effects in controlling the glaucomatous condition. S.V. Araujo et al., Ophthalmol., Vol. 102, 1753 (1995). GCs are known to have antifibrotic action. A. Shukla et al., Wound Repair & Regen., Vol. 7, 133 (1999). For example, dexamethasone was shown to block TGF-β (transforming growth factor-β)-induced collagen synthesis. C. Boxall et al., Eur. Respir. J., Vol. 27, 208 (2006).

However, steroidal drugs can have side effects that threaten the overall health of the patient. Chronic administration of glucocorticoids can lead to drug-induced osteoporosis by suppressing intestinal calcium absorption and inhibiting bone formation. Other adverse side effects of chronic administration of glucocorticoids include hypertension, hyperglycemia, hyperlipidemia (increased levels of triglycerides) and hypercholesterolemia (increased levels of cholesterol) because of the effects of these drugs on the body metabolic processes.

In addition, it is known that certain glucocorticoids have a greater potential for elevating IOP than other compounds in this class. For example, it is known that prednisolone, which is a very potent ocular anti-inflammatory agent, has a greater tendency to elevate IOP than fluorometholone, which has moderate ocular anti-inflammatory activity. It is also known that the risk of IOP elevation associated with the topical ophthalmic use of glucocorticoids increases over time. In other words, the chronic (i.e., long-term) use of these agents increases the risk of significant IOP elevation. Therefore, the long-term use of GCs to improve the outcome of GFS may exacerbate the condition they are intended to treat.

Therefore, improved alternatives to current pharmacological therapy adjunctive to GFS are needed.

### SUMMARY

The present invention refers to compositions, their use and a method for manufacturing the compositions as defined in the claims.

The present invention provides, compositions being suitable for reducing the risk of non-functioning, poorly functioning, or failing glaucoma filtering bleb.

The present invention provides, compositions, and methods for ensuring a functioning filtering bleb after filtration surgery to control or prevent the progression of a glaucoma condition.

Such glaucoma condition may be selected from the group consisting of primary open-angle glaucoma, primary angle-closure glaucoma, secondary open-angle glaucoma, secondary angle-closure glaucoma, pigmentary glaucoma, neovascular glaucoma, pseudophakic glaucoma, malignant glaucoma, uveitic glaucoma, glaucoma due to peripheral anterior synechia, and combinations thereof.

The compositions may comprise at least a mimetic of a glucocorticoid for controlling or preventing the progression of such a glaucoma condition.

A composition for controlling or preventing the progression of such a glaucoma condition comprises at least a dissociated glucocorticoid receptor agonist ("DIGRA"), a pharmaceutically acceptable salt thereof, or a pharmaceutically acceptable ester thereof.

A composition of the present invention further comprises an antagonist to TGF-β. In one embodiment, such an antagonist to TGF-β comprises an antibody to TGF-β.

A composition of the present invention may comprise a topical formulation; injectable formulation; or implantable formulation, system, or device.

The present invention provides the use of a DIGRA, a pharmaceutically acceptable salt thereof, or a pharmaceutically acceptable ester thereof, for the preparation of a medicament for pharmacological adjunctive treatment associated with glaucoma filtration surgery, wherein the medicament is to be administered into a subject in need of such treatment.

Other features and advantages of the present invention will become apparent from the appended figures and the following detailed description and claims.

### BRIEF DESCRIPTION OF THE FIGURES

Figures 1A-1F show the effects of BOL-303242-X and dexamethasone on the IL-1β-stimulated production of Il-6, IL-7, TGF-α, TNF-α, VGEF, and MCP-1 in human corneal epithelium cells ("HCECs") at p < 0.05.
Figure 2 shows the effects of BOL-303242-X and dexamethasone on the IL-1β-stimulated production of G-CSF in HCECs at p < 0.05.
Figures 3A-3C show the effects of BOL-303242-X and dexamethasone on the IL-1β-stimulated production of GM-CSF, IL-8, and RANTES in HCECs at p < 0.05.

In these figures, "*" denotes comparison to control, and "**" to IL-1β.
Figure 4 shows the effect of TGF-β treatment on collagen level produced by human Tenon's fibroblasts.
Figure 5 shows the effect of TGF-β treatment on level of α-smooth muscle actin produced by human Tenon's fibroblasts.
Figure 6 shows the effect of TGF-β treatment on level of tissue transglutaminase produced by human Tenon's fibroblasts.

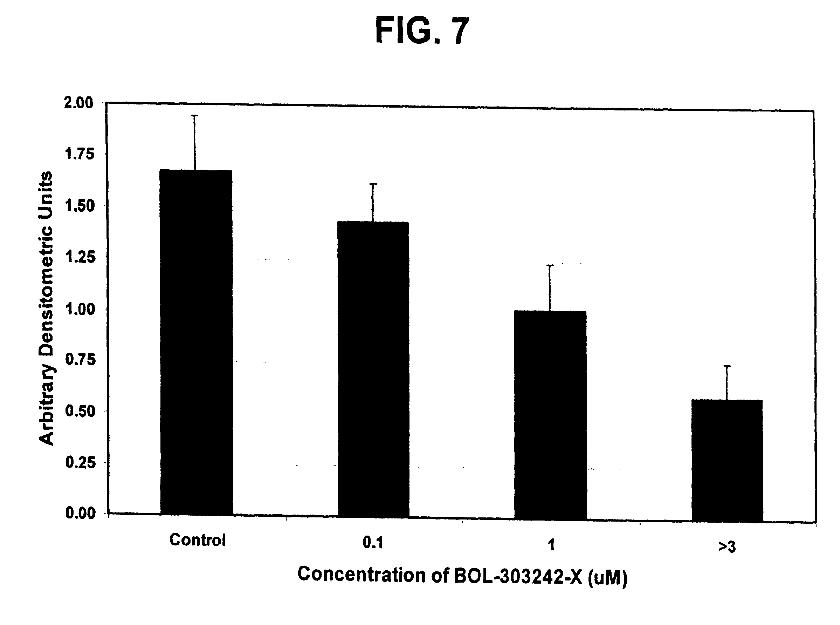

Figure 7 shows the inhibitory effect of BOL-303242-X on the expression of collagen by human Tenon's fibroblasts.

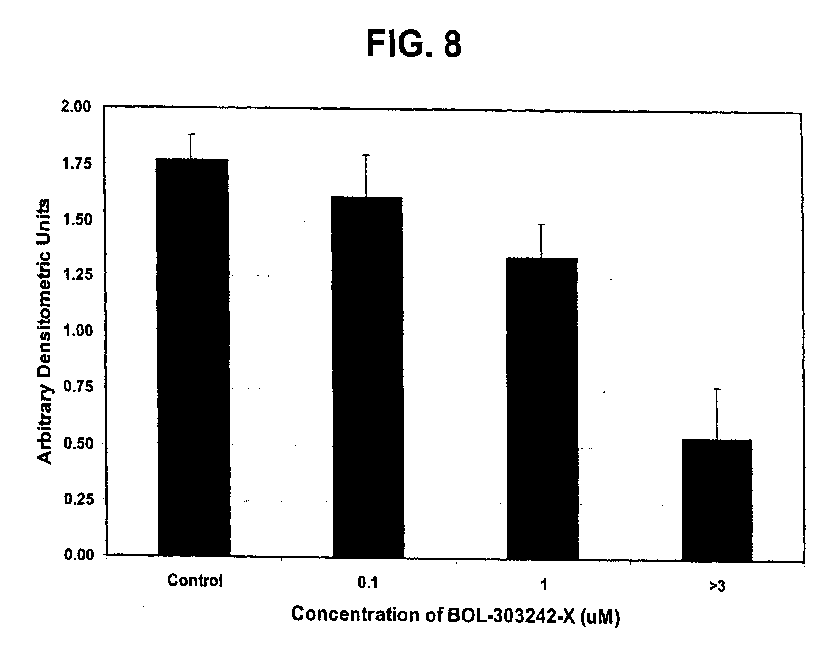

Figure 8 shows the inhibitory effect of BOL-303242-X on the expression of α-smooth muscle actin by human Tenon's fibroblasts.

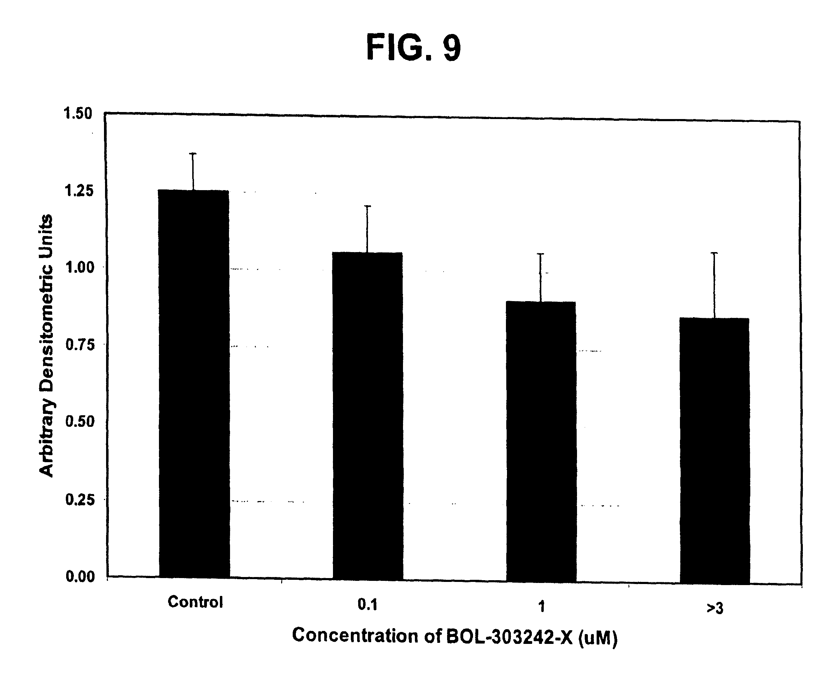

Figure 9 shows the inhibitory effect of BOL-303242-X on the expression of tisuue transglutaminase by human Tenon's fibroblasts.

### DETAILED DESCRIPTION

As used herein, the term "control" also includes reduction, amelioration, and alleviation.

As used herein, the term "antagonist to TGF-β" also includes compounds or materials that inhibit or impede the activity, transcription, expression, or signaling cascade of one or more TGF-β isoforms. The term "antagonist to TGF-β" also includes a prodrug, a pharmaceutically acceptable salt, hydrate, or ester thereof.

As used herein, a dissociated glucocorticoid receptor agonist ("DIGRA") is a compound that is capable of binding to the glucocorticoid receptor (which is a polypeptide) and, upon binding, is capable of producing differentiated levels of transrepression and transactivation of gene expression. A compound that binds to a polypeptide is sometimes herein referred to as a ligand.

As used herein, the term "alkyl" or "alkyl group" means a linear- or branched-chain saturated aliphatic hydrocarbon monovalent group, which may be unsubstituted or substituted. The group may be partially or completely substituted with halogen atoms (F, Cl, Br, or I). Non-limiting examples of alkyl groups include methyl, ethyl, n-propyl, 1-methylethyl(isopropyl), n-butyl, n-pentyl, 1,1-dimethylethyl (t-butyl), and the like. It may be abbreviated as "Alk".

As used herein, the term "alkenyl" or "alkenyl group" means a linear- or branched-chain aliphatic hydrocarbon monovalent radical containing at least one carbon-carbon double bond. This term is exemplified by groups such as ethenyl, propenyl, n-butenyl, isobutenyl, 3-methylbut-2-enyl, n-pentenyl, heptenyl, octenyl, decenyl, and the like.

As used herein, the term "alkynyl" or "alkynyl group" means a linear- or branched-chain aliphatic hydrocarbon monovalent radical containing at least one carbon-carbon triple bond. This term is exemplified by groups such as ethynyl, propynyl, n-butynyl, 2-butynyl, 3-methylbutynyl, n-pentynyl, heptynyl, octynyl, decynyl, and the like.

As used herein, the term "alkylene" or "alkylene group" means a linear- or branched-chain saturated aliphatic hydrocarbon divalent radical having the specified number of carbon atoms. This term is exemplified by groups such as methylene, ethylene, propylene, n-butylene, and the like, and may alternatively and equivalently be denoted herein as "-(alkyl)-".

The term "alkenylene" or "alkenylene group" means a linear- or branched-chain aliphatic hydrocarbon divalent radical having the specified number of carbon atoms and at least one carbon-carbon double bond. This term is exemplified by groups such as ethenylene, propenylene, n-butenylene, and the like, and may alternatively and equivalently be denoted herein as "-(alkylenyl)-".

The term "alkynylene" or "alkynylene group" means a linear- or branched-chain aliphatic hydrocarbon divalent radical containing at least one carbon-carbon triple bond. This term is exemplified by groups such as ethynylene, propynylene, n-butynylene, 2-butynylene, 3-methylbutynylene, n-pentynylene, heptynylene, octynylene, decynylene, and the like, and may alternatively and equivalently be denoted herein as "-(alkynyl)-".

As used herein, the term "aryl" or "aryl group" means an aromatic carbocyclic monovalent or divalent radical of from 5 to 14 carbon atoms having a single ring (e.g., phenyl or phenylene), multiple condensed rings (e.g., naphthyl or anthranyl), or multiple bridged rings (e.g., biphenyl). Unless otherwise specified, the aryl ring may be attached at any suitable carbon atom which results in a stable structure and, if substituted, may be substituted at any suitable carbon atom which results in a stable structure. Non-limiting examples of aryl groups include phenyl, naphthyl, anthryl, phenanthryl, indanyl, indenyl, biphenyl, and the like. It may be abbreviated as "Ar".

The term "heteroaryl" or "heteroaryl group" means a stable aromatic 5- to 14-membered, monocyclic or polycyclic monovalent or divalent radical, which may comprise one or more fused or bridged ring(s), preferably a 5- to 7-membered monocyclic or 7- to 10-membered bicyclic radical, having from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Non-limiting examples of heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrimidinyl, furanopyrazinyl, furanopyridazinyl, dihydrobenzofuranyl, dihydrofuranopyridinyl, dihydrofuranopyrimidinyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, benzoxazinyl, benzoxazinonyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl, and the like.

The term "heterocycle", "heterocycle group", "heterocyclyl", "heterocyclyl group", "heterocyclic", or "heterocyclic group" means a stable non-aromatic 5- to 14-membered monocyclic or polycyclic, monovalent or divalent, ring which may comprise one or more fused or bridged ring(s), preferably a 5- to 7-membered monocyclic or 7- to 10-membered bicyclic ring, having from one to three heteroatoms in at least one ring independently selected from nitrogen, oxygen, and sulfur, wherein any sulfur heteroatoms may optionally be oxidized and any nitrogen heteroatom may optionally be oxidized or be quaternized. As used herein, a heterocyclyl group excludes heterocycloalkyl, heterocycloalkenyl, and heterocycloalkynyl groups. Unless otherwise specified, the heterocyclyl ring may be attached at any suitable heteroatom or carbon atom which results in a stable structure and, if substituted, may be substituted at any suitable heteroatom or carbon atom which results in a stable structure. Non-limiting examples of heterocycles include pyrrolinyl, pyrrolidinyl, pyrazolinyl, pyrazolidinyl, piperidinyl, morpholinyl, thiomorpholinyl, piperazinyl, tetrahydropyranyl, tetrahydrothiopyranyl, tetrahydrofuranyl, hexahydropyrimidinyl, hexahydropyridazinyl, and the like.

The term "cycloalkyl" or "cycloalkyl group" means a stable aliphatic saturated 3- to 15-membered monocyclic or polycyclic monovalent radical consisting solely of carbon and hydrogen atoms which may comprise one or more fused or bridged ring(s), preferably a 5- to 7-membered monocyclic or 7- to 10-membered bicyclic ring. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

The term "cycloalkenyl" or "cycloalkenyl group" means a stable aliphatic 5-to 15-membered monocyclic or polycyclic monovalent radical having at least one carbon-carbon double bond and consisting solely of carbon and hydrogen atoms which may comprise one or more fused or bridged ring(s), preferably a 5- to 7-membered monocyclic or 7- to 10-membered bicyclic ring. Unless otherwise specified, the cycloalkenyl ring may be attached at any carbon atom which results in a stable structure and, if substituted, may be substituted at any suitable carbon atom which results in a stable structure. Exemplary cycloalkenyl groups include cyclopentenyl, cyclohexenyl, cycloheptenyl, cyclooctenyl, cyclononenyl, cyclodecenyl, norbornenyl, 2-methylcyclopentenyl, 2-methylcyclooctenyl, and the like.

The term "cycloalkynyl" or "cycloalkynyl group" means a stable aliphatic 8-to 15-membered monocyclic or polycyclic monovalent radical having at least one carbon-carbon triple bond and consisting solely of carbon and hydrogen atoms which may comprise one or more fused or bridged ring(s), preferably a 8- to 10-membered monocyclic or 12- to 15-membered bicyclic ring. Unless otherwise specified, the cycloalkynyl ring may be attached at any carbon atom which results in a stable structure and, if substituted, may be substituted at any suitable carbon atom which results in a stable structure. Exemplary cycloalkynyl groups include cyclooctynyl, cyclononynyl, cyclodecynyl, 2-methylcyclooctynyl, and the like.

The term "carbocycle" or "carbocyclic group" means a stable aliphatic 3- to 15-membered monocyclic or polycyclic monovalent or divalent radical consisting solely of carbon and hydrogen atoms which may comprise one or more fused or bridged rings, preferably a 5- to 7-membered monocyclic or 7- to 10-membered bicyclic ring. Unless otherwise specified, the carbocycle may be attached at any carbon atom which results in a stable structure and, if substituted, may be substituted at any suitable carbon atom which results in a stable structure. The term comprises cycloalkyl (including spiro cycloalkyl), cycloalkylene, cycloalkenyl, cycloalkenylene, cycloalkynyl, and cycloalkynylene, and the like.

The terms "heterocycloalkyl", "heterocycloalkenyl", and "heterocycloalkynyl" mean cycloalkyl, cycloalkenyl, and cycloalkynyl group, respectively, having at least a heteroatom in at least one ring, respectively.

Glucocorticoids ("GCs") are among the most potent drugs used for the treatment of many diseases, including allergic and chronic inflammatory diseases or of inflammation resulting from infections. However, as mentioned above, long-term treatment with GCs is often associated with numerous adverse side effects, such as diabetes, osteoporosis, hypertension, glaucoma, or cataract. These side effects, like other physiological manifestations, are results of aberrant expression of genes responsible for such diseases. Research in the last decade has provided important insights into the molecular basis of GC-mediated actions on the expression of GC-responsive genes. GCs exert most of their genomic effects by binding to the cytoplasmic GC receptor ("GR"). The binding of GC to GR induces the translocation of the GC-GR complex to the cell nucleus where it modulates gene transcription either by a positive (transactivation) or negative (transrepression) mode of regulation. There has been growing evidence that both beneficial and undesirable effects of GC treatment are the results of undifferentiated levels of expression of these two mechanisms; in other words, they proceed at similar levels of effectiveness.

GCs markedly affect most aspects of wound healing. When administered sufficiently early after injury, high GC levels delay the appearance of inflammatory cells, fibroblasts, the deposition of ground substance, collagen, regenerating capillaries, tissue contraction, and epithelial cell migration through effecting a reduction of TGF-β and IGF-1 (insulin-like growth factor-1) production in wounds and a reduction of collagen deposition therein. C. Wicke et al., Arch. Surg., Vol. 135, 1265 (2000). Dexamethasone can significantly inhibit human Tenon's capsule fibroblasts growth in cell cultures. These cells are a source of collagen production and deposition in the wound repair process. R. Sun et al., Ophthalmic Surg. Laser, Vol. 30, 382 (1999). It was also demonstrated that GC treatment of rat skin fibroblasts decreased the binding of GC receptor ("GCR") to the GC response element ("GRE") and of TGF-β activator protein to the TGF-β response element of the type-I procollagen genes proα1(I) and proα2(I). Thus, GCs coordinately regulate procollagen gene expression through both the GRE and TGF-β response element. N. Meisler et al., J. Cell. Biochem., Vol. 59, 376 (1995); K.R. Cutroneo et al., J. Cell. Biochem., Vol. 92, 6 (2004). Moreover, GCs can down-regulate the expression of TGF-β, TGF-β2, and TGF-β3 isoforms in hepatic stellate cells, and thus the TGF-β signaling pathway. U. Bolkenius et al., Biochem. Biophys. Res. Comm., Vol. 325, 1264 (2004). Since TGF-β isoforms are expressed and secreted from a wide variety of cells, GCs can have the same effect in cells other than hepatic stellate cells as well. The present invention provides GC mimetics, compositions comprising the same, and methods of using the same to achieve such effects of GCs without or with lower levels of undesired side effects mentioned above.

Studies have demonstrated that peptide growth factors and their receptors are key modulators of the wound healing process, participating in a well coordinated process that involves inflammation, cell proliferation, matrix deposition, and tissue remodeling. One of the most important modulators of wound repair is the family of TGF-β isoforms, which are found in large amounts in, and upon degranulation released from, platelets. Furthermore, they are produced by several cell types that are present in wounds, including activated macrophages, neutrophils, eosinophils, and fibroblasts. TGF-βs, acting in an autocrine manner, regulate fibroblast proliferation and collagen synthesis during wound repair. TGF-β1, TGF-β2, and TGF-β3 have been found in various parts of the anterior segment of the human eye. L.R. Pasquale et al., Invest. Ophthalmol. Vis. Sci., Vol. 34, No. 1, 23 (1993). TGF-β2 has been found at an increased level in the aqueous humor of patients suffering from primary open angle glaucoma ("POAG"). R.C. Tripathi et al., Exp. Eye Res., Vol. 59, 723 (1994). The present invention provides a composition comprising an antagonist to one or more TGF-βs, and a method of using such a composition to reduce the risk of having a non- or poorly functioning filtering bleb following GFS. The present invention provides a composition comprising a material that controls, reduces the levels, or reduces or inhibits the activity of one or more TGF-βs.

The present invention provides a composition comprising a DIGRA or pharmaceutically acceptable derivative thereof (such as a pharmaceutically acceptable salt, or ester thereof) that can control, reduce, or inhibit the production of at least a cytokine, chemokine or other factor that can stimulate fibroblasts and/or immune cells to produce TGF-β.

Immediately after wounding, such as a result of GFS, TGF-β1 is released in large amounts from platelets. This initial burst of active TGF-β1 serves as a chemoattractant for neutrophils, macrophages, and fibroblasts. As well as active forms, latent TGF-βs are also produced and sequestered within the wound matrix, and then released in active form over a longer period of time by proteolytic enzymes. Cells of the injured tissue synthesize and release macrophage chemoattractant protein-1 ("MCP-1"), which recruits macrophages, neutrophils, T cells, and mast cells during wound healing. These cells then produce additional TGF-β as the body attempts to accelerate wound healing. S.N. Iyer et al., J. Pharmacol. & Expt'l Therapeutics, Vol. 291, No. 1, 367 (1999). Thus, MCP-1 indirectly up-regulates matrix deposition in this process. MCP-1 knock-out mice have significantly delayed wound reepithelization and angiogenesis, and collagen synthesis. S. Werner and R. Grose, Physiol. Rev., Vol. 83, 835 (2003). Polymorphonuclear leukocytes and activated macrophages also produce an increased amount of various pro-inflammatory cytokines, such as IL-1β, IL-6, and TNF-α, which play an important role in fibroblast proliferation and matrix deposition. For example, TNF-α and IL-1β (along with TGF-β1 and TGF-β2) can stimulate the proliferation of human Tenon's capsule fibroblasts. I.A. Cunliffe et al., Br. J. Ophthalmol., Vol. 79, 590 (1995); P.O. Denk et al., Curr. Eye Res., Vol. 27, No.1, 35 (2003); S.C. Thornton et al., J. Leukocyte Biol., Vol. 47, 312 (1990). TNF-α and IL-1 are also produced by fibroblasts, and thus, further accelerate matrix deposition and produce the undesired scarring aspect of post-surgical wound healing. IL-6 has been shown to be another important factor in initiating wound healing and a chemoattractant for neutrophils. The expression of IL-6 is prolonged in adult wounds and excessive levels of this cytokine have been associated with scarring. S. Werner and R. Grose, *supra.* Granulocyte-macrophage colony-stimulating factor ("GM-CSF"), a cytokine also acting as a growth factor, can prolong the survival of eosinophils and promote the activation of eosinophils, which, in turn, produce TGF-βs. Thus, a high level of GM-CSF indirectly accelerates the matrix deposition process through increasing the availability of TGF-βs. Other cytokines or chemokines that also promote fibroblast proliferation and collagen deposition, at least in the context of airway fibrosis and remodeling, include IL-4, IL-9, IL-11, IL-13, IL-17, TGF-α, PDGF, and β-FGF (fibroblast growth factor). L.C. Borish and J.W. Steinke, J. Allergy Clin. Immunol., Vol. 111, No. 2, S460 (2003).

Thus, the present invention provides the use of a DIGRA, a pharmaceutically acceptable salt thereof; or a pharmaceutically acceptable ester thereof, for the preparation of a medicament that may reduce the risk of having a non- or poorly functioning filtering bleb following GFS, wherein the medicament is to be administered to a subject that has undergone said GFS a composition that comprises a material that reduces or suppresses the production of chemokines or cytokines that can stimulate the production of TGF-βs.

Such chemokines or cytokines may be selected from the group consisting of IL-1β, IL-6, TNF-α, MCP-1, GM-CSF, and combinations thereof.

The present invention provides the use of a DIGRA, a phamaceutically acceptable salt thereof, or a pharmaceutically acceptable ester thereof, for the preparation of a medicament that may reduce the risk of having a non- or poorly functioning filtering bleb following GFS, wherein the medicament is to be administered to a subject that has undergone said GFS a composition that comprises a material that reduces or suppresses the production of chemokines or cytokines that can promote fibrosis or collagen deposition, said chemokines or cytokines being selected from the group consisting of IL-1β, IL-4, IL-6, IL-9, IL-11, IL-13, IL-17, TNF-α, TGF-α, MCP-1, GM-CSF, PDGF (platelet derived growth factor), β-FGF, CTGF (connective tissue growth factor), and combinations thereof.

The present invention may provide the risk of non-functioning, poorly functioning, or failing glaucoma filtering bleb. As used herein, the term "non-functioning, poorly functioning, or failing filtering bleb" means a filtering bleb that does not provide adequate control of IOP after GFS for bringing such IOP to a normal or near normal level.

The present invention may provide compositions, and methods for ensuring a functioning filtering bleb after filtration surgery to control or prevent the progression of a glaucoma condition.

Such glaucoma condition may be selected from the group consisting of primary open-angle glaucoma, primary angle-closure glaucoma, secondary open-angle glaucoma, secondary angle-closure glaucoma, pigmentary glaucoma, neovascular glaucoma, pseudophakic glaucoma, malignant glaucoma, uveitic glaucoma, glaucoma due to peripheral anterior synechia, and combinations thereof.

The compositions may comprise at least a mimetic of a glucocorticoid for controlling or preventing the progression of such a glaucoma condition. As used herein, a mimetic of a glucocorticoid is or comprises a compound that exhibits or produces a beneficial physiological effect similar to a glucocorticoid.

A composition for controlling or preventing the progression of such a glaucoma condition comprises at least a dissociated glucocorticoid receptor agonist ("DIGRA"), a pharmaceutically acceptable salt thereof, or a pharmaceutically acceptable ester thereof.

A composition of the present invention further comprises an antagonist to TGF-β. In one embodiment, such an antagonist to TGF-β comprises an antibody to TGF-β.

Non-limiting examples of antibodies to TGF-β include the compounds disclosed in M.F. Cordeiro, Clin. Sci., Vol. 104, 181 (2003) or A.L. Meade et al., Invest. Ophthalmol. Vis. Sci., Vol. 44, 3394 (2003). Other antibodies to TGF-β are available, for example, from Abcam, Inc., Cambridge, Massachusetts.

Such an antibody to TGF-β is produced by the well-known recombinant technique.

In another aspect, such an antibody comprises a humanized antibody. Such a humanized antibody comprises Fv domains of murine anti-TGF-β monoclonal antibody and Fc domains of a human immunoglobulin molecule. Such a humanized antibody comprises CDRs (complementary determining regions) from the heavy- and light-chain variable domains of murine monoclonal anti-TGF-β antibody grafted into the appropriate framework regions of human variable domains and Fc domains of a human immunoglobulin molecule. Processes for humanization of non-human antibodies are known, such as that disclosed by S.L. Morrison et al., PNAS, Vol. 81, 6851 (1984). In addition, human immunoglobulin transgenic mice provide an alternative technique for generation of monoclonal antibodies that are well tolerated in human. See; e.g., D.M. Fishwild et al., Nat. Biotech., Vol. 14, 845 (1996). Still another method for obtaining humanized murine monoclonal antibodies is disclosed in U.S. Patent 7,087,409.

The antagonist to at least one TGF-β isoform may comprise a soluble extracellular domain of a TGF receptor type I or II ("sTGFRI" or "sTGFRII"). Such sTGFRI or sTGFRII can bind to TGF-β isoforms and prevent them from initiating a TGF-β signaling cascade that upregulates the expression of gene under its control, such as the proα1(I) and proα2(I) genes.

The antagonist to at least one TGF-β isoform may comprise a compound known as SB-431542, which is a TGF-β1 inhibitor. SB-431542 has a formula shown below and is available from Sigma Aldrich.

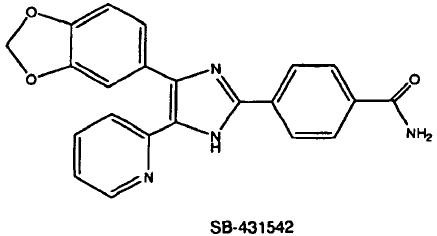

The antagonist to at least one TGF-β isoform may comprise an anti-TGF-β2 antisense oligonucleotide known as AP 12009, which was developed by Antisense Pharma GmBH, Regensburg, Germany.

The antagonist to at least one TGF-β isoform may comprise prolactin. See European Patent Application EP 0921809. Prolactin also has been shown to inhibit the chemotaxis of fibroblasts. Such inhibition would be advantageous in preventing excessive tissue scarring after GFS.

A composition of the present invention may comprise: (a) a DIGRA, a pharmaceutically acceptable salt thereof, or a pharmaceutically acceptable ester thereof; (b) an antagonist to at least one of TGF-β1 and TGF-β2; and (c) a non-steroidal anti-inflammatory drug ("NSAID") other than said DIGRA, said pharmaceutically acceptable salt thereof, and said pharmaceutically acceptable ester thereof. Non-limiting examples of such NSAIDs are disclosed herein below.

Said at least a DIGRA has Formula I. 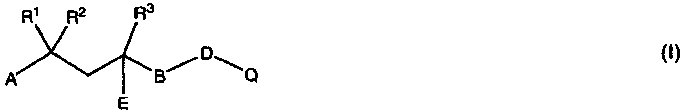 wherein A and Q are independently selected from the group consisting of unsubstituted and substituted aryl and heteroaryl groups, unsubstituted and substituted cycloalkyl and heterocycloalkyl groups, unsubstituted and substituted cycloalkenyl and heterocycloalkenyl groups, unsubstituted and substituted cycloalkynyl and heterocycloalkynyl groups, and unsubstituted and substituted heterocyclic groups; R¹ and R² are independently selected from the group consisting of hydrogen, unsubstituted C₁-C₁₅ (alternatively, C₁-C₁₀, or C₁-C₅, or C₁-C₃) linear or branched alkyl groups, substituted C₁-C₁₅ (alternatively, C₁-C₁₀, or C₁-C₅, or C₁-C₃) linear or branched alkyl groups, unsubstituted C₃-C₁₅ cycloalkyl groups, and substituted C₃-C₁₅ (alternatively, C₃-C₆, or C₃-C₅) cycloalkyl groups; R³ is selected from the group consisting of hydrogen, unsubstituted C₁-C₁₅ (alternatively, C₁-C₁₀, or C₁-C₅, or C₁-C₃) linear or branched alkyl groups, substituted C₁-C₁₅ (alternatively, C₁-C₁₀, or C₁-C₅, or C₁-C₃) linear or branched alkyl groups, unsubstituted C₃-C₁₅ (alternatively, C₃-C₆, or C₃-C₅) cycloalkyl and heterocycloalkyl groups, substituted C₃-C₁₅ (alternatively, C₃-C₆, or C₃-C₅) cycloalkyl and heterocycloalkyl groups, aryl groups, heteroaryl groups, and heterocyclylic groups; B comprises a carbonyl, amino, divalent hydrocarbon, or heterohydrocarbon group; E is hydroxy or amino group; and D is absent or comprises a carbonyl group, -NH-, or -NR'-, wherein R' comprises an unsubstituted or substituted C₁-C₁₅ (alternatively, C₁-C₁₀, or C₁-C₅, or C₁-C₃) linear or branched alkyl group; and wherein R¹ and R² together may form an unsubstituted or substituted C₃-C₁₅ cycloalkyl group.

In one embodiment, B can comprise one or more unsaturated carbon-carbon bonds.

In another embodiment, B can comprise an alkylenecarbonyl, alkyleneoxycarbonyl, alkylenecarbonyloxy, alkyleneoxycarbonylamino, alkyleneamino, alkenylenecarbonyl, alkenyleneoxycarbonyl, alkenylenecarbonyloxy, alkenyleneoxycarbonylamino, alkenyleneamino, alkynylenecarbonyl, alkynyleneoxycarbonyl, alkynylenecarbonyloxy, alkynyleneoxycarbonylamino, alkynyleneamino, arylcarbonyloxy, aryloxycarbonyl, or ureido group.

In still another embodiment, A and Q are independently selected from the group consisting of aryl and heteroaryl groups substituted with at least a halogen atom, cyano group, hydroxy group, or C₁-C₁₀ alkoxy group (alternatively, C₁-C₅ alkoxy group, or C₁-C₃ alkoxy group); R¹, R², and R³ are independently selected from the group consisting of unsubstituted and substituted C₁-C₅ alkyl groups (preferably, C₁-C₃ alkyl groups); B is a C₁-C₅ alkylene group (alternatively, C₁-C₃ alkyl groups); D is the -NH- or -NR'- group, wherein R' is a C₁-C₅ alkyl group (preferably, C₁-C₃ alkyl group); and E is the hydroxy group.

In yet another embodiment, A comprises a dihydrobenzofuranyl group substituted with a halogen atom; Q comprises a quinolinyl or isoquinolinyl group substituted with a C₁-C₁₀ alkyl group; R¹ and R² are independently selected from the group consisting of unsubstituted and substituted C₁-C₅ alkyl groups (preferably, C₁-C₃ alkyl groups); B is a C₁-C₃ alkylene group; D is the -NH- group; E is the hydroxy group; and R³ comprises a completely halogenated C₁-C₁₀ alkyl group (preferably, completely halogenated C₁-C₅ alkyl group; more preferably, completely halogenated C₁-C₃ alkyl group).

In still another embodiment, A comprises a dihydrobenzofuranyl group substituted with a fluorine atom; Q comprises a quinolinyl or isoquinolinyl group substituted with a methyl group; R¹ and R² are independently selected from the group consisting of unsubstituted and substituted C₁-C₅ alkyl groups; B is a C₁-C₃ alkylene group; D is the -NH- group; E is the hydroxy group; and R³ comprises a trifluoromethyl group.

In a further embodiment, said at least a DIGRA has Formula II or III. 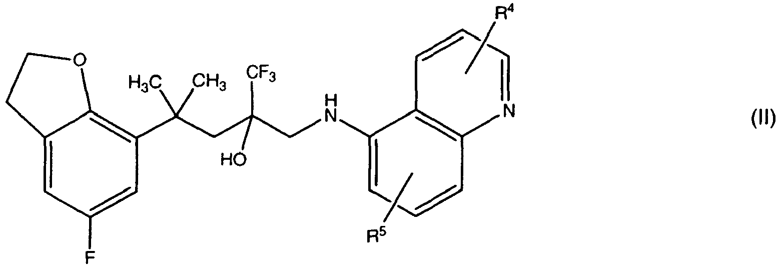 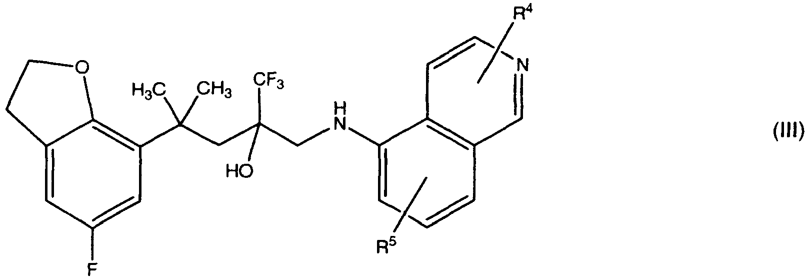 wherein R⁴ and R⁵ are independently selected from the group consisting of hydrogen, halogen, cyano, hydroxy, C₁-C₁₀ (alternatively, C₁-C₅ or C₁-C₃) alkoxy groups, unsubstituted C₁-C₁₀ (alternatively, C₁-C₅ or C₁-C₃) linear or branched alkyl groups, substituted C₁-C₁₀ (alternatively, C₁-C₅ or C₁-C₃) linear or branched alkyl groups, unsubstituted C₃-C₁₀ (alternatively, C₃-C₆ or C₃-C₅) cyclic alkyl groups, and substituted C₃-C₁₀ (alternatively, C₃-C₆ or C₃-C₅) cyclic alkyl groups.

In still another embodiment, said at least a DIGRA has Formula IV.

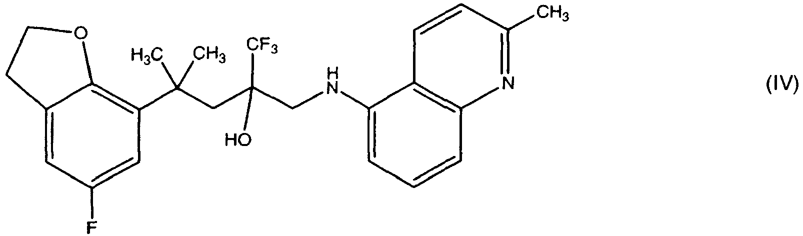

Methods for preparing compounds of Formula I, II, III, or IV are disclosed, for example, in U.S. Patents 6,897,224; 6,903,215; 6,960,581, Still other methods for preparing such compounds also can be found in U.S. Patent Application Publication 2006/0116396, or PCT Patent Application WO 2006/050998 A1.

Non-limiting examples of compounds having Formula I include 5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-1-methylisoquinoline, 5-[4-(5-fluoro-2,3-dihydrobenzofuran-7-yl)-2-hydroxy-4-methyl-2-trifluoromethyl-pentylamino]isoquinol-1(2H)-one, 5-[4-(5-fluoro-2,3-dihydrobenzofuran-7-yl)-2-hydroxy-4-methyl-2-trifluoromethyl-pentylamino]-2,6-dimethylquinoline, 5-[4-(5-fluoro-2,3-dihydrobenzofuran-7-yl)-2-hydroxy-4-methyl-2-trifluoromethyl-pentylamino]-6-chloro-2-methylquinoline, 5-[4-(5-fluoro-2,3-dihydrobenzofuran-7-yl)-2-hydroxy-4-methyl-2-trifluoromethyl-pentylamino]isoquinoline, 5-[4-(5-fluoro-2,3-dihydrobenzofuran-7-yl)-2-hydroxy-4-methyl-2-trifluoromethyl-pentylamino]quinoline, 5-[4-(2,3-dihydro-5-fluoro-7-benzofuranyl)-2-hydroxy-4-methyl-2-trifluoromethyl-pentylamino]quinolin-2[1H]-one, 6-fluro-5-[4-(5-fluoro-2,3-dihydrobenzofuran-7-yl)-2-hydroxy-4-methyl-2-trifluoromethyl-pentylamino]-2-methylquinoline, 8-fluoro-,5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-2-methylisoquinol-1-[2h]-one, and enantiomers thereof.

In yet another embodiment, said at least a DIGRA has Formula I, wherein
(a) A is an aryl group optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl;
(c) R³ is the trifluoromethyl group;
(d) B is C₁-C₅ alkyl, C₂-C₅ alkenyl, or C₂-C₅ alkynyl, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q is an azaindolyl group optionally independently substituted with one to three substituent groups, wherein each substituent group of Q is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, halogen, hydroxy, oxo, cyano, amino, and trifluoromethyl.

Non-limiting examples of these compounds include 1,1,1-trifluoro-4-(5-fluoro-2-methoxyphenyl)-4-methyl-2-(1H-pyrrolo[2,3-c] pyridin-2-ylmethyl)pentan-2-ol; 1,1,1-trifluoro-4-(5-fluoro-2-methoxyphenyl)-4-methyl-2-(1H-pyrrolo[3,2-c] pyridin-2-ylmethyl)pentan-2-ol; 1,1,1-trifluoro-4-methyl-4-phenyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[2,3-c] pyridin-2-ylmethyl)pentan-2-ol; 1,1,1-trifluoro-4-methyl-4-phenyl-2-(1H-pyrrolo[3,2-c]pyridin-2-ylmethyl)pentan-2-ol; 1,1,1-trifluoro-4-(4-fluoro-2-methoxyphenyl)-4-methyl-2-(1H-pyrrolo[3,2-c] pyridin-2-ylmethyl)pentan-2-ol; 5-fluoro-2-[4,4,4-trifluoro-3-hydroxy-1,1-dimethyl-3-(1H-pyrrolo[2,3-c]pyridin-2-ylmethyl)butyl]phenol; 4-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methoxyphenyl)-4-methyl-2-(1H-pyrrolo[3,2-c]pyridin-2-ylmethyl)pentan-2-ol; 1,1,1-trifluoro-4-(5-fluoro-2-methoxyphenyl)-4-methyl-2-(3-methyl-1H-pyrrolo[2,3-c]pyridin-2-ylmethyl)pentan-2-ol; and 4-fluoro-2-[4,4,4-trifluoro-3-hydroxy-1,1-dimethyl-3-(1H-pyrrolo[2,3-c]pyridin-2-ylmethyl)butyl]phenol.

In still another embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl, or R¹ and R² together with the carbon atom they are commonly attached to form a C₃-C₈ spiro cycloalkyl ring;
(c) B is the methylene or carbonyl group;
(d) R³ is a carbocycle, heterocyclyl, aryl, heteroaryl, carbocycle-C₁-C₈ alkyl, aryl-C₁-C₈ alkyl, aryl-C₁-C₈ haloalkyl, heterocyclyl-C₁-C₈ alkyl, heteroaryl-C₁-C₈ alkyl, carbocycle-C₂-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups;
(e) D is the -NH- group;
(f) E is the hydroxy group; and
(g) Q comprises a methylated benzoxazinone.

Non-limiting examples of these compounds include 2-benzyl-4-(5-fluoro-2-methoxyphenyl)-2-hydroxy-4-methylpentanoic acid(4-methyl-1-oxo-1H-benzo[d][1,2]oxazin-6-yl)amide; 2-benzyl-4-(5-fluoro-2-hydroxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-methoxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-hydroxyphenyl)-2-hydroxy-4-methylpentanoic acid(4-methyl-1-oxo-1H-benzo[d][1,2]oxazin-6-yl)amide; 2-benzyl-2-hydroxy-4-methyl-4-methylpentanoic acid(4-methyl-1-oxo-1H-benzo[d][1,2]oxazin-6-yl)amide; and 2-cyclohexylmethyl-2-hydroxy-4-methylpentanoic acid(4-methyl-1-oxo-1H-benzo[d][1,2]oxazin-6-yl)amide.

In still another embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl, or R¹ and R² together with the carbon atom they are commonly attached to form a C₃-C₈ spiro cycloalkyl ring;
(c) R³ is the trifluoromethyl group;
(d) B is C₁-C₅ alkyl, C₂-C₅ alkenyl, or C₂-C₅ alkynyl, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q is an aryl or heteroaryl group one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, acyl, C₁-C₃ silanyloxy, C₁-C₅ alkoxycarbonyl, carboxy, halogen, hydroxy, oxo, cyano, heteroaryl, heterocyclyl, amino wherein the nitrogen atom is optionally independently mono- or di-substituted by C₁-C₅ alkyl or aryl, ureido wherein either nitrogen atom is optionally independently substituted with C₁-C₅ alkyl, and trifluoromethyl.

Non-limiting examples of these compounds include 2-(3,5-difluorobenzyl)-1,1,1-trifluoro-4-(5-fluoro-2-methoxyphenyl)-4-methylpentan-2-ol; 2-biphenyl-4-ylmethyl-1,1,1-trifluoro-4-(5-fluoro-2-methoxyphenyl)-4-methylpentan-2-ol; 2-(3,5-dimethylbenzyl)-1,1,1-trifluoro-4-(5-fluoro-2-methoxyphenyl)-4-methylpentan-2-ol; 2-(3-bromobenzyl)-1,1,1-trifluoro-4-(5-fluoro-2-methoxyphenyl)-4-methylpentan-2-ol; 2-(3,5-dichlorobenzyl)-1,1,1-trifluoro-4-(5-fluoro-2-methoxyphenyl)-4-methylpentan-2-ol; 2-(3,5-bis-trifluoromethylbenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(3-fluoro-5-trifluoromethylbenzyl)-4-methylpentan-2-ol; 2-(3-chloro-2-fluoro-5-trifluoromethylbenzyl-)-1,1,1-trifluoro-4-(5-fluoro-2-methoxyphenyl)-4-methylpentan-2-ol; 4-[4-(5-fluoro-2-methoxyphenyl)-2-hydroxy-4-methyl-2-trifluoromethylpentyl]benzonitrile; 2-(3,5-dibromobenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(2-fluoro-3-trifluoromethylbenzyl)-4-methylpentan-2-ol; 1,1,1-trifluoro-4-(5-fluoro-2-methoxyphenyl)-2-(2-fluoro-5-trifluoromethylbenzyl)-4-methylpentan-2-ol.

In still another embodiment, said at least a DIGRA has Formula I, wherein
(a) A is an aryl, heteroaryl, or C₅-C₁₅ cycloalkyl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen, C₁-C₅ alkyl, C₅-C₁₅ arylalkyl, or R¹ and R² together with the carbon atom they are commonly attached to form a C₃-C₈ spiro cycloalkyl ring;
(c) R³ is the trifluoromethyl group;
(d) B is the carbonyl group or methylene group, which is optionally independently substituted with one or two substituent groups selected from C₁-C₅ alkyl, hydroxy, and halogen;
(e) D is absent;
(f) E is the hydroxy group or amino group wherein the nitrogen atom is optionally independently mono- or di-substituted by C₁-C₅ alkyl; and
(g) Q comprises a pyrrolidine, morpholine, thiomorpholine, piperazine, piperidine, 1H-pyridin-4-one, 1H-pyridin-2-one, 1H-pyridin-4-ylideneamine, 1H-quinolin-4-ylideneamine, pyran, tetrahydropyran, 1,4-diazepane, 2,5-diazabicyclo[2.2.1]heptane, 2,3,4,5-tetrahydrobenzo[b][1,4]diazepine, dihydroquinoline, tetrahydroquinoline, 5,6,7,8-tetrahydro-1H-quinolin-4-one, tetrahydroisoquinoline, decahydroisoquinoline, 2,3-dihydro-1H-isoindole, 2,3-dihydro-1H-indole, chroman, 1,2,3,4-tetrahydroquinoxaline, 1,2-dihydroindazol-3-one, 3,4-dihydro-2H-benzo[1,4]oxazine, 4H-benzo[1,4]thiamine, 3,4-dihydro-2H-benzo[1,4]thiazine, 1,2-dihydrobenzo[d] [1,3]oxazin4-one, 3,4-dihydrobenzo[1,4]oxazin4-one, 3H-quinazolin4-one, 3,4-dihydro-1H-quinoxalin-2-one, 1H-quinnolin-4-one, 1H-quinazolin4-one, 1H-[1,5]naphthyridin-4-one, 5,6,7,8-tetrahydro-1H-[1,- 5]naphthyridin-4-one, 2,3-dihydro-1H-[1,5]naphthyridin-4-one, 1,2-dihydropyrido[3,2-d][1,3]oxazin-4-one, pyrrolo[3,4-c]pyridine-1,3-dione, 1,2-dihydropyrrolo[3,4-c]pyridin-3-one, or tetrahydro[b][1,4]diazepinone group, each optionally independently substituted with one to three substituent groups, wherein each substituent group of Q is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by C₁-C₅ alkyl, ureido wherein either nitrogen atom is optionally independently substituted with C₁-C₅ alkyl, or C₁-C₅ alkylthio wherein the sulfur atom is optionally oxidized to a sulfoxide or sulfone, wherein each substituent group of Q is optionally independently substituted with one to three substituent groups selected from C₁-C₃ alkyl, C₁-C₃ alkoxy, C₁-C₃ alkoxycarbonyl, acyl, aryl, benzyl, heteroaryl, heterocyclyl, halogen, hydroxy, oxo, cyano, amino wherein the nitrogen atom is optionally independently mono- or di-substituted by C₁-C₅ alkyl, or ureido wherein either nitrogen atom is optionally independently substituted with C₁-C₅ alkyl.

Non-limiting examples of these compounds include 2-(2,6-dimethylmorpholin-4-ylmethyl)-1,1,1-trifluoro-4-(5-fluoro-2-methoxyphenyl)-4-methylpentan-2-ol; 1-[4-(5-fluoro-2-methoxyphenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-3,5-dimethylpiperidin-4-one; 1-[4-(5-fluoro-2-methoxyphenyl)-2-hydroxy-4-methyl-2-trifluoromethylpentyl]-3-methyl-1H-quinolin-4-one; 1-[4-(5-fluoro-2-methoxyphenyl)-2-hydroxy-4-methyl-2-trifluoromethylpentyl)-2,3-dihydro-1H-quinolin-4-one; 1-[4-(4-fluorophenyl)-2-hydroxy-4-methyl-2-trifluoromethylpentyl]-1H-quinolin-4-one; 1-[4-(3-fluorophenyl)-2-hydroxy-4-methyl-2-trifluoromethylpentyl]-1H-quinolin-4-one; 1-[4-(4-fluoro-2-hydroxyphenyl)-2-hydroxy-4-methyl-2-trifluoromethylpentyl]-1H-quinolin-4-one; 1-[4-phenyl-2-hydroxy-4-methyl--2-trifluoromethylpentyl]-1H-quinolin-4-one; 1-[4-(5-fluoro-2,3-dihydrobenzofuran-7-yl)-2-hydroxy-4-methyl-2-trifluoromethylpentyl]-1H-quinolin-4-one; 1-[4-(5-bromo-2,3-dihydrobenzofuran-7-yl)-2-hydroxy-4-methyl-2-trifluoromethylpentyl]-1H-quinolin-4-one; 1-[4-(5-methyl-2,3-dihydrobenzofuran-7-y-1)-2-hydroxy-4-methyl-2-trifluoromethylpentyl]-1H-quinolin-4-one; 1-[4-(5-chloro-2,3-dihydrobenzofuran-7-yl)-2-hydroxy-4-methyl-2-trifluoromethylpentyl]-1H-quinolin-4-one; 1-[4-(2,3-dihydrobenzofuran-7-yl)-2-hydroxy-4-methyl-2-trifluoromethylpentyl]- 1H-quinolin-4-one; 1-[4-(5-fluoro-2-hydroxyphenyl)-2-hydroxy-4-methyl-2-trifluoromethylpentyl]-1H-[1,5]naphthyridin-4-one; -[4-(5-fluoro-2-methoxyphenyl)-2-hydroxy-2,4-dimethylpentyl]-3,5-dimethyl-1H-pyridin-4-one; 1-[2-hydroxy-4-(2-methoxy-5-thiophen-2-ylphenyl)-4-methyl-2-trifluoromethylpentyl]-1H-quinolin-4-one; 1-[4-(6-bromobenzo[1,3]dioxol-4-yl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-3-methyl-1H-quinolin-4-one; 1-[2-hydroxy-4-(4-hydroxybiphenyl-3-yl)-4-methyl-2-trifluoromethylpentyl]-1H-quinolin-4-one; 1-{4-[5-(3,5-dimethylisoxazol-4-yl)-2-hydroxyphenyl]-2-hydroxy-4-methyl-2-trifluoromethylpentyl}-1H-quinolin-4-one; 1-[2-hydroxy-4-(2-hydroxy-5-thiophen-3-ylphenyl)-4-methyl-2-trifluoromethylpentyl]-1H-quinolin-4-one; 1-{4-[5-(3,5-dimethylisoxazol-4-yl)-2-methoxyphenyl]-2-hydroxy-4-methyl-2-trifluoromethylpentyl}-1H-quinolin-4-one; 1-[2-hydroxy-4-methyl-4-(3-pyridin-3-ylphenyl)-2-trifluoromethylpentyl]-1H-quinolin-4-one; 4-methoxy-3-[4,4,4-trifluoro-3-hydroxy-1,1-dimethyl-3-(4-oxo-4H-quinolin-1-ylmethyl)butyl]benzaldehyde; 1-[2-hydroxy-4-(2-methoxy-5-thiophen-3-ylphenyl)-4-methyl-2-trifluoromethylpentyl]-1H-quinolin-4-one; 1-[4-(5-furan-3-yl-2-methoxyphenyl)-2-hydroxy-4-methyl-2-trifluoromethylpentyl]-1H-quinolin-4-one; 1-[2-hydroxy-4-(4-methoxybiphenyl-3-yl)-4-methyl-2-trifluoromethylpentyl]-1H-quinolin-4-one; 1-[4-(5-acetyl-2-hydroxyphenyl)-2-hydroxy-4-methyl-2-trifluoromethylpentyl)-1H-quinolin-4-one; 1-[3,3,3-trifluoro-2-(6-fluoro-4-methylchroman-4-ylmethyl)-2-hydroxypropyl]-1H-quinolin-4-one; 1-(4-{3-[1-(benzyloxyimino)ethyl]phenyl}-2-hydroxy-4-methyl-2-trifluoromethylpentyl)-1H-quinolin-4-one; 1-[4-(5-acetyl-2-methoxyphenyl)-2-hydroxy-4-methyl-2-trifluoromethylpentyl]-1H-quinolin-4-one; 1-(2-hydroxy-4-{3-[1-(methoxyimino)ethyl]phenyl}-4-methyl-2-trifluoromethylpentyl)-1H-quinolin-4-one; 1-[4-(5-bromo-2-hydroxyphenyl)-2-hydroxy-4-methyl-2-trifluoromethylpentyl]-1H-quinolin-4-one; 1-(2-hydroxy-4-{3-[1-(hydroxyimino)ethyl]phenyl}-4-methyl-2-trifluoromethylpentyl)-1H-quinolin-4-one; 1-[4-(5-bromo-2-methoxyphenyl)-2-hydroxy-4-methyl-2-trifluoromethylpentyl]-1H-quinolin-4-one; 1-[4-(3,5-difluorophenyl)-2-hydroxy-4-methyl-2-trifluoromethylpentyl]-1H-quinolin-4-one; 1-[4-(3,5-dimethylphenyl)-2-hydroxy-4-methyl-2-trifluoromethylpentyl]-1H-quinolin-4-one; 1-{2-hydroxy-4-methyl-4-[3-(2-methyl-[1,3]dioxolan-2-yl)phenyl]-2-trifluoromethylpentyl}-1H-quinolin-4-one: 1-[4-(2,3-dihydrobenzofuran-7-yl)-2-hydroxy-4-methyl-2-trifluoromethylpentyl]-1H-[1,5]naphthyridin-4-one; 1-[4-(3-[1,3]dioxan-2-ylphenyl)-2-hydroxy-4-methyl-2-trifluoromethylpentyl]-1H-quinolin-4-one; 1-{4-[3-(3,5-dimethylisoxazol-4-yl)phenyl]-2-hydroxy-4-methyl-2-trifluoromethylpentyl}-1H-quinolin-4-one; 1-[4-(2,3-dihydrobenzofuran-7-yl)-2-hydroxy-4-methyl-2-trifluoromethylpentyl]-3,5-dimethyl-1H-pyridin-4-one; 1-[4-(5-fluoro-2-methoxyphenyl)-2-hydroxy-4-methyl-2-trifluoromethylpentyl]-2-hydroxymethyl-3,5-dimethyl-1H-pyridin-4-one; 1-[4-(5-fluoro-2-hydroxyphenyl)-2-hydroxy-4-methyl-2-trifluoromethylpentyl]-3-hydroxymethyl-1H-quinolin-4-one; 1-[4-(3-bromophenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-6-methyl-1H-quinolin-4-one; 6-chloro-1-[4-(5-fluoro-2-hydroxyphenyl)-2-hydroxy-4-methyl-2-trifluoromethylpentyl]-1H-quinolin-4-one; 1-[-4-(2-difluoromethoxy-5-fluorophenyl)-2-hydroxy-4-methyl-2-trifluoromethylpentyl]-1H-quinolin-4-one; 1-(4-biphenyl-3-yl-2-hydroxy-4-methyl-2-trifluoromethylpentyl)-1H-quinolin-4-one; 1-[2-hydroxy-4-(2-hydroxy-5-methylphenyl)-4-methyl-2-trifluoromethylpentyl]-1H-quinolin-4-one; 1-[2-hydroxy-4-(3-isopropoxyphenyl)-4-methyl-2-trifluoromethylpentyl]-1H-quinolin-4-one; 1-[4-(3-ethoxyphenyl)-2-hydroxy-4-methyl-2-trifluoromethylpentyl]-1H-quinolin-4-one; 1-[2-hydroxy-4-(2-methoxy-5-methylphenyl)-4-methyl-2-trifluoromethylpentyl]-1H-quinolin-4-one; 1-[4-(2,5-dimethylphenyl)-2-hydroxy-4-methyl-2-trifluoromethylpentyl]-1H-quinolin-4-one; 1-[2-hydroxy-4-(3-methoxyphenyl)-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1,2-dihydroindazol-3-one; 7-fluoro-1-[4-(5-fluoro-2-hydroxyphenyl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-3,5-dimethyl-1H-pyridin-4-one; 7-fluoro-1-[4-(5-fluoro-2-methoxyphenyl)-2-hydroxy-4-methyl-2-trifluoromethylpentyl]-1H-quinolin-4-one; 1-(2-hydroxy-4-methyl-4-phenyl-2-trifluoromethylhexyl)-1H-quinolin-4-one; 1-[4-(4-fluoro-2-methylphenyl)-2-hydroxy-4-methyl-2-trifluoromethylpentyl]-H-quinolin-4-one; 1-[4-(3,4-dimethylphenyl)-2-hydroxy-4-methyl-2-trifluoromethylpentyl]-1H-quinolin-4-one; 8-fluoro-1-[4-(5-fluoro-2-hydroxyphenyl)-2-hydroxy-4-methyl-2-trifluoromethylpentyl]-1H-quinolin-4-one; 6-fluoro-1-[4-(5-fluoro-2-hydroxyphenyl)-2-hydroxy-4-methyl-2-trifluoromethylpentyl]-1H-quinolin-4-one; 7-chloro-1-[4-(5-fluoro-2-hydroxyphenyl)-2-hydroxy-4-methyl-2-trifluoromethylpentyl]-1H-quinolin-4-one; 1-[4-(5-fluoro-2-isopropoxyphenyl)-2-hydroxy-4-methyl-2-trifluoromethylpentyl]-H-quinolin-4-one; 1-[4-(2-ethoxy-5-fluorophenyl)-2-hydroxy-4-methyl-2-trifluoromethylpentyl]-1H-quinolin-4-one; 8-fluoro-1-[4-(5-fluoro-2-methoxyphenyl)-2-hydroxy-4-methyl-2-trifluoromethylpentyl]-1H-quinolin-4-one; 6-fluoro-1-[4-(5-fluoro-2-methoxyphenyl)-2-hydroxy-4-methyl-2-trifluoromethylpentyl]-1H-quinolin-4-one; 1-[2-hydroxy-4-(5-methanesulfonyl-2,3-dihydrobenzofuran-7-yl)-4-methyl-2-trifluoromethylpentyl]-1H-quinolin-4-one; 1-[2-hydroxy-4-methyl-4-(5-methylsulfanyl-2,3-dihydrobenzofuran-7-yl)-2-trifluoromethylpentyl]-1H-quinolin-4-one; 7-chloro-1-[4-(5-fluoro-2-methoxyphenyl)-2-hydroxy-4-methyl-2-trifluoromethylpentyl]-1H-quinolin-4-one; 3-chloro-1-[4-(5-fluoro-2-methoxyphenyl)-2-hydroxy-4-methyl-2-trifluoromethylpentyl]-5-trifluoromethyl-1H-pyridin-2-one; 1-[2-hydroxy-4-(5-methanesulfonyl-2,3-dihydrobenzofuran-7-yl)-4-methyl-2-trifluoromethylpentyl]-3-methyl-1H-quinolin-4-one; 1-[2-hydroxy-4-(2-methoxy-5-pyridin-3-ylphenyl)-4-methyl-2-trifluoromethylpentyl]-1H-quinolin-4-one; 1-[2-hydroxy-4-(2-hydroxy-3,5-dimethylphenyl)-4-methyl-2-trifluoromethylpentyl]-H-quinolin-4-one; 1-[4-(3-[1,3]dioxan-2-yl-4-fluorophenyl)-2-hydroxy-4-methyl-2-trifluoromethylpentyl]-1H-quinolin-4-one; 2-(1,1-dioxo-2,3-dihydro-1H-1λ⁶-benzo[1,4]thiazin-4-ylmethyl)-1,1,1-trifluoro-4-(5-fluoro-2-methoxyphenyl)-4-methylpentan-2-ol; 2-(2,3-dihydrobenzo[1,4]oxazin4-ylmethyl)-1,1,1-trifluoro-4-(5-fluoro-2-methoxyphenyl)-4-methylpentan-2-ol; 1-[4-(5-fluoro-2-hydroxyphenyl)-2-hydroxy-4-methyl-2-trifluoromethylpentyl]-H-quinolin-4-one; 1-[4-(5-fluoro-2-hydroxyphenyl)-2-hydroxy-4-methyl-2-trifluoromethylpentyl]-H-[1,5]naphthyridin-4-one; 1-[4-(5-fluoro-2-methylphenyl)-2-hydroxy-4-methyl-2-trifluoromethylpentyl]-H-quinolin-4-one; 1-[4-(2,4-dimethylphenyl)-2-hydroxy-4-methyl-2-trifluoromethylpentyl]-1H-quinolin-4-one; 1-[4-(4-fluoro-2-methoxyphenyl)-2-hydroxy-4-methyl-2-trifluoromethylpentyl]-H-quinolin-4-one; 1-[4-(3-fluoro-4-methoxyphenyl)-2-hydroxy-4-methyl-2-trifluoromethylpentyl]-1H-quinolin-4-one; 1-(4-benzo[1,3]dioxol-4-yl-2-hydroxy-4-methyl-2-trifluoromethylpentyl)-1H-quinolin-4-one; 1-[4-(5-fluoro-2-methoxyphenyl)-2-hydroxy-4-methyl-2-trifluoromethylpentyl]-1,2-dihydroindazol-3-one; 1,1,1-trifluoro-4-(5-fluoro-2-methoxyphenyl)-4-methyl-2-(1-oxo-2,3-dihydro-1H-1λ⁴-benzo[1,4-]thiazin-4-ylmethyl)pentan-2-ol; 1-[4-(5-fluoro-2-methoxyphenyl)-2-hydroxy-4-methyl-2-trifluoromethylpentyl]-2-hydroxymethyl-3,5-dimethyl-1H-pyridin--4-one; 1-[4-(2,3-dihydrobenzofuran-7-yl)-2-hydroxy-4-methyl-2-trifluoromethylpentyl]-3-methyl-1H-quinolin-4-one; 1-[2-hydroxy-4-(2-methoxy-3,5-dimethylphenyl)-4-methyl-2-trifluoromethylpentyl]-1H-quinolin-4-one; 1-[2-hydroxy-4-(2-hydroxy-5-pyridin-3-ylphenyl)-4-methyl-2-trifluoromethylpentyl]-1H-quinolin-4-one; and 1-[2-hydroxy-4-(2-hydroxy-5-pyridin-5-ylphenyl)-4-methyl-2-trifluoromethylpentyl]-1H-quinolin-4-one.

In still another embodiment, said at least a DIGRA has Formula I, wherein A, R¹, R², B, D, E, and Q have the meanings disclosed immediately above, and R³ is hydrogen, C₁-C₈ alkyl, C₂-C₈ alkenyl, C₂-C₈ alkynyl, carbocycle, heterocyclyl, aryl, heteroaryl, carbocycte-C₁-C₈ alkyl, carboxy, alkoxycarbonyl, aryl-C₁-C₈ alkyl, aryl-C₁-C₈ haloalkyl, heterocyclyl-C₁-C₈ alkyl, heteroaryl-C₁-C₈ alkyl, carbocycle-C₂-C₈ alkenyl, aryl-C₂-C₈ alkenyl, heterocyclyl-C₁-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone, wherein R³ cannot be trifluoromethyl.

In still another embodiment, said at least a DIGRA has Formula I, wherein
(a) A is an aryl, heteroaryl, or C₅-C₁₅ cycloalkyl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl, or R¹ and R² together with the carbon atom they are commonly attached to form a C₃-C₈ spiro cycloalkyl ring;
(c) R³ is the trifluoromethyl group;
(d) B is the carbonyl group;
(e) D is the -NH- group;
(f) E is the hydroxy group; and
(g) Q comprises an optionally substituted phenyl group having the formula 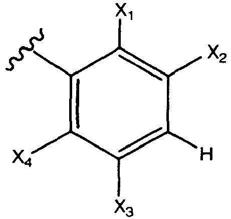 wherein X₁, X₂, X₃ and X₄ are each independently selected from the group consisting of hydrogen, halogen, hydroxy, trifluoromethyl, trifluoromethoxy, C₁-C₅ alkyl, C₂-C₅ alkenyl, C₂-C₅ alkynyl, C₁-C₅ alkoxy, C₁-C₅ alkylthio wherein the sulfur atom is optionally oxidized to a sulfoxide or sulfone, C₁-C₅ alkanoyl, C₁-C₅ alkoxycarbonyl, C₁-C₅ acyloxy, C₁-C₅ alkanoylamino, C₁-C₅ carbamoyloxy, urea, aryl, and amino wherein the nitrogen atom may be independently mono- or di-substituted by C₁-C₅ alkyl, and wherein said aryl group is optionally substituted by one or more hydroxy or C₁-C₅ alkoxy groups, and wherein either nitrogen atom of the urea group may be independently substituted by C₁-C₅ alkyl; or Q is an aromatic 5- to 7-membered monocyclic ring having from one to four heteroatoms in the ring independently selected from nitrogen, oxygen, and sulfur, optionally independently substituted with one to three substituent groups selected from the group consisting of hydrogen, halogen, hydroxy, trifluoromethyl, trifluoromethoxy, C₁-C₅ alkyl, C₂-C₅ alkenyl, C₂-C₅ alkynyl, C₁-C₅ alkoxy, C₁-C₅ alkylthio wherein the sulfur atom is optionally oxidized to a sulfoxide or sulfone, C₁-C₅ alkanoyl, C₁-C₅ alkoxycarbonyl, C₁-C₅ acyloxy, C₁-C₅ alkanoylamino, C₁-C5 carbamoyloxy, urea, aryl optionally substituted by one or more hydroxy or C₁-C₅ alkoxy groups, and amino wherein the nitrogen atom may be independently mono- or di-substituted by C₁-C₅ alkyl, and wherein either nitrogen atom of the urea group may be independently substituted by C₁-C₅ alkyl.

Non-limiting examples of these compounds include 4-(5-fluoro-2-hydroxyphenyl)-2-hydroxy-4-methyl-2-trifluoromethyl-pentanoic acid (3,5-dichloro-phenyl)-amide; 4-(5-fluoro-2-hydroxy-phenyl)-2-hydroxy-4-methyl-2-trifluoromethyl-pentanoic acid (3-chloro-phenyl)-amide; 4-(5-fluoro-2-hydroxy-phenyl)-2-hydroxy-4-methyl-2-trifluoromethyl-pentanoic acid (2-chloro-phenyl)-amide; 4-(5-fluoro-2-hydroxy-phenyl)-2-hydroxy-4-methyl-2-trifluoromethyl-pentanoic acid (2,6-dichloro-pyrimidin-4-yl)-amide; 4-(5-fluoro-2-hydroxy-phenyl)-2-hydroxy-4-methyl-2-trifluoromethyl-pentanoic acid (2,6-dichloro-pyridin-4-yl)-amide; 4-(5-fluoro-2-hydroxy-phenyl)-2-hydroxy-4-methyl-2-trifluoromethyl-pentanoic acid (2,3-dichloro-phenyl)-amide; 4-(5-fluoro-2-hydroxy-phenyl)-2-hydroxy-4-methyl-2-trifluoromethyl-pentanoic acid (3,5-dimethylphenyl)-amide; 4-(5-fluoro-2-hydroxy-phenyl)-2-hydroxy-4-methyl-2-trifluoromethyl-pentanoic acid (3,5-bis-trifluoromethyl-phenyl)-amide; 4-(5-fluoro-2-hydroxy-phenyl)-2-hydroxy-4-methyl-2-trifluoromethyl-pentanoic acid (2,5-dichloro-phenyl)-amide; 4-(5-fluoro-2-hydroxy-phenyl)-2-hydroxy-4-methyl-2-trifluoromethyl-pentanoic acid (3-bromo-phenyl)-amide; 4-(5-fluoro-2-hydroxy-phenyl)-2-hydroxy-4-methyl-2-trifluoromethyl-pentanoic acid (3,5-difluoro-phenyl)-amide; 4-(5-fluoro-2-hydroxyphenyl)-2-hydroxy-4-methyl-2-trifluoromethyl-pentanoic acid (3,5-dibromo-phenyl)-amide.

In still another embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₄ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C5 alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl;
(c) R³ is C₁-C₈ alkyl, C₂-C₈ alkenyl, C₂-C₈ alkynyl, carbocycle, heterocyclyl, aryl, heteroaryl, carbocycle-C₁-C₈ alkyl, aryl-C₁-C₈ alkyl, aryl-C₁-C₈ haloalkyl, heterocyclyl-C₁-C₈ alkyl, heteroaryl-C₁-C₈ alkyl, carbocycle-C₂-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, or C₁-C₅ alkylthio wherein the sulfur atom is optionally oxidized to a sulfoxide or sulfone, wherein R³ cannot be trifluoromethyl;
(d) B is C₁-C₅ alkylene, C₂-C₅ alkenylene, or C₂-C₅ alkynylene, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q comprises an azaindolyl group optionally independently substituted with one to three substituent groups, wherein each substituent group of Q is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, *C₁-C₅* alkylsulfonylamino, aminosulfonyl, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by C₁-C₅ alkyl, ureido wherein either nitrogen atom is optionally independently substituted with C₁-C₅ alkyl, or C₁-C₅ alkylthio wherein the sulfur atom is optionally oxidized to a sulfoxide or sulfone, wherein each substituent group of Q is optionally independently substituted with one to three substituent groups selected from C₁-C₃ alkyl, C₁-C₃ alkoxy, halogen, hydroxy, oxo, cyano, amino, or trifluoromethyl.

Non-limiting examples of these compounds include 1,1,1-trifluoro-4-(5-fluoro-2-methoxyphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(1H-pyrrolo[2,3-b]pyridin-2-ylmethyl)pentan-2-ol; 1,1,1-trifluoro-4-(5-fluoro-2-methoxyphenyl)-4-methyl-2-(1H-pyrrolo[3,2-c]pyridin-2-ylmethyl)pentan-2-ol; 1,1,1-trifluoro-4-(5-fluoro-2-methoxyphenyl)-4-methyl-2-(1H-pyrrolo[3,2-b]pyridin-2-ylmethyl)pentan-2-ol; 4-fluoro-2-[4,4,4-trifluoro-3-hydroxy-1,1-dimethyl-3-(1H-pyrrolo[2,3-c]pyridin-2-ylmethyl)butyl]phenol; 4-fluoro-2-[4,4,4-trifluoro-3-hydroxy-1,1-dimethyl-3-(1H-pyrrolo[2,3-b]pyridin-2-ylmethyl)butyl]phenol; 4-fluoro-2-[4,4,4-trifluoro-3-hydroxy-1,1-dimethyl-3-(1H-pyrrolo[3,2-c]pyridin-2-ylmethyl)butyl]phenol; 4-fluoro-2-[4,4,4-trifluoro-3-hydroxy-1,1-dimethyl-3-(1H-pyrrolo[3,2-b]pyridin-2-ylmethyl)butyl]phenol; 1,1,1-trifluoro-4-(3-fluorophenyl)-4-methyl-2-(1H-pyrrolo[2,3-c]pyridin-2-ylmethyl)pentan-2-ol; 1,1,1-trifluoro-4-(4-fluorophenyl)-4-methyl-2-1H-pyrrolo[2,3-c]pyridin-2-ylmethyl)pentan-2-ol; 4-(2,3-dihydrobenzofuran-7-yl)-1,1,1-trifluoro-4-methyl-2-(1H-pyrrolo[2,3-c]pyridin-2-yelmethyl)pentan-2-ol; 4-(2,3-dihydrobenzofuran-7-yl)-1,1,1-trifluoro-4-methyl-2-(1H-pyrrolo[3,2-c]pyridin-2-yelmethyl)pentan-2-ol; 1,1,1-trifluoro-4-methyl-4-phenyl-2-(1H-pyrrolo[2,3-c]pyridine-2-ylmethyl)pentan-2-ol; 1,1,1-trifluoro-4-(4-fluoro-2-methoxyphenyl)-4-methyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[3,2-c]pyridin-2-ylmethyl)pentan-2-ol; 1,1,1-trifluoro-4-methyl-4-phenyl-2-(1H-pyrrolo[3,2-c]pyridin-2-ylmethyl)pentan-2-ol; 1,1,1-trifluoro-4-(4-fluorophenyl)-4-methyl-2-(1H-pyrrolo[3,2-c]pyridin-2-ylmethyl)pentan-2-ol; 5-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methylphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(3-methyl-1H-pyrrolo[2,3-c]pyridin-2-ylmethyl)pentan-2-ol; 4-fluoro-2-[4,4,4-trifluoro-3-hydroxy-1,1-dimethyl-3-(3-methyl-1H-pyrrolo[2,3-c]pyridin-2-ylmethyl)butyl]phenol; 5-fluoro-2-[4,4,4-trifluoro-3-hydroxy-1,1-dimethyl-3-(1H-pyrrolo[3,2-c]pyridin-2-ylmethyl)butyl]pentan-2-ol; 1,1,1-trifluoro-4-(5-fluoro-2,3-dihydrobenzofuran-7-yl)-4-methyl-2-(1H-pyrrolo[2,3-c]pyridine-2-ylmethyl)pentan-2-ol; 4-fluoro-2-[4,4,4-trifluoro-3-hydroxy-1,1-dimethyl-3-(1H-pyrrolo[2,3-c]-[3-methylpyridin]-2-ylmethyl)butyl]phenol; 4-fluoro-2-[4,4,4-trifluoro-3-hydroxy-1,1-dimethyl-3-(1H-pyrrolo[2,3-c]-[2-fluoropyridin]-2-ylmethyl)butyl]phenol; and 4-fluoro-2-[4,4,4-trifluoro-3-hydroxy-1,1-dimethyl-3-(1H-pyrrolo[2,3-c]-[2-trifluoromethylpyridin]-2-ylmethyl)butyl]phenol.

In still another embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl, or R¹ and R² together with the carbon atom they are commonly attached to form a C₃-C₈ spiro cycloalkyl ring;
(c) R³ is the trifluoromethyl group;
(d) B is C₁-C₅ alkylene, C₂-C₅ alkenylene, or C₂-C₅ alkynylene, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q comprises a heteroaryl group optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, acyl, C₁-C₃ silanyloxy, C₁-C₅ alkoxycarbonyl, carboxy, halogen, hydroxy, oxo, cyano, heteroaryl, heterocyclyl, amino wherein the nitrogen atom is optionally independently mono- or di-substituted by C₁-C₅ alkyl or aryl, ureido wherein either nitrogen atom is optionally independently substituted with C₁-C₅ alkyl, or trifluoromethyl.

Non-limiting examples of these compounds include 4-cyclohexyl-1,1,1-trifluoro-4-methyl-2-quinolin-4-ylmethylpentan-2-ol; 4-pyrimidin-5-yl-2-(4,4,4-trifluoro-3-hydroxy-1,1-dimethyl-3-(1H-pyrrolo[2,3-c]pyridin-2-ylmethyl)butyl]phenol; 4-pyrimidin-5-yl-2-[4,4,4-trifluoro-3-hydroxy-1,1-dimethyl-3-(1H-pyrrolo[3,2-c]pyridin-2-ylmethyl)butyl]phenol; 1,1,1-trifluoro-4-(5-fluoro-2-methoxyphenyl)-4-methyl-2-(3-methyl-1H-pyrrolo[3,2-c]pyridin-2-ylmethyl)pentan-2-ol; 1,1,1-trifluoro-4-(5-fluoro-2,3-dihydrobenzofuran-7-yl)-4-methyl-2-(1H-pyrrolo[3,2-c]pyridin-2-ylmethyl)pentan-2-ol; 1,1,1-trifluoro-4-(5-fluoro-2-methylphenyl)-4-methyl-2-(3-methyl-1H-pyrrolo[2,3-c]pyridin-2-ylmethyl)pentan-2-ol;2-(4,6-dimethyl-1H-pyrrolo[3,2-c]pyridin-2-ylmethyl)-1,1,1-tritluoro-4-(5-fluoro-2-methoxyphenyl)-4-methylpentan-2-ol; 2-(5,7-dimethyl-1H-pyrrolo[2,3-c]pyridin-2-ylmethyl)-1,1,1-trifluoro-4-fluoro-2-methoxyphenyl)-4-methylpentan-2-ol; 2-[4-(5-fluoro-2-methoxyphenyl)-2-hydroxy-4-methyl-2-trifluoromethylpentyl]-1H-pyrrolo[3,2-b]pyridine-5-carbonitrile; 1,1,1-trifluoro-4-(5-fluoro-2-methoxyphenyl)-4-methyl-2-(6-methyl-1H-pyrrolo[3,2-c]pyridin-2-ylmethyl)pentan-2-ol; 1,1,1-trifluoro-4-(5-fluoro-2-methoxyphenyl)-4-methyl-2-(4-methyl-1H-pyrrolo[3,2-c]pyridin-2-ylmethyl)pentan-2-ol; 2-[4-(5-fluoro-2-methoxyphenyl)-2-hydroxy-4-methyl-2-trifluoromethylpentyl]-4-methyl-1H-pyrrolo[3,2-c]pyridine-6-carbonitrile; 2-[4-(5-fluoro-2-methoxyphenyl)-2-hydroxy-4-methyl-2-trifluoromethylpentyl]-1H-pyrrolo[2,3-c]pyridine-5-carbonitrile; 2-[4-(5-fluoro-2-methoxyphenyl)-2-hydroxy-4-methyl-2-trifluoromethylpentyl]-1H-pyrrolo[3,2-c]pyridine-4-carbonitrile; 1,1,1-trifluoro-4-(5-fluoro-2-methoxyphenyl)-4-methyl-2-(5H-pyrrolo[3,2-d]pyrimidin-6-ylmethyl)pentan-2-ol; 1,1,1-trifluoro-4-(5-fluoro-2-methoxyphenyl)-4-methyl-2-thieno[2,3-d]pyridazin-2-ylmethylpentan-2-ol; 1,1,1-trifluoro-4-(5-fluoro-2-methoxyphenyl)-4-methyl-2-(5H-pyrrolo[3,2-c]pyridazin-6-ylmethyl)pentan-2-ol; 1,1_{,}1-trifluoro-4-(5-fluoro-2-methoxyphenyl)-4-methyl-2-(2-methyl-5H-pyrrolo[3,2-d]pyrimidin-6-ylmethyl)pentan-2-ol; 1,1,1-trifluoro-4-(5-fluoro-2-methylphenyl)-4-methyl-2-(1H-pyrrolo[2,3-d]pyridazin-2-ylmethyl)pentan-2-ol; 2-(4,6-dimethyl-H-pyrrolo[3,2-c]pyridin-2-ylmethyl)-1,1,1-trifluoro-4-(5-fluoro-2-methylphenyl)-4-methylpentan-2-ol; 4-(5-chloro-2,3-dihydrobenzofuran-7-yl)-2-(4,6-dimethyl-1H-pyrrolo[3,2-c]pyridin-2-ylmethyl)-1,1,1-trifluoro-4-methylpentan-2-ol; 2-[4-(5-fluoro-2-methylphenyl)-2-hydroxy-4-methyl-2-trifluoromethylpentyl]-1H-pyrrolo[3,2-b]pyridine-5-carbonitrile; 4-(5-chloro-2,3-dihydrobenzofuran-7-yl)-1,1,1-trifluoro-4-methyl-2-(3-methyl-1H-pyrrolo[2,3-c]pyridin-2-ylmethyl)pentan-2-ol; 1,1,1-trifluoro-4-(5-fluoro-2-methylphenyl)-4-methyl-2-(5H-pyrrolo[3,2-c]- pyridazin-6-ylmethyl)pentan-2-ol; 4-(5-chloro-2,3-dihydrobenzofuran-7-yl)-1,1,1-trifluoro-4-methyl-2-(5H-pyrrolo[3,2-c]pyridazin-6-ylmethyl)pentan-2-ol; 4-(5-chloro-2,3-dihydrobenzofuran-7-yl)-1,1,1-trifluoro-4-methyl-2-(1-H-pyrrolo[2,3-d]pyridazin-2-ylmethyl)pentan-2-ol; 1,1,1-trifluoro-4-(5-fluoro-2-methoxyphenyl)-2-(7-fluoro-1H-pyrrolo[2,3-c]pyridin-2ylmethyl)-4-methylpentan-2-ol; 1,1,1-trifluoro-4-(5-fluoro-2-methoxyphenyl)-4-methyl-2-(4-methyl-1H-pyrrolo[2,3-c]pyridin-2-ylmethyl)pentan-2-ol; 2-(5,7-dichloro-1H-pyrrolo[2,3-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-(5-trifluoromethyl-1H-pyrrolo[2,3-c]pyridin-2-ylmethyl)pentan-2-ol; 1,1,1-trifluoro-4-(5-fluoro-2-methoxyphenyl)-2-(5-methoxy-1H-pyrrolo[2,3-c]pyridin-2-ylmethyl)-4-methylpentan-2-ol; 1,1,1-trifluoro-4-(5-fluoro-2-methylphenyl)-4-methyl-2-(4-methyl-1H-pyrrolo[2,3-c]pyridin-2-ylmethyl)pentan-2-ol; 1,1,1-trifluoro-4-(5-fluoro-2-methylphenyl)-2-(5-isopropoxy-1H-pyrrolo[2,3-c]pyridin-2-ylmethyl)-4-methylpentan-2-ol; 1,1,1-trifluoro-4-(5-fluoro-2-methylphenyl)-2-(5-methoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(5-methoxy-1H-pyrrolo[2,3-c]pyridin-2-ylmethyl)-4-methylpentan-2-ol; 1,1,1-trifluoro-4-(5-fluoro-2-methylphenyl)-2-(7-fluoro-1H-pyrrolo[2,3-c]pyridin-2-ylmethyl)-4-methylpentan-2-ol; 4-(5-chloro-2,3-dihydrobenzofuran-7-yl)-1-trifluoro-4-methyl-2-(5-trifluoromethyl-1H-pyrrolo[2,3-c]pyridin-2-ylmethyl)pentan-2-ol; 1,1,1-trifluoro-4-(5-fluoro-2-methylphenyl)-4-methyl-2-(5-trifluoromethyl-1H-pyrrolo[2,3-c]pyridin-2-ylmethyl)pentan-2-ol; 4-(5-chloro-2,3-dihydrobenzofuran-7-yl)-1,1,1-trifluoro-2-(5-isopropoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(7 -fluoro-1H-pyrrolo[2,3-c]pyridin-2-ylmethyl)-4-methylpentan-2-ol; 4-(5-chloro-2,3-dihydrobenzofuran-7-yl)-2-(5-dimethylamino-1H-pyrrolo[2,3-c]pyridin-2-ylmethyl)-1,1,1-trifluoro-4-methylpentan-2-ol; 4-(5-chloro-2,3-dihydrobenzofuran-7-yl)-1,1,1-trifluoro-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(5-morpholin-4-yl-1H-pyrrolo[2,3-c]pyridin-2-ylmethyl)pentan-2-ol; 1,1,1-trifluoro-4-(5-fluoro-2-methylphenyl)-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-2-(5-ethoxy-1H-pyrrolo[2,3-c]pyridin-2-ylmethyl)-1,1,1-trifluoro-4-methylpentan-2-ol; 2-(5-benzyloxy-1H-pyrrolo[2,3-c]pyridin-2-ylmethyl)-1,1,1-trifluoro-4-(5-fluoro-2-methylphenyl)-4-methylpentan-2-ol; 2-(5-benzyloxy-1H-pyrrolo[2,3-c]pyridin-2-ylmethyl)-4-(5-chloro-2,3-dihydrobenzofiran-7-yl)-1,1,1-trifluoro-4-methylpentan-2-ol; 1,1,1-trifluoro-4-(5-fluoro-2-methoxyphenyl)-2-(5-chloro-1H-pyrrolo[2,3-c-]pyridin-2-ylmethyl)-4-methylpentan-2-ol; 1,1,1-trifluoro-4-(5-fluoro-2-methoxyphenyl)-4-methyl-2-[5-(methylamino)-1H-pyrrolo[2,3-c]pyridin-2-ylmethyl]pentan-2-ol; 1,1,1-trifluoro-4-(5-fluoro-2-methoxyphenyl)-4-methyl-2-(5-amino-1H-pyrrolo[2,3-c]pyridin-2-ylmethyl)pentan-2-ol; 1,1,1-trifluoro-4-(5-fluoro-2-methylphenyl)-4-methyl-2-(6-amino-1H-pyrrol-o[2,3-c]pyridin-2-ylmethyl)pentan-2-ol; 4-(5-chloro-2,3-dihydrobenzofuran-7-yl)-1,1,1-trifluoro-2-(5-amino-1H-pyrrolo[2,3-c]pyridin-2-ylmethyl)-4-methylpentan-2-ol; 4-(5-chloro-2,3-dihydrobenzofuran-7-yl)-1,1,1-trifluoro-4-methyl-2-(5-methylamino-1H-pyrrolo[2,3-c]pyridin-2-ylmethyl)pentan-2-ol; 7-[4-(5-fluoro-2-methoxyphenyl)-2-hydroxy-4-methyl-2-trifluoromethylpentyl]-1-pyrrol[2,3-b]pyridin-7-ium chloride; 6-[4-(5-fluoro-2-methoxyphenyl)-2-hydroxy-4-methyl-2-trifluoromethylpentyl]-2-methyl-1H-pyrrolo[2,3-c]pyridin-6-ium chloride; 4-(5-bromo-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-methyl-4-(5-methyl-2,3-dihydrobenzofuran-7-yl)-2-(1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-b]pyridin-1-ylmethylpentan-2-ol; 1,1,1-trifluoro-4-(5-fluoro-2-methoxyphenyl)-4-methyl-2-(6-oxy-1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-c]pyridin-1-ylmethylpentan-2-ol; 2-benzo[b]thiophen-2-ylmethyl-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-thieno[2,3-c]pyridin-2-ylmethylpentan-2-ol; 1,1,1-trifluoro-4-(5-fluoro-2-methoxyphenyl)-2-indazol-1-ylmethyl-4-methylpentan-2-ol; 1,1,1-trifluoro-4-(5-fluoro-2-methoxyphenyl)-4-methyl-2-pyrazolo[1,5-a]pyridin-2-ylmethylpentan-2-ol; 4-(5-chloro-2,3-dihydrobenzofuran-7-yl)-2,4-dimethyl-1-thieno[2,3-c]pyridin-2-ylpentan-2-ol; 4-(5-fluoro-2-methylphenyl)-2,4-dimethyl-1-thieno[2,3-c]pyridin-2-ylpentan-2-ol; 1,1,1-trifluoro-4-(5-fluoro-2-methoxyphenyl)-2-furo[2,3-c]pyridin-2-ylmethy-1-4-methylpentan-2-ol; 4-(5-chloro-2,3-dihydrobenzofuran-7-yl)-1-furo[2,3-c]pyridin-2-yl-2,4-dimethylpentan-2-ol; 4-(5-fluoro-2-methylphenyl)-1-furo-[2,3-c]pyridin-2-yl-2,4-dimethylpentan-2-ol; 1,1,1-trifluoro-4-(5-fluoro-2-methylphenyl)-4-methyl-2-(1H-pyrrolo[3,2-c]pyridin-2-ylmethyl)pentan-2-ol- ; 1,1,1-trifluoro-4-methyl-4-(5-methyl-2,3-dihydrobenzofuran-7-yl)-2-(1H-pyrrolo[3,2-c]pyridin-2-ylmethyl)pentan-2-ol; 4-(5-chloro-2,3-dihydrobenzofuran-7-yl)-1,1,1-trifluoro-4-methyl-2-(1H-pyrrolo[3,2-c]pyridin-2-ylmethyl)pentan-2-ol; 4-(5-bromo-2,3-dihydrobenzofuran-7-yl)-1,1,1-trifluoro-4-methyl-2-(1H-pyrrolo[3,2-c]pyridin-2-ylmethyl)pentan-2-ol; 2-(3-dimethylaminomethyl-1H-pyrrolo[3,2-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-pyrrolo[3,2-c]pyridin-1-ylmethylpentan-2-ol; 1,1,1-trifluoro-4-(5-fluoro-2-methoxyphenyl)-4-methyl-2-pyrrolo[3,2-b]pyridin-1-ylmethylpentan-2-ol; 1,1,1-trifluoro-4-(5-fluoro-2-methoxyphenyl)-2-furo[3,2-c]pyridin-2-ylmethyl-4-methylpentan-2-ol; 4-(5-chloro-2.3-dihydrobenzofuran-7-yl)-1,1,1-trifluoro-4-methyl-2-pyrrolo[3,2-b]pyridin-1-ylmethylpentan-2-ol; 1,1,1-trifluoro-4-(5-fluoro-2-methoxyphenyl)-4-methyl-2-thieno[3,2-c]pyridin-2-ylmethylpentan-2-ol; 4-(5-chloro-2,3-dihydrobenzofuran-7-yl)-1,1,1-trifluoro-4-methyl-2-thieno[3,2-c]pyridin-2-ylmethylpentan-2-ol; 1,1,1-trifluoro-4-(5-fluoro-2-methylphenyl)-4-methyl-2-pyrrolo[3,2-b]pyridin-1-ylmethylpentan-2-ol; 1,1,1-trifluoro-4-(5-fluoro-2-methylphenyl)-4-methyl-2-thieno[3,2-c]pyridin-2-ylmethylpentan-2-ol; 4-fluoro-2-(4,4,4-trifluoro-3-hydroxy-1,1-dimethyl-3-thieno[3,2-c]pyridin-2-ylmethylbutyl)phenol; 4-fluoro-2-(4,4,4-trifluoro-3-furo[3,2-c]pyridin-2-ylmethyl-3-hydroxy-1,1-dimethylbutyl)phenol; 4-fluoro-2-(4,4,4-trifluoro-3-hydroxy-1,1-dimethyl-3-pyrrolo[3,2-b]pyridin-1-ylmethylbutyl)phenol; 2-[4-(5-fluoro-2-hydroxyphenyl)-2-hydroxy-4-methyl-2-trifluoromethylpentyl]-1H-indole-6-carboxylic acid; 2-[4-(5-fluoro-2-hydroxyphenyl)-2-hydroxy-4-methyl-2-trifluoromethylpentyl]-1H-indole-6-carboxylic acid dimethylamide; {2-[4-(5-fluoro-2-hydroxyphenyl)-2-hydroxy-4-methyl-2-trifluoromethylpentyl]-1H-indol-6-yl}morpholin-4-ylmethanone; 2-[4-(5-fluoro-2-methoxyphenyl)-2-hydroxy-4-methyl-2-trifluoromethylpentyl]-1H-indole-6-carboxylic acid dimethylamide; {2-[4-(5-fluoro-2-methoxyphenyl)-2-hydroxy-4-methyl-2-trifluoromethylpentyl]-1H-indol-6-yl}morpholin-4-ylmethanone; 2-[4-(5-fluoro-2-hydroxyphenyl)-2-hydroxy-4-methyl-2-trifluoromethylpentyl]-1H-indole-6-carboxylic acid amide; 2-[4-(5-fluoro-2-methoxyphenyl)-2-hydroxy-4-methyl-2-trifluoromethylpentyl]-1H-indole-6-carboxylic acid amide; 4-fluoro-2-[4,4,4-trifluoro-3-hydroxy-1,1-dimethyl-3-(5-nitro-1H-indol-2-ylmethyl)butyl]phenol; 2-[4-(5-fluoro-2-methoxyphenyl)-2-hydroxy-4-methyl-2-trifluoromethylpentyl]-1H-indole-6-carbonitrile; 2-[4-(5-fluoro-2-hydroxyphenyl)-2-hydroxy-4-methyl-2-trifluoromethylpentyl]-1H-indole-6-carbonitrile; N-{2-[4-(5-fluoro-2-methoxyphenyl)-2-hydroxy-4-methyl-2-trifluoromethylpentyl]-1H-indol-5-yl}acetamide; 1,1,1-trifluoro-4-(4-fluoro-2-methoxyphenyl)-2-(7-fluoro-4-methyl-1H-indo-1-2-ylmethyl)-4-methylpentan-2-ol; 5-fluoro-2-[4,4,4-trifluoro-3-(7-fluoro-4-methyl-1H-indol-2-ylmethyl)-3-hydroxy-1,1-dimethylbutyl]phenol; 2-[4-(3-[1,3]dioxolan-2-ylphenyl)-2-hydroxy-4-methyl-2-trifluoromethylpentyl]-1H-indole-5-carbonitrile; 2-[4-(5-fluoro-2-methoxyphenyl)-2-hydroxy-4-methyl-2-trifluoromethylpentyl]-1H-indole-5-carboxylic acid-2-trimethylsilanylethyl ester; 2-[4-(5-fluoro-2-methoxyphenyl)-2-hydroxy-4-methyl-2-trifluoromethylpentyl]-1H-indole-5-carboxylic acid; 2-[4-(4-fluoro-2-hydroxyphenyl)-2-hydroxy-4-methyl-2-trifluoromethylpenty-1]-4-methyl-1H-indole-6-carbonitrile; {2-[4-(5-Fluoro-2-methoxyphenyl)-2-hydroxy-4-methyl-2-trifluoromethylpentyl]-1H-indol-5-yl}piperidin-1-ylmethanone; 2-[4-(5-fluoro-2-methoxyphenyl)-2-hydroxy-4-methyl-2-trifluoromethylpentyl]-1H-indole-5-carboxylic acid methylamide; {2-[4-(5-fluoro-2-methoxyphenyl)-2-hydroxy-4-methyl-2-trifluoromethylpentyl]-1H-indol-5-yl}pyrrolidin-1-ylmethanone; 1-{2-[4-(5-fluoro-2-methoxyphenyl)-2-hydroxy-4-methyl-2-trifluoromethytpentyl]1H-indole-5-carbonyl}piperidin-4-one; 2-[4-(5-fluoro-2-methoxyphenyl)-2-hydroxy-4-methyl-2-trifluoromethylpentyl]-1H-indole-5-carboxylic acid (2-hydroxyethyl)amide; {2-[4-(5-fluoro-2-methoxyphenyl)-2-hydroxy-4-methyl-2-trifluoromethylpentyl]-1H-indol-5-yl}(4-hydroxypiperidin-1-yl)methanone; {2-[4-(5-fluoro-2-methoxyphenyl)-2-hydroxy-4-methyl-2-trifluoromethylpentyl]-1H-indol-5-yl}(3-hydroxypyrrolidin-1-yl)methanone; 2-[4-(5-fluoro-2-methoxyphenyl)-2-hydroxy-4-methyl-2-trifluoromethylpentyl]-1H-indole-5-carboxylic acid cyanomethylamide; 2-[4-(5-fluoro-2-methoxyphenyl)-2-hydroxy-4-methyl-2-trifluoromethylpentyl]-1H-indole-5-carboxylic acid (2-dimethylaminoethyl)amide; {2-[4-(5-fluoro-2-methoxyphenyl)-2-hydroxy-4-methyl-2-trifluoromethylpentyl]-1H-indol-5-yl}(4-methylpiperazin-1-yl)methanone; ({2-[4-(5-fluoro-2-methoxyphenyl)-2-hydroxy-4-methyl-2-trifluoromethylpentyl]-1H-indole-5-carbonyl}amino)acetic acid methyl ester; 2-[4-(5-fluoro-2-methoxyphenyl)-2-hydroxy-4-methyl-2-trifluoromethylpentyl]-1H-indole-5-carboxylic acid carbamoylmethylamide; 4-({2-[4-(5-fluoro-2-methoxyphenyl)-2-hydroxy-4-methyl-2-trifluoromethylpentyl]-1H-indole-5-carbonyl}amino)butyric acid methyl ester; ({2-[4-(5-fluoro-2-methoxyphenyl)-2-hydroxy-4-methyl-2-trifluoromethylpentyl]-1H-indole-5-carbonyl}amino)acetic acid; 4-({2-[4-(5-fluoro-2-methoxyphenyl)-2-hydroxy-4-methyl-2-trifluoromethylpentyl]-1H-indole-5-carbonyl}amino)butyric acid; 2-[4-(3-dimethylaminomethylphenyl)-2-hydroxy-4-methyl-2-trifluoromethylpentyl]-1H-indole-5-carbonitrile; 4-fluoro-2-[4,4,4-trifluoro-3-hydroxy-1,1-dimethyl-3-(5-trifluoromethyl-1H-indol-2-ylmethyl)butyl]phenol; 2-[4-(5-bromo-2,3-dihydrobenzofuran-7-yl)-2-hydroxy-4-methyl-2-trifluoromethylpentyl]-4-methyl-1H-indole-6-carbonitrile; 2-[2-hydroxy-4-(5-methanesulfonyl-2,3-dihydrobenzofuran-7-yl)-4-methyl-2-trifluoromethylpentyl]-4-methyl-1H-indole-6-carbonitrile; 2-[4-(5-bromo-2,3-dihydrobenzofuran-7-yl)-2-hydroxy-4-methyl-2-trifluoromethylpentyl]-1H-indole-5-carboxylic acid; 2-[4-(5-bromo-2,3-dihydrobenzofuran-7-yl)-2-hydroxy-4-methyl-2-trifluoromethylpentyl]-1H-indole-5-carboxylic acid amide; 2-[4-(5-bromo-2,3-dihydrobenzofuran-7-yl)-2-hydroxy-4-methyl-2-trifluoromethylpentyl]-1H-indole-5-carboxylic acid dimethylamide; 2-[4-(5-Bromo-2,3-dihydrobenzofuran-7-yl)-2-hydroxy-4-methyl-2-trifluoromethylpentyl]-1H-indole-5-carboxylic acid cyanomethylamide; {2-[4-(5-bromo-2,3-dihydrobenzofuran-7-yl)-2-hydroxy-4-methyl-2-trifluoromethylpentyl]-1H-indol-5-yl}pyrrolidin-1-ylmethanone; {2-[4-(5-bromo-2,3-dihydrobenzofuran-7-yl)-2-hydroxy-4-methyl-2-trifluoro-methylpentyl]-1H-indol-5-yl}morpholin-4-ylmethanone; 2-[4-(5-fluoro-2-methoxyphenyl)-2-hydroxy-4-methyl-2-trifluoromethylpentyl]- I H-indole-5-carboxylic acid amide; {2-[4-(5-fluoro-2-methoxyphenyl)-2-hydroxy-4-methyl-2-trifluoromethylpentyl]-1H-indol-5-yl}morphotin-4-ylmethanone; 2-(4-benzo[1,3]dioxol-4-yl-2-hydroxy-4-methyl-2-trifluoromethylpentyl)-4-methyl-1H-indole-6-carbonitrile; 1,1,1-trifluoro-4-methyl-4-phenyl-2-quinolin-4-ylmethylhexan-2-ol; 2-[2-hydroxy-4-methyl-4-(5-methylsulfanyl-2-,3-dihydrobenzofuran-7-yl)-2-trifluoromethylpentyl]-1H-indole-3-carbonitrile; 7-(4,4,4-trifluoro-3-hydroxy-1,1-dimethyl-3-quinolin-4-ylmethylbutyl)-2,3-dihydrobenzofuran-5-carbonitrile; 2-[2-hydroxy-4-(5-methanesulfonyl-2,3-dihydrobenzofuran-7-yl)-4-methyl-2-trifluoromethylpentyl]-1H-indole-3-carbonitrile; 2-[2-hydroxy-4-(2-hydroxy-5-methylphenyl)-4-methyl-2-trifluoro-methylpentyl]-4-methyl-1H-indole-6-carbonitrile; 1,1,1-trifluoro-4-(5-fluoro-2,3-dihydrobenzofuran-7-yl)-4-methyl-2-(5-methylsulfanyl-1H-indol-2-ylmethyl)pentan-2-ol; 2-[2-hydroxy-4-(2-methoxy-5-methylsulfanylphenyl)-4-methyl-2-trifluoromethylpentyl]-1H-indole-3-carbonitrile; 2-[2-Hydroxy-4-(5-methanesulfonyl-2-methoxyphenyl)-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-5-sulfonic acid dimethylamide; 1,1,1-trifluoro-4-(5-fluoro-2,3-dihydrobenzofuran-7-y-1)-4-methyl-2-(5-phenyl-1H-indol-2-ylmethyl)pentan-2-ol; 2-[4-(5-tert-butyl-2-hydroxyphenyl)-2-hydroxy-4-methyl-2-trifluoromethylpentyl]-1H-indole-3-carbonitrile; 2-[2-hydroxy-4-(2-hydroxy-5-isopropylphenyl)-4-methyl-2-trifluoromethylpentyl]-1H-indote-3-carbonitrile; 2-[2-hydroxy-4-(2-hydroxy-3,5-dimethylphenyl)-4-methyl-2-trifluoromethylpentyl]-1H-indole-3-carbonitrile; 2-[2-hydroxy-4-(5-hydroxy-2,4-dimethylphenyl)-4-methyl-2-trifluoromethylpentyl]-1H-indole-3-carbonitrile; 2-[4-(5-tert-butyl-2-methoxyphenyl)-2-hydroxy-2-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1-methyl-1H-indole-3-carbonitrile; 2-[2-hydroxy-4-(5-isopropyl-2-methoxyphenyl)-4-methyl-2-trifluoromethylpentyl]-1H-indole-3-carbonitrile; 2-[2-hydroxy-4-(5-isopropyl-2-methoxyphenyl)-4-methyl-2-trifluoromethylpentyl]-1-methyl-1H-indole-3-carbonitrile; 2-[2-hydroxy-4-(2-hydroxy-5-methanesulfonylphenyl)-4-methyl-2-trifluoromethylpentyl]-1H-indole-3-carbonitrile; 2-[2-hydroxy-4-(2-methoxy-5-methylphenyl)-4-methyl-2-trifluoromethylpentyl]-4-methyl-IH-indole-6-carbonitrile; 1,1,1-trifluoro-4-methyl-2-quinolin-4-ylmethyl-4-o-tolylpentan-2-ol; 1,1,1-trifluoro-4-methyl-2-quinolin-4-ylmethyl-4-m-tolylpentan-2-ol; 1,1,1-trifluoro-4-(2-fluorophenyl)-2-(1H-indol-2-ylmethyl)-4-methylpentan-2-ol; 1,1,1-trifluoro-4-(2-fluorophenyl)-4-methyl-2-quinolin-4-ylmethylpentan-2-ol; 1,1,1-trifluoro-4-(3-fluorophenyl)-2-(1H-indol-2-ylmethyl)-4-methylpentan-2-ol; 1,1,1-trifluoro-4-(3-fluorophenyl)-4-methyl-2-quinolin-4-ylmethylpentan-2-ol; 1,1,1-trifluoro-4-(4-fluorophenyl)-2-(1H-indol-2-ylmethyl)-4-methylpentan-2-ol; 1,1,1-trifluoro-4-(4-fluorophenyl)-4-methyl-2-quinolin-4-ylmethylpentan-2-ol; 3-(4,4,4-trifluoro-3-hydroxy-1,1-dimethyl-3-quinolin-4-ylmethylbutyl)phenol; 1,1,1-trifluoro-4-methyl-2-quinolin-4-ylmethyl-4-(2-trifluoromethylphenyl)pentan-2-ol: 1,1,1-trifluoro-2-(1H-indol-2-ylmethyl)-4-methyl-4-(4-trifluoromethylphenyl)pentan-2-ol; 1,1,1-trifluoro-4-methyl-2-quinolin-4-ylmethyl-4-(4-trifluoromethylphenyl)pentan-2-ol; 4-(3-chlorophenyl)-1,1,1-trifluoro-2-(1H-indol-2-ylmethyl)-4-methylpentan-2-ol; 4-(3-chlorophenyl)-1,1,1,-trifluoro-4-methyl-2-quinolin-4-ylmethylpentan-2-ol; 4-(4-dimethylaminophenyl)-1,1,1-trifluoro-2-(1H-indol-2-ylmethyl)-4-methylpentan-2-ol; 4-biphenyl-3-yl-1,1,1-trifluoro-4-methyl-2-quinolin-4-ylmethylpentan-2-ol; 4-(3-bromophenyl)-1,1,1-trifluoro-2-(1H-indol-2-ylmethyl)-4-methylpentan-2-ol; 4-(2-difluoromethoxy-5-fluorophenyl)-1,1,1-trifluoro-2-(1H-indol-2-ylmethyl)-4-methylpentan-2-ol; 4-biphenyl-3-yl-1,1,1-trifluoro-2-(1H-indol-2-ylmethyl)-4-methylpentan-2-ol; 4-(4-dimethylaminophenyl)-1,1,1-trifluoro-4-methyl-2-quinolin-4-ylmethylpentan-2-ol; 2-[4-(5-fluoro-2-methylphenyl)-2-hydroxy-4-methyl-2-trifluoromethylpentyl]-1,6-dihydropyrrolo[2,3-c]pyridin-5-one; 2-[4-(5-Fluoro-2-methylphenyl)-2-hydroxy-4-methyl-2-trifluoromethylpentyl]-6-methyl-1,6-dihydropyrrolo[2,3-c]pyridin-5-one; 2-[4-(5-fluoro-2-methyl-phenyl)-2-hydroxy-4-methyl-2-trifluoromethylpentyl]-4-methyl-1,4-dihydropyrrolo[3,2-b]pyridin-5-one; 1,1,1-trifluoro-4-(5-fluoro-2-methylphenyl)-2-(6-methoxy-1H-pyrrolo[3,2-c]pyridin-2-ylmethyl)-4-methylpentan-2-ol; 2-[4-(5-fluoro-2-methylphenyl)-2-hydroxy-4-methyl-2-trifluoromethylpentyl]-5-methyl-1,5-dihydropyrrolo[3,2-c]pyridin-6-one; 2-[4-(5-fluoro-2-methyl-phenyl)-2-hydroxy-4-methyl-2-trifluoromethylpentyl]-1,3a-dihydropyrrolo[3,-2-c]pyridin-6-one; 2-[4-(5-fluoro-2-methylphenyl)-2-hydroxy-4-methyl-2-trifluoromethylpentyl]-1,7-dihydropyrrolo[3,2-c]pyridine-4,6-dione; 6-[4-(5-fluoro-2-methylphenyl)-2-hydroxy-4-methyl-2-trfluoromethylpentyl]-3-methyl-1,7-dihydropyrrolo[2,3-d]pyrimidine-2,4-dione; 2-[4-(5-chloro-2,3-dihydrobenzofuran-7-yl)-2-hydroxy-4-methyl-2-trifluoro- methylpentyl]-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-6-methyl-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-1,4-dihydropyrrolo[3,2-b]pyridin-5-one; 2-[4-(5-chloro-2,3-dihydrobenzofiran-7-yl)-2-hydroxy-4-methyl-2-trifluoromethylpentyl]-4-methyl-1,4-dihydropyrrolo[3,2-b]pyridin-5-one; 2-[4-(5-chloro-2,3-dihydrobenzofuran-7-yl)-2-hydroxy-4-methyl-2-trifluoro-methylpentyl]-1,5-dihydropyrrolo[3,2-c]pyridin-6-one; 2-[4-(5-chloro-2,3-dihydrobenzofuran-7-yl)-2-hydroxy-4-methyl-2-trifluoromethylpentyl]-5-methyl-1,5-dihydropyrrolo[3,2-c]pyridin-6-one; 4-(5-chloro-2,3-dihydrobenzofuran-7-yl)-1,1,1-trifluoro-2-(6-methoxy-5,6-dihydro-1H-pyrrolo[3,2-c]pyridin-2-ylmethyl)-4-methylpentan-2-ol; 2-[4-(5-chloro-2,3-dihydrobenzofuran-7-yl)-2-hydroxy-4-methyl-2-trifluoromethylpentyl]-1,7-dihydropyrrolo[3,2-c]pyridine-4,6-dione; 6-[4-(5-chloro-2,3-dihydrobenzofuran-7-yl)-2-hydroxy-4-methyl-2-trifluoromethylpentyl]-3-methyl-1,7-dihydropyrrolo[2,3-d]pyrimidine-2,4-dione; 2-[4-(3-dimethylaminomethylphenyl)-2-hydroxy-4-methyl-2-trifluoromethylpentyl]-1H-indole-5-carbonitrile; 1,1,1-trifluoro-2-(1H-indol-2-ylmethyl)-4-methyl-4-(3-morpholin-4-ylmethylphenyl)pentan-2-ol; 1,1,1-trifluoro-4-methyl-4-(3-morpholin-4-ylmethylphenyl)-2-(1H-pyrrolo[2-,3-d]pyridazin-2-ylmethyl)pentan-2-ol; 1,1,1-trifluoro-4-(5-fluoro-2-methylphenyl)-4-methyl-2-(5-morpholin-4-ylmethyl-1H-indol-2-ylmethyl)pentan-2-ol; 1,1,1-trifluoro-4-(5-fluoro-2-methylphenyl)-4-methyl-2-(5-morpholin-4-ylmethyl-1H-pyrrolo[2,3-c]pyridin-2-ylmethyl)pentan-2-ol; {2-[4-(5-fluoro-2-methylphenyl)-2-hydroxy-4-methyl -2-trifuoromethylpentyl]-1H-indol-5-yl}phenylmethanone; {2-[4-(5-fluoro-2-methylphenyl)-2-hydroxy-4-methyl-2-trifluoromethylpenty-1]-1H-pyrrolo[2,3-c]pyridin-5-yl} phenylmethanone; {2-[4-(5-fluoro-2-methylphenyl)-2-hydroxy-4-methyl-2-trifluoromethylpentyl]-1H-indol-5-yl}furan-2-ylmethanone; {2-[4-(5-fluoro-2-methylphenyl)-2-hydroxy-4-methyl-2-trifluoromethylpentyl]-1H-pyrrolo[2,3-c]pyridin-5-yl}furan-2-ylmethanone; 1,1,1-trifluoro-2-(1H-indol-2-ylmethyl)-4-methyl-4-pyridin-2-ylpentan-2-ol; 1,1,1-trifluoro-4-methyl-4-pyridin-4-yl-2-quinolin-4-ylmethylpentan-2-ol; 2-(2,6-dimethylpyridin-4-ylmethyl)-1,1,1-trifluoro-4-(5-fluoro-2-methoxyphenyl)-4-methylpentan-2-ol; 2-[3-(2,6-dimethylpyridin-4-ylmethyl)-4,4,4-trifluoro-3-hydroxy-1,1-dimethylbutyl]-4-fluorophenol; 1,1,1-trifluoro-4,4-dimethyl-5-phenyl-2-quinolin-4-ylmethylpentan-2-ol; 1,1,1-trifluoro-4-(5-fluoro-2-methoxyphenyl)-4-methyl-2-pyridin-4-ylmethylpentan-2-ol; 4-fluoro-2-[4,4,4-trifluoro-3-(2-fluoropyridin-4-ylmethyl)-3-hydroxy-1,1-dimethylbutyl]phenol; 2-[3-(2-bromopyridin-4-ylmethyl)-4,4,4-trifluoro-3-hydroxy-1,1-dimethylbutyl]-4-fluorophenol; 2-(6,8-dimethylquinolin-4-ylmethyl)-1,1,1-trifluoro-4-(5-fluoro-2-methoxy- phenyl)-4-methylpentan-2-ol; 4-[4-(5-fluoro-2-methoxyphenyl)-2-hydroxy-4-methyl-2-trifluoromethylpentyl]pyridine-2-carbonitrile; 2,6-dichloro-4-[4-(5-fluoro-2-methoxyphenyl)-2-hydroxy-4-methyl-2-trifluoromethylpentyl]nicotinonitrile; 4-[4-(5-fluoro-2-methoxyphenyl)-2-hydroxy-4-methyl-2-trifluoromethylpentyl]quinolin-2-ol; 2,6-dichloro-4-[4-(5-fluoro-2-hydroxyphenyl)-2-hydroxy-4-methyl-2-trifluoromethylpentyl]nicotinonitrile; 2-(2-chloro-8-methylquinolin-4-ylmethyl)-1,1,1-trifluoro-4-(5-fluoro-2-methoxyphenyl)-4-methylpentan-2-ol; 2-(2,6-dichloroquinolin-4-ylmethyl)-1,1,1-trifluoro-4-(5-fluoro-2-methoxyphenyl)-4-methylpentan-2-ol; 2-[3-(2-chloro-8-methylquinolin-4-ylmethyl)-4,4,4-trifluoro-3-hydroxy-1,1-dimethylbutyl]-4-fluorophenol; 2-[3-(2,6-dichloroquinolin-4-ylmethyl)-4,4,4-trifluoro-3-hydroxy-1,1-dimethylbutyl]-4-fluorophenol; 4-(2,3-dihydrobenzofuran-7-yl)-2-(2,6-dimethylpyridin-4-ylmethyl)-1,1,1-trifluoro-4-methylpentan-2-ol; 2-(2,6-dimethylpyridin-4-ylmethyl)-1,1,1-trifluoro-4-(3-fluorophenyl)-4-methylpentan-2-ol; 2-(2,6-dimethylpyridin-4-ylmethyl)-1,1,1-trifluoro-4-(4-fluorophenyl)-4-methylpentan-2-ol; 1,1,1-trifluoro-4-(5-fluoro-2-methylphenyl)-4-methyl-2-quinolin-4-ylmethylpentan-2-ol; 2-(2,6-dimethylpyridin-4-ylmethyl)-1,1,1-trifluoro-4-(5-fluoro-2-methylphenyl)-4-methylpentan-2-ol; 2-(2,6-dimethylpyridin-4-ylmethyl)-1,1,1-trifluoro-4-methyl-4-m-tolylpentan-2-ol; 1,1,1-trifluoro-4-(5-fluoro-2-methoxyphenyl)-4-methyl-2-(2-methylquinolin-4-ylmethyl)pentan-2-ol; 4-fluoro-2-(4,4,4-trifluoro-3-hydroxy-1,1,1-dimethyl-3-quinolin-4-ylmethylbutyl)phenol; 4-fluoro-2-[4,4,4-trifluoro-3-hydroxy-1,1-dimethyl-3-(2-methylquinolin-4-ylmethyl)butyl]phenol; 2-(2,6-dimethylpyridin-4-ylmethyl)-1,1,1-trifluoro-4-(4-fluoro-2-methoxyphenyl)-4-methylpentan-2-ol; 1,1,1-trifluoro-4-(5-fluoro-2-methoxyphenyl)-4-methyl-2-(7-methylquinolin-4-ylmethyl)pentan-2-ol;2-[3-(2,6-dimethylpyridin-4-ylmethyl)-4,4,4-trifluoro-3-hydroxy-1,1-dimethylbutyl]-5-fluorophenol; and 2-(5,7-dimethylquinolin-4-ylmethyl)-1,1,1-trifluoro-4-(5-fluoro-2-methoxyphenyl)-4-methylpentan-2-ol.

In still another embodiment, said at least a DIGRA has Formula I, wherein
(a) A is an aryl or heteroaryl group, each optionally independently substituted with one to three substituent groups, which are independently selected from the group consisting _{O}f C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino. C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl;
(c) R³ is hydrogen, C₁-C₈ alkyl, C₂-C₈ alkenyl, C₁-C₈ alkynyl, carbocycle, heterocyclyl, aryl, heteroaryl, carbocycle-C₁-C₈ alkyl, carboxy, alkoxycarbonyl, aryl-C₁-C₈ alkyl, aryl-C₁-C₈ haloalkyl, heterocyclyl-C₁-C₈ alkyl, heteroaryl-C₁-C₈ alkyl, carbocycle-C₂-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone, wherein R³ cannot be trifluoromethyl;
(d) B is C₁-C₅ alkylene, C₂-C₅ alkenylene, or C₂-C₅ alkynylene, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q comprises a heteroaryl group optionally independently substituted with one to three substituent groups, which are independently selected from the group consisting of C₁-C₅ alkyl, C₁-C₅ alkenyl, C₁-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, acyl, C₁-C₃ silanyloxy, C₁-C₅ alkoxycarbonyl, carboxy, halogen, hydroxy, oxo, cyano, heteroaryl, heterocyclyl, amino wherein the nitrogen atom is optionally independently mono- or di-substituted by C₁-C₅ alkyl or aryl, ureido wherein either nitrogen atom is optionally independently substituted with C₁-C₅ alkyl, or trifluoromethyl.

Non-limiting examples of these compounds include 2-cyclopropyl-4-(5-fluoro-2-methoxyphenyl)-4-methyl-1-(1H-pyrrolo[3,2-c]pyridin-2-yl)pentan-2-ol; 4-(5-fluoro-2-methoxyphenyl)-2-hydroxy-4-methyl-2-(1H-pyrrolo[2,3-c]pyridin-2-ylmethyl)pentanoic acid; 4-(5-fluoro-2-methoxyphenyl)-2-hydroxy-4-methyl-2-(1H-pyrrolo[2,3-c]pyridin-2-ylmethyl)pentanoic acid methyl ester; 2-cyclopropyl-4-(5-fluoro-2-methylphenyl)-4-methyl-1-(1H-pyrrolo[2,3-c]pyridin-2-yl)pentan-2-ol; 4-(5-chloro-2,3-dihydrobenzofuran-7-yl)-2-cyclopropyl-4-methyl-1-(1H-pyrrolo[2,3-c]pyridin-2-yl)pentan-2-ol; 2-cyclopropyl-4-(5-fluoro-2-methylphenyl)-4-methyl-1-(1H-pyrrolo[3,2-c]pyridin-2-yl)pentan-2-ol; 4-(5-chloro-2,3-dihydrobenzofuran-7-yl)-2-cyclopropyl-4-methyl-1-(1H-pyrrolo[3,2-c]pyridin-2-yl)pentan-2-ol; 4-(5-fluoro-2-methoxyphenyl)-2,4-dimethyl-1-(1H-pyrrolo[2,3-c]pyridin-2-yl)pentan-2-ol; 5-(5-fluoro-2-methoxyphenyl)-2,5-dimethyl-3-(1H-pyrrolo[2,3-c]pyridin-2-ylmethyl)hexan-3-ol; 5-(5-fluoro-2-methoxyphenyl)-2,2,5-trimethyl-3-(1H-pyrrolo[2,3-c]pyridin-2-ylmethyl)hexan-3-ol; 2-cyclohexyl-4-(5-fluoro-2-methoxyphenyl)-4-methyl-1-(1H-pyrrolo[2,3-c]pyridin-2-yl)pentan-2-ol; 2-cyclopentyl-4-(5-fluoro-2-methoxyphenyl)-4-methyl-1-(1H-pyrrolo[2,3-c]pyridin-2-yl)pentan-2-ol; 5-(5-fluoro-2-methoxyphenyl)-5-methyl-3-(1H-pyrrolo[2,3-c]pyridin-2-ylmethyl)hexan-3-ol; 2-(5-fluoro-2-methoxyphenyl)-2,6-dimethyl-4-(1H-pyrrolo[2,3-c]pyridin-2-ylmethyl)heptan-4-ol; 2-(5-fluoro-2-methoxyphenyl)-2,5,5-trimethyl-4-(1H-pyrrolo[2,3-c]pyridin-2-ylmethyl)heptan-4-ol; 1,1-difluoro-4-(5-fluoro-2-methoxyphenyl)-4-methyl-2-(1H-pyrrolo[2,3-c]pyridin-2-ylmethyl)pentan-2-ol; 1-cyclohexyl-4-(5-fluoro-2-methoxyphenyl)-4-methyl-2-(1H-pyrrolo[2,3-c]pyridin-2-ylmethyl)pentan-2-ol; 5-(5-fluoro-2-methylphenyl)-2,5-dimethyl-3-(1H-pyrrolo[2,3-c]pyridin-2-ylmethyl)hexan-3-ol; 5-(5-fuoro-2-methylphenyl-)-2,2,5-trimethyl-3-(1H-pyrrolo[2,3-c]pyridin-2-ylmethyl)hexan-3-ol; 5-(5-chloro-2,3-dihydrobenzofuran-7-yl)-2,5-dimethyl-3-(1H-pyrrolo[2,3-c]pyridin-2-ylmethyl)hexan-3-ol; 2-cyclobutyl-4-(5-fluoro-2-methoxyphenyl)-4-methyl-1-(1H-pyrrolo[2,3-c]pyridin-2-yl)pentan-2-ol; 2-(5-fluoro-2-methoxyphenyl)-2,6,6-trimethyl-4-(1H-pyrrolo[2,3-c]pyridin-2-ylmethyl)heptan-4-ol; 5-(5-fluoro-2-methoxyphenyl)-5-methyl-3-(1H-pyrrolo[2,3-c]pyridin-2-ylmethyl)hex-1-en-3-ol, 5-(5-fluoro-2-methoxyphenyl)-5-methyl-3-(1H-pyrrolo[2,3-c]pyridin-2-ylmethyl)hex-1-yn-3-ol; 1-fluoro-4-(5-fluoro-2-methoxyphenyl)-4-methyl-2-(1H-pyrrolo[2,3-c]pyridin-2-ylmethyl)pentan-2-ol; 2,2-difluoro-5-(5-fluoro-2-methoxyphenyl)-5-methyl-3-(1H-pyrrolo[2,3-c]pyridin-2-ylmethyl)hexan-3-ol; 2-fluoro-5-(5-fluoro-2-methoxyphenyl)-2,5-dimethyl-3-(1H-pyrrolo[2,3-c]pyridin-2-ylmethyl)hexan-3-ol; 2-fluoro-5-(5-fluoro-2-methoxyphenyl)-5-methyl-3-(1H-pyrrolo[2,3-c]pyridin-2-ylmethyl)hexan-3-ol; 5-(5-fluoro-2-methoxyphenyl)-2,5-dimethyl-3-(1H-pyrrolo[2,3-c]pyridin-2-ylmethyl)hex-1-en-3-ol; 1,1,1-trifluoro-5-(5-fluoro-2-methoxyphenyl)-5-methyl-3-(1H-pyrrolo[2,3-c]pyridin-2-ylmethyl)hexan-3-ol; 4-(5-fluoro-2-methoxyphenyl)-4-methyl-2-phenyl-1-(1H-pyrrolo[2,3-c]pyridin-2-yl)pentan-2-ol; 5-(5-chloro-2,3-dihydrobenzofuran-7-yl)-2,2,5-trimethyl-3-(1H-pyrrolo[2,3-c]pyridin-2-ylmethyl)hexan-3-ol; 5-(5-fluoro-2-methylphenyl)-2,2.5-trimethyl-3-thieno[2,3-c]pyridin-2-ylmethylhexan-3-ol; 1,1-difluoro-4-(5-fluoro-2-methoxyphenyl)-4-methyl-2-(1H-pyrrolo[3,2-c]pyridin-2-ylmethyl)pentan-2-ol; 5-(5-fluoro-2-methoxyphenyl)-2,5-dimethyl-3-(1H-pyrrolo[3,2-c]pyridin-2-ylmethyl)hexan-3-ol; 5-(5-fluoro-2-methoxyphenyl)-2,2,5-trimethyl-3-(1H-pyrrolo[3,2-c]pyridin-2-ylmethyl)hexan-3-ol; 2-(1-fluorocyclopropyl)-4-(5-fluoro-2-methoxyphenyl)-4-methyl-1-(1H-pyrrolo[2,3-c]pyridin-2-yl)pentan-2-ol; 2-(1-fluorocyclopropyl)-4-(4-fluorophenyl)-4-methyl-1-quinolin-4-ylpentan-2-ol; 2-[4,4-difluoro-3-hydroxy-1,1-dimethyl-3-(1H-pyrrolol[3,2-c]pyridin-2-ylmethyl)butyl]-4-fluorophenol; 5-(5-chloro-2,3-dihydrobenzofuran-7-yl)-2,5-dimethyl-3-(1H-pyrrolo [3,2-c]pyridin-2-ylmethyl)hexan-3-ol; 5-(5-fluoro-2-methylphenyl)-2,5-dimethyl-3-(1H-pyrrolo[3,2-c]pyridin-2-ylmethyl)hexan-3-ol; 5-(5-fluoro-2-methylphenyl)-2,2,5-trimethyl-3-(1H-pyrrolo[3,2-c]pyridin-2-ylmethyl)hexan-3-ol; 4-(5-chloro-2,3-dihydrobenzofuran-7-yl)-1,1-difluoro-4-methyl-2-(1H-pyrrolo[3,2-c]pyridin-2-ylmethyl)pentan-2-ol; 4-(5-chloro-2,3-dihydrobenzofuran-7-yl)-1,1-difluoro-4-methyl-2-pyrrolo[3,2-b]pyridin-1-ylmethylpentan-2-ol; 5-(5-chloro-2,3-dihydrobenzofuran-7-yl)-2,2,5-trimethyl-3-(1H-pyrrolo[3,2-c]pyridin-2-ylmethyl)hexan-3-ol; 5-(5-fluoro-2-methylphenyl)-2,2,5-trimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(5-phenyl-1H-pyrrolo[2,3-c]pyridin-2-ylmethyl)hexan-3-ol; 5-(5-fluoro-2-methylphenyl)-2,2,5-trimethyl-3-(5-phenyl-1H-pyrrolo[2,3-c]pyridin-2-ylmethyl)hexan-3-ol; 5-(5-fluoro-2-methylphenyl)-2,5-dimethyl-3-(5-phenyl-1H-pyrrolo[2,3-c]pyridin-2-ylmethyl)hexan-3-ol; 5-(5-fluoro-2-methylphenyl)-5-methyl-3-(5-phenyl-1H-pyrrolo[2,3-c]pyridin-2-ylmethyl)hexan-3-ol; 4-(5-fluoro-2-methylphenyl)-2,4-dimethyl-1-(5-phenyl-1H-pyrrolo[2,3-c]pyridin-2-yl)pentan-2-ol; 4-(5-chloro-2,3-dihydrobenzofuran-7-yl)-1,1-difluoro-4-methyl-2-(6-methyl-1H-pyrrolo[3,2-c]pyridin-2-ylmethyl)pentan-2-ol; 5-(5-fluoro-2-methylphenyl)-2,5-dimethyl-3-(5-pyridin-3-yl-1H-pyrrolo[2,3-c]pyridin-2-ylmethyl)hexan-3-ol; 5-(5-chloro-2,3-dihydrobenzofuran-7-yl)-5-methyl-3-(5-phenyl-1H-pyrrolo[2,3-c]pyridin-2-ylmethyl)hexan-3-ol; 4-(5-chloro-2,3-dihydrobenzofuran-7-yl)-2,4-dimethyl-1-(5-phenyl-1H-pyrrolo[2,3-c]pyridin-2-yl)pentan-2-ol; 1,1-difluoro-4-(5-methanesulfonyl-2,3-dihydrobenzofuran-7-yl)-4-methyl-2-(1H-pyrrolo[2,3-c]pyridin-2-ylmethyl)pentan-2-ol; 5-(5-chloro-2,3-dihydrobenzofuran-7-yl)-2,5-dimethyl-3-(5-pyridin-3-yl-1H-pyrrolo[2,3-c]pyridin-2-ylmethyl)hexan-3-ol; 2-(5-bromo-1H-indol-2-ylmethyl)-1,1-difluoro-4-(5-methanesulfonyl-2,3-dihydrobenzofuran-7-yl)-4-methylpentan-2-ol; and 2-[2-difluoromethyl-2-hydroxy-4-(5-methanesulfonyl-2,3-dihydrobenzofuran-7-yl)-4-methylpentyl]-4-methyl-1H-indole-6-carbonitrile.

In still another embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently C₁-C₅ alkyl, wherein one or both are independently substituted with hydroxy, C₁-C₅ alkoxy, C₁-C₅ alkylthio wherein the sulfur atom is optionally oxidized to a sulfoxide or sulfone, amino wherein the nitrogen atom is optionally independently mono- or di-substituted by C₁-C₅ alkyl or aryl;
(c) R³ is hydrogen, C₁-C₈ alkyl, C₂-C₈ alkenyl, C₂-C₈ alkynyl, carbocycle, heterocyclyl, aryl, heteroaryl, carbocycle-C₁-C₈ alkyl, carboxy, alkoxycarbonyl, aryl-C₁-C₈ alkyl, aryl-C₁-C₈ haloalkyl, heterocyclyl-C₁-C₈ alkyl, heteroaryl-C₁-C₈ alkyl, carbocycle-C₂-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone;
(d) B is C₁-C₅ alkylene, C₂-C₅ alkenylene, or C₂-C₅ alkynylene, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q comprises a heteroaryl group optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, acyl, C₁-C₃ silanyloxy, C₁-C₅ alkoxycarbonyl, carboxy, halogen, hydroxy, oxo, cyano, heteroaryl, heterocyclyl, amino wherein the nitrogen atom is optionally independently mono- or di-substituted by C₁-C₅ alkyl or aryl, ureido wherein either nitrogen atom is optionally independently substituted with C₁-C₅ alkyl, or trifluoromethyl.

In still another embodiment, said at least a DIGRA has Formula I, wherein
(a) A is an aryl, heteroaryl, heterocyclyl, or C₃-C₈ cycloalkyl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen, C₁-C₅ alkyl, C₅-C₁₅ arylalkyl, or R' and R² together with the carbon atom they are commonly attached to form a C₃-C₈ spiro cycloalkyl ring;
(c) B is the carbonyl group or methylene group, which is optionally independently substituted with one or two substituent groups selected from the group consisting of C₁-C₃ alkyl, hydroxy, and halogen;
(d) R³ is the trifluoromethyl group;
(e) D is absent;
(f) E is the hydroxy group or amino group wherein the nitrogen atom is optionally independently mono- or di-substituted by C₁-C₅ alkyl; and
(g) Q comprises a 5- to 7-membered heterocyclyl ring fused to a 5- to 7-membered heteroaryl or heterocyclyl ring, each optionally independently substituted with one to three substituent groups, wherein each substituent group of Q is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by C₁-C₅ alkyl, ureido wherein either nitrogen atom is optionally independently substituted with C₁-C₅ alkyl, or C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, C₁-C₃ alkoxycarbonyl, acyl, aryl, benzyl, heteroaryl, heterocyclyl, halogen, hydroxy, oxo, cyano, amino wherein the nitrogen atom is optionally independently mono- or di-substituted by C₁-C₅ alkyl, and ureido wherein either nitrogen atom is optionally independently substituted with C₁-C₅ alkyl or trifluoromethyl, wherein Q cannot be 1H-[1,5]naphthyridin-4-one.

Non-limiting examples of these compounds include 4-[4-(5-fluoro-2-methoxyphenyl)-2-hydroxy-4-methyl-2-trifluoromethylpentyl]-4H-thieno[3,2-b]pyridin-7-one; 4-[4-(5-fluoro-2-hydroxyphenyl)-2-hydroxy-4-methyl-2-trifluoromethylpenty-1]-4H-thieno[3,2-b]pyridin-7-one; 4-[4-(2,3-dihydrobenzofuran-7-yl)-2-hydroxy-4-methyl-2-trifluoromethylpentyl]-4H-thieno[3,2-b]pyridin-7-one; 1-[4-(5-fluoro-2-methoxyphenyl)-2-hydroxy-4-methyl-2-trifluoromethylpentyl]-1H-[1,6]naphthyridin-4-one; 1-[4-(5-fluoro-2-hydroxyphenyl)-2-hydroxy-4-methyl-2-trifluoromethylpentyl]-1H-[1,6]naphthyridin-4-one; 4-[4-(5-fluoro-2-methylphenyl)-2-hydroxy-4-methyl-2-trifluoromethylpentyl]-4H-thieno[3,2-b]pyridin-7-one; 4-[2-hydroxy-4-(5-methanesulfonyl-2,3-dihydrobenzofuran-7-yl)-4-methyl-2-trifluoromethylpentyl]-4H-thieno[3,2-b]pyridin-7-one; 1-[2-hydroxy-4-(5-methanesulfonyl-2,3-dihydrobenzofuran-7-yl)-4-methyl-2-trifluoromethylpentyl]-1H-[1,6]naphthyridin-4-one; 1-[4-(5-fluoro-2-methylphenyl)-2-hydroxy-4-methyl-2-trifluoromethylpentyl]-1H-[1,6]naphthyridin-4-one; 4-[2-hydroxy-4-(2-methoxy-3-methylphenyl)-4-methyl-2-trifluoromethylpentyl]-4H-thieno[3,2-b]pyridin-7-one; 4-[2-hydroxy-4-(2-methoxyphenyl)-4-methyl-2-trifluoromethylpentyl]-4H-thieno[3,2-b]pyridin-7-one; 4-[4-(3-bromo-2-methoxyphenyl)-2-hydroxy-4-methyl-2-trifluoromethylpentyl]-4H-thieno[3,2-b]pyridin-7-one; 4-[2-hydroxy-4-(2-hydroxy-3-methylphenyl)-4-methyl-2-trifluoromethylpentyl]-4H-thieno[3,2-b]pyridin-7-one; 4-[4-(3-bromo-2-hydroxyphenyl)-2-hydroxy-4-methyl-2-trifluoromethylpentyl]-4H-thieno[3,2-b]pyridin-7-one; 3-bromo-1-[4-(5-chloro-2,3-dihydrobenzofuran-7-yl)-2-hydroxy-4-methyl-2-trifluoromethylpentyl]-1H-[1,6]naphthyridin-4-one; 6-chloro-4-[4-(2,3-dihydrobenzofuran-7-yl)-2-hydroxy-4-methyl-2-trifluoromethylpentyl]-4H-thieno[3,2-b]pyridin-7-one; 6-bromo-4-[4-(2,3-dihydrobenzofuran-7-yl)-2-hydroxy-4-methyl-2-trifluoromethylpentyl]-4H-thieno[3,2-b]pyridin-7-one; 3-chloro-1- -14-(5-fluoro-2-hydroxyphenyl)-2-hydroxy-4-methyl-2-trifluoromethylpentyl]-1H-[1,6]naphthyridin-4-one; 1-[4-(5-chloro-2,3-dihydrobenzofuran-7-yl)-2-hydroxy-4-methyl-2-trifluoromethylpentyl]-3-methyl-1H-[1,6]naphthyridin-4-one; 1-[4-(5-Chloro-2,3-dihydrobenzofuran-7-yl)-2-hydroxy-4-methyl-2-trifluoromethylpentyl]-3-methyl-1H-[1,7]naphthyridin-4-one; 1-[2-hydroxy-4-(2-methoxy-3,5-dimethylphenyl)-4-methyl-2-trifluoromethylpentyl]-3-methyl-1H-[1,6]naphthyridin-4-one; 1-[2-hydroxy-4-(2-methoxy-3,5-dimethylphenyl)-4-methyl-2-trifluoromethylpentyl]-3-methyl-1H-[1,7]naphthyridin-4-one; 1-[2-hydroxy-4-(2-hydroxy-3,5-dimethylphenyl)-4-methyl-2-trifluoromethylpentyl]-3-methyl-1H-[1,6]naphthyridin-4-one; 1-[4-(5-fluoro-2-methylphenyl)-2-hydroxy-4-methyl-2-trifluoromethylpentyl]-1H-[1,8]naphthyridin-4-one; 1-[4-(5-fluoro-2-methylphenyl)-2-hydroxy-4-methyl-2-trifluoromethylpentyl]-1H-[1,7]naphthyridin-4-one; 4-[4-(5-fluoro-2-hydroxyphenyl)-2-hydroxy-4-methyl-2-trifluoromethylpenty- 1]-4H-thiazolo[4,5-b]pyridin-7-one; 4-[4-(5-fluoro-2-hydroxyphenyl)-2-hydroxy-4-methyl-2-trifluoromethylpentyl]-4H-oxazolo[4.5-b]pyridin-7-one; 4-[4-(5-fluoro-2-methylphenyl)-2-hydroxy-4-methyl-2-trifluoromethylpentyl]-4H-furo[3,2-b]pyridin-7-one; 7-[4-(5-fluoro-2-methylphenyl)-2-hydroxy-4-methyl-2-trifluoromethylpentyl]-7H-thieno[2,3-b]pyridin-4-one; 4-[4-(5-fluoro-2-hydroxyphenyl)-2-hydroxy-4-methyl-2-trifluoromethylpentyl]-4H-oxazolo[5,4-b]pyridin-7-one; 4-[4-(5-fluoro-2-hydroxyphenyl)-2-hydroxy-4-methyl-2-trifluoromethylpentyl]-4H-thiazolo[5,4-b]pyridin-7-one; 7-[4-(5-fluoro-2-methylphenyl)-2-hydroxy-4-methyl-2-trifluoromethylpentyl]-7H-furo[2,3-b]pyridin-4-one; 4-[4-(5-fluoro-2-methylphenyl)-2-hydroxy-4-methyl-2-tritluoromethylpentyl]-1,4-dihydropyrrolo[3,2-b]pyridin-7-one; 1-[4-(5-fluoro-2-hydroxyphenyl)-2-hydroxy-4-methyl-2-trifluoromethylpentyl]-5,6,7,8-tetrahydro-1H-[1,6]naphthyridin-4-one; 1-[4-(5-fluoro-2-methylphenyl)-2-hydroxy-4-methyl-1-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-1H-[1,8]naphthyridin-4-one; 1-[2-hydroxy-4-(5-methanesulfonyl-2,3-dihydrobenzofuran-7-yl)-4-methyl-2-trifluoromethylpentyl]-1H-[1,7]naphthyridin-4-one; 4-[2-hydroxy-4-(5-methanesulfonyl-2,3-dihydrobenzofuran-7-yl)-4-methyl-2-trifluoromethylpentyl]-4-H-thiazolo[4,5-b]pyridin-7-one; 4-[4-(2,3-dihydrobenzofuran-7-yl)-2-hydroxy-4-methyl-2-trifluoromethylpentyl]-4H-oxazolo[4,5-b]pyridin-7-one; 4-[2-hydroxy-4-(5-methanesulfonyl-2,3-dihydrobenzofuran-7-yl)-4-methyl-2-trifluoromethylpentyl]-4H-furo[3,2-b]pyridin-7-one; 7-[4-(2,3-dihydrobenzofuran-7-yl)-2-hydroxy-4-methyl-2-trifluoromethylpentyl]-7H-thieno[2,3-b]pyridin-4-one; 4-[2-hydroxy-4-(5-methanesulfonyl-2,3-dihydrobenzofuran-7-yl)-4-methyl-2-trifluoromethylpentyl]-4H-oxazolo[5,4-b]pyridin-7-one; 4-[2-hydroxy-4-(5-methanesulfonyl-2,3-dihydrobenzofuran-7-yl)-4-methyl-2-trifluoromethylpentyl]-4H-thiazolo[5,4-b]pyridin-7-one; 7-[4-(2,3-dihydrobenzofuran-7-yl)-2-hydroxy-4-methyl-2-trifluoromethylpentyl]-7H-furo[2,3-b]pyridin-4-one; 4-[4-(2,3-dihydrobenzofuran-7-yl)-2-hydroxy-4-methyl-2-trifluoromethylpentyl]-1,4-dihydropyrrolo[3,2-b]pyridin-7-one; 1-[2-hydroxy-4-(5-methanesulfonyl-2,3-dihydrobenzofuran-7-yl)-4-methyl-2-trifluoromethylpentyl]-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 1-[4-(2,3-dihydrobenzofuran-7-yl)-2-hydroxy-4-methyl-2-tritluoromethylpentyl]-5-methyl-5,6,7,8-tetrahydro-1H-[1,5]naphthyridin-4-one; 4-[2-hydroxy-4-(4-methoxybiphenyl-3-yl)-4-methyl-2-trifluoromethylpentyl]-4H-thieno[3,2-b]pyridin-7-one; 4-[2-hydroxy-4-(2-methoxy-5-pyridin-3-ylphenyl)-4-methyl-2-trifluoromethylpentyl]-4H-thieno[3,2-b]pyridin-7-one; 4-[2-hydroxy-4-(2-methoxy-5-pyrimidin-5-ylphenyl)-4-methyl-2-trifluoromethylpentyl]-4H-thieno[3,2-b]pyridin-7-one; 4-[2-hydroxy-4-(2-methoxy-5-thiophen-3-ylphenyl)-4-methyl- 2-trifluoromethylpentyl]-4H-thieno[3,2-b]pyridin-7-one; 4-[2-hydroxy-4-(4-hydroxybiphenyl-3-yl)-4-methyl-2-trifluoromethylpentyl]-4H-thieno[3,2-b]pyridin-7-one; 4-[2-hydroxy-4-(2-hydroxy-5-pyridin-3-ylphenyl)-4-methyl-2-trifluoromethylpentyl]-4H-thieno[3,2-b]pyridin-7-one; 4-[2-hydroxy-4-(2-hydroxy-5-pyrimidin-5-ylphenyl)-4-methyl-2-trifluoromethylpentyl]-4H-thieno[3,2-b]pyridin-7-one; 4-[2-Hydroxy-4-(2-hydroxy-5-thiophen-3-ylphenyl)-4-methyl-2-trifluoromethylpentyl]-4H-thieno[3,2-b]pyridin-7-one; 1-[2-hydroxy-4-(4-methoxybiphenyl-3-yl)-4-methyl-2-trifluoromethylpentyl]-1H-[1,6]naphthyridin-4-one; 1-[2-hydroxy-4-(2-methoxy-5-pyridin-3-ylphenyl)-4-methyl-2-trifluoromethylpentyl]-1H-[1,6]naphthyridin-4-one; 1-[2-hydroxy-4-(2-methoxy-5-pyrimidin-5-ylphenyl)-4-methyl-2-trifluoromethylpentyl]-1H-[1,6]naphthyridin-4-one; 1-[2-hydroxy-4-(2-methoxy-5-thiophen-3-ylphenyl)-4-methyl-2-trifluoromethylpentyl]-1H-[1,6]naphthyridin-4-one-; 1-[2-hydroxy-4-(2-methoxy-5-thiophen-3-ylphenyl)-4-methyl-2-trifluoromethylpentyl]-1H-[1,6]naphthyridin-4-one; 1-[2-hydroxy-4-(2-hydroxy-5-pyridin-3-ylphenyl)-4-methyl-2-trifluoromethylpentyl]-1H-[1,6]naphthyridin-4-one; 1-[2-hydroxy-4-(2-hydroxy-5-pyrimidin-5-ylphenyl)-4-methyl-2-trifluoromethylpentyl]-1H-[1,6]naphthyridin-4-one; -[2-hydroxy-4-(2-hydroxy-5-thiophen-3-yphenyl)-4-methyl-2-trifluoromethylpentyl]-1H-[1,6]naphthyridin-4-one; 5-[4-(5-fluoro-2-methoxyphenyl)-2-hydroxy-4-methyl-2-trifluoromethylpentyl]-5H-pyrido[3,2-d]pyrimidin-8-one; 1-[4-(5-fluoro-2-methoxyphenyl)-2-hydroxy-4-methyl-2-trifluoromethylpentyl]-1H-pyrido[2,3-d]pyridazin-4-one; 5-[4-(5-fluoro-2-methoxyphenyl)-2-hydroxy-4-methyl-2-trifluoromethylpenty-1]-5H-pyrido[3,2-c]pyridazin-8-one; 4-[4-(2-fifluoromethoxy-3-methylphenyl- )-2-hydroxy-4-methyl-2-trifluoromethylpentyl]-4H-thieno[3,2-b]pyridin-7-one; 3-chloro-1-[4-(2,3-dihydrobenzofuran-7-yl)-2-hydroxy-4-methyl-2-trifluoromethylpentyl]-1H-[1,6]naphthyridin-4-one; 4-(4-benzo[1,3]dioxol-4-yl-2-hydroxy-4-methyl-2-trifluoromethylpentyl)-6-bromo-4H-thieno[3,2-b]pyridin-7-one; 4-(4-benzo[1,3]dioxol-4-yl-2-hydroxy-4-methyl-2-trifluoromethylpentyl)-6-chloro-4H-thieno[3,2-b]pyridin-7-one; 6-chloro-4-[2-hydroxy-4-methyl-4-(5-pyridin-3-yl-2,3-dihydrobenzofuran-7-yl)-2-trifluoromethylpentyl]-4H-thieno[3,2-b]pyridin-7-one; 1-(4-benzo[1,3]dioxol-4-yl-2-hydroxy-4-methyl-2-trifluoromethylpentyl)-3-chloro-1H-[1,6]naphthyridin-4-one; 6-chloro-4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 3-chloro-1-[2-hydroxy-4-methyl-4-(5-pyridin-3-yl-2,3-dihydrobenzofuran-7-yl)-2-trifluoromethylpentyl]-1H-[1,6]naphthyridin-4-one; 4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 6-chloro-4-[2-hydroxy-4-(2-methoxy-5-pyridin-3-ylphenyl)-4-methyl-2-trifluoromethylpentyl]-4H-thieno[3,2-b]pyridin-7-one; 6-chloro-4-[2-hydroxy-4-(2-methoxy-5-pyrimidin-5-ylphenyl)-4-methyl-2-trifluoromethylpentyl]-4H-thieno[3,2-b]pyridin-7-one; 6-chloro-4-[2-hydroxy-4-(2-hydroxy-5-pyridin-3-ylphenyl)-4-methyl-2-trifluoromethylpentyl]-4H-thieno[3,2-b]pyridin-7-one; 6-chloro-4-[2-hydroxy-4-(-2-hydroxy-5-pyrimidin-5-ylphenyl)-4-methyl-2-trifluoromethylpentyl]-4H-thieno[3,2-b]pyridin-7-one; 4-(4-biphenyl-3-yl-2-hydroxy-4-methyl-2-trifluoro-methylpentyl)-6-chloro-4H-thieno[3,2-b]pyridin-7-one; 4-(4-biphenyl-3-yl-2-hydroxy-4-methyl-2-trifluoromethylpentyl)-4H-thieno[3,2-b]pyridin-7-one; 3-chloro-1-{4-[5-(5-chloropyridin-3-yl)-2,3-dihydrobenzofuran-7-yl]-2-hydroxy-4-methyl-2-trifluoromethylpentyl}-1H-[1,6]naphthyridin-4-one; 6-chloro-4-{4-[5-(2,6-dimethylpyridin-4-yl)-2-methoxyphenyl]-2-hydroxy-4-methyl-2-trifluoromethylpentyl}-4H-thieno[3,2-b]pyridin-7-one- ; 4-[2-hydroxy-4-(2-hydroxy-5-pyridin-2-ylphenyl)-4-methyl-2-trifluoromethylpentyl]-4H-thieno[3,2-b]pyridin-7-one; 6-chloro-4-[2-hydroxy-4-methyl-4-(5-pyrazin-2-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-2-yl-2,3-dihydrobenzofuran-7-yl)-2-trifluoromethylpentyl]-1H-[1,6]naphthyridin-4-one; 5-{7-[3-(6-chloro-7-oxo-7H-thieno[3,2-b]pyridin-4-ylmethyl)-4,4,-4-trifluoro-3-hydroxy-1,1 - dimethylbutyl]-2,3-dihydrobenzofuran-5-yl}nicotinonitrile; 4-{4-Methoxy-3-[4,4,4-trifluoro-3-hydroxy-1,1-dimethyl-3-(7-oxo-7H-thieno[3,2-b]pyridin-4-ylmethyl)butyl]phenyl}pyridine-2-carbonitrile; 6-chloro-4-{4-[5-(2-fluoro-6-methylpyridin-4-yl)-2-methoxyphenyl]-2-hydroxy-4-methyl-2-trifluoromethylpentyl}-4H-thieno[3,2-b]pyridin-7-one; 3-chloro-1-{2-hydroxy-4-[5-(1H-imidazol-4-yl)-2,3-dihydrobenzofuran-7-yl]-4-methyl-2-trifluoromethylpentyl}-1H-[1,6]naphthyridin-4-one; 6-chloro-4-[2-hydroxy-4-methyl-4-(5-morpholin-4-yl-2,3-dihydrobenzofuran-7-yl)-2-trifluoromethylpentyl]-4H-thieno[3,2-b]pyridin-7-one; and 1-[2-hydroxy-4-methyl-4-(5-piperidin-1-yl-2,3-dihydrobenzofuran-7-yl)-2-trifluoromethylpentyl]-1H-[1,6]naphthyridin-4-one.

In yet another embodiment, said at least a DIGRA has Formula I, wherein A, B, D, E, R¹, and R² have the meanings disclosed immediately above, and R³ is hydrogen, C₁-C₈ alkyl, C₂-C₈ alkenyl, C₂-C₈ alkynyl, carbocycle, heterocyclyl, aryl, heteroaryl, carbocycle-C₁-C₈ alkyl, carboxy, alkoxycarbonyl, aryl-C₁-C₈ alkyl, aryl-C₁-C₈ haloalkyl, heterocyclyl-C₁-C₈ alkyl, heteroaryl-C₁-C₈ alkyl, carbocycle-C₁-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone, wherein R³ cannot be trifluoromethyl.

In yet another embodiment, said at least a DIGRA has Formula I, wherein
(a) A is an aryl, heteroaryl, heterocyclyl, or C₃-C₈ cycloalkyl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl;
(c) R³ is the trifluoromethyl group;
(d) B is C₁-C₅ alkylene, C₂-C₅ alkenylene, or C₂-C₅ alkynylene, each optionally independently substituted with one to three substituent groups, wherein each substituent group of B is independently C₁-C₃ alkyl, hydroxy, halogen, amino, or oxo;
(e) D is absent;
(f) E is the hydroxy group; and
(g) Q comprises an indolyl group optionally substituted with one to three substituent groups, wherein each substituent group of Q is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by C₁-C₅ alkyl, ureido wherein either nitrogen atom is optionally independently substituted with C₁-C₅ alkyl, or C₁-C₅ alkylthio wherein the sulfur atom is optionally oxidized to a sulfoxide or sulfone, wherein each substituent group of Q is optionally independently substituted with one to three substituent groups selected from the group consisting of C₁-C₃ alkyl, C₁-C₃ alkoxy, halogen, hydroxy, oxo, cyano, amino, and trifluoromethyl.

Non-limiting examples of these compounds include 4-(5-bromo-2,3-dihydrobenzofuran-7-yl)-1,1-trifluoro-2-(1H-indol-2-ylmethyl)-4-methylpentan-2-ol; 1,1,1-trifluoro-2-(1H-indol-2-ylmethyl)-4-methyl-4-pyridin-2-ylpentan-2-ol; 4-(2,3-dihydro-5-cyanobenzofuran-7-yl)-1,1,1-trifluoro-2-(1H-indol-2-yl-methyl)-4-methylpentan-2-ol; 4-(2,3-dihydrobenzofuran-7-yl)-1,1,1-trifluoro-2-(1H-indol-2-ylmethyl)-4-methylpentan-2-ol; 1,1,1-trifluoro-4-(5-fluoro-2,3-dihydrobenzofuran-7-yl)-2-(1H-indol-2-ylmethyl)-4-methylpentan-2-ol; 1,1,1-trifluoro-2-(1H-indol-2-ylmethyl)-4-methyl-4-(5-methyl-2,3-dihydrobenzofuran-7-yl)pentan-2-ol; 4-(2,3-dihydrobenzofuran-5-yl)-1,1,1-trifluoro-2-(1H-indol-2-ylmethyl)-4-methylpentan-2-ol; 2-[4-(2,3-dihydrobenzofuran-7-yl)-2-hydroxy-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-3-carbonitrile; 2-[4-(5-bromo-2,3-dihydrobenzofuran-7-yl)-2-hydroxy-4-methyl-2-trifluoromethylpentyl]-1H-indole-3-carbonitrile; 2-[4-(2,3-dihydrobenzofuran-7-yl)-2-hydroxy-4-methyl-2-trifluoromethylpentyl]-4-methyl-1H-indole-6-carbonitrile; 2-[4-(2,3-dihydrobenzofuran-7-yl)-2-hydroxy-4-methyl-2-trifluoromethylpentyl]-1H-indole-5-carbonitrile; 4-(2,3-dihydrobenzofuran-7-yl)-1,1,1-trifluoro-2-(7-fluoro-1H-indol-2-ylmethyl)4-methylpentan-2-ol; 1-[4-(2,3-dihydrobenzofuran-7-yl)-2-hydroxy-4-methyl-2-trifluoromethylpentyl]-1H-indole-3-carbonitrile; 4-(2,3-dihydrobenzofuran-7-yl)-1,1,1-trifluoro-4-methyl-2-(5-trifluoromethyl-1H-indol-2-ylmethyl)pentan-2-ol; and 1,1,1-trifluoro-2-(1H-indol-2-ylmethyl)-4-methyl-4-thiophen-3-ylpentan-2-ol.

In a further embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R¹ and R² are each independently hydrogen or C₁-C₅ alkyl, or R¹ and R² together with the carbon atom they are commonly attached to form a C₃-C₈ spiro cycloalkyl ring;
(c) R³ is carbocycle, heterocyclyl, aryl, heteroaryl, carbocycle-C₁-C₈ alkyl, carboxy, alkoxycarbonyl, aryl-C₁-C₈ alkyl, aryl-C₁-C₈ haloalkyl, heterocyclyl-C₁-C8 alkyl, heteroaryl-C₁-C₈ alkyl, carbocycle-C₂-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone;
(d) B is the methylene or carbonyl group;
(e) D is the -NH- group;
(f) E is the hydroxy group; and
(g) Q comprises the group

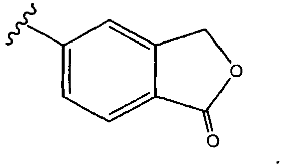

Non-limiting examples of these compounds include 2-benzyl-2-hydroxy-4-methyl-4-phenylpentanoic acid (1-oxo-1,3-dihydroisobenzofuran-5-yl)amide; 2-hydroxy-4-methyl-2,4-diphenylpentanoic acid (1-oxo-1,3-dihydroisobenzofuran-5-yl)amide; 2-hydroxy-4-methyl-2-phenethyl-4-phenylpentanoic acid (1-oxo-1,3-dihydroisobenzofuran-5-yl)amide; 2-hydroxy-2-(3-methoxybenzyl)₄-methyl-4-phenylpentanoic acid (1-oxo-1,3-dihydroisobenzofuran-5-yl)amide; 2-hydroxy-2-(4-methoxybenzyl)-4-methyl-4-phenylpentanoic acid (1-oxo-1,3-dihydroisobenzofuran-5-yl)amide; 2-hydroxy-2-[2-(4-methoxyphenyl)ethyl]4-methyl-4-phenylpentanoic acid (1-oxo- 1,3-dihydroisobenzofuran-5-yl)amide; 2-cyclohexylmethyl-2-hydroxy-4-methyl-4-phenylpentanoic acid (1-oxo-1,3-dihydroisobenzofuran-5-yl)amide; 2-(4-tert-butylbenzyl)-2-hydroxy-4-methyl-4-phenylpentanoic acid (1-oxo-1,3-dihydroisobenzofuran-5-yl)amide; 2-biphenyl-4-ylmethyl-2-hydroxy-4-methyl-4-phenylpentanoic acid (1-oxo-1,3-dihydroisobenzofuran-5-yl)amide; 2-hydroxy-4-methyl-2-naphthalen-2-ylmethyl-4-phenylpentanoic acid (1-oxo-1,3-dihydroisobenzofuran-5-yl)amide; 2-hydroxy-2-(3-hydroxybenzyl)-4-methyl-4-phenylpentanoic acid (1-oxo-1,3-dihydroisobenzofuran-5-yl)amide; 2-hydroxy-4-methyl-2-(2-methyl-2-phenylpropyl)-4-phenylpentanoic acid (1-oxo-1,3-dihydroisobenzofuran-5-yl)amide; 2-benzyl-4-(5-fluoro-2-methoxyphenyl)-2-hydroxy-4-methylpentanoic acid (1-oxo-1,3-dihydroisobenzofuran-5-yl)amide; 2-cyclohexylmethyl-4-(5-fluoro-2-methoxyphenyl)-2-hydroxy-4-methylpentanoic acid (1-oxo-1,3-dihydroisobenzofuran-5-yl)amide; 2-benzyl-4-(5-Fluoro-2-hydroxyphenyl)-2-hydroxy-4-methylpentanoic acid (1-oxo-1,3-dihydroisobenzofuran-5-yl)amide; 2-cyclohexylmethyl-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methyl-2-phenylpropyl)pentanoic acid (1-oxo-1,3-dihydroisobenzofuran-5-yl)amide; 2-(2-chloro-6-fluorobenzyl)-4-(5-fluoro-2-methoxyphenyl)-2-hydroxy-4-methylpentanoic acid (1-oxo-1,3-dihydroisobenzofuran-5-yl)amide; 2-(3-fluorobenzyl)-4-(5-fluoro-2-methoxyphenyl)-2-hydroxy-4-methylpentanoic acid (1-oxo-1,3-dihydroisobenzofuran-5-yl)amide; 2-(2-fluorobenzyl)-4-(5-fluoro-2-methoxyphenyl)-2-hydroxy-4-methylpentanoic acid (1-oxo-1,3-dihydroisobenzofuran-5-yl)amide; 2-(3,4-difluorobenzyl)-4-(5-fluoro-2-methoxyphenyl)-2-hydroxy-4-methylpentanoic acid (1-oxo-1,3-dihydroisobenzofuran-5-yl)amide; 2-(2-chloro-6-fluorobenzyl)-4-(5-fluoro-2-hydroxyphenyl)-2-hydroxy-4-methylpentanoic acid (1-oxo-1,3-dihydroisobenzofuran-5-yl)amide; 2-(3-fluorobenzyl)-4-(5-fluoro-2-hydroxyphenyl)-2-hydroxy-4-methylpentanoic acid (1-oxo-1,3-dihydroisobenzofuran-5-yl)amide; 2-(2-fluorobenzyl)-4-(5-fluoro-2-hydroxyphenyl)-2-hydroxy-4-methylpentanoic acid (1-oxo-1,3-dihydroisobenzofuran-5-yl)amide; 2-(3,4-difluorobenzyl)-4-(5-fluoro-2-hydroxyphenyl)-2-hydroxy-4-methylpentanoic acid (1-oxo-1,3-dihydroisobenzofuran-5-yl)amide; 2-(4-fluorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(3-methylbenzyl)pentanoic acid (1-oxo-1,3-dihydroisobenzofuran-5-yl)amide; 2-(4-fluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(3-methylbenzyl)pentanoic acid (1-oxo-1,3-dihydroisobenzofuran-5-yl)amide; 2-(3,5-difluorophenyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-hydroxyphenyl)-2-hydroxy-4-methylpentanoic acid (1-oxo-1,3-dihydroisobenzofuran-5-yl)amide; 2-(3-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-2-[2-(4-methoxyphenyl)ethyl]-4-methylpentanoic acid (1-oxo-1,3-dihydroisobenzofuran-5-yl)amide; 4-(5-fluoro-2-methoxyphenyl)-2-hydroxy-2-(2-methoxybenzyl)₄-methylpentanoic acid (1-oxo-1,3-dihydroisobenzofuran-5-yl)amide; 4-(5-fluoro-2-methoxyphenyl)-2-hydroxy-4-methyl-2-phenethylpentanoic acid (1-oxo-1,3-dihydroisobenzofuran-5-yl)amide; 2-(2-chlorobenzyl)-4-(5-tluoro-2-methoxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-phenethylpentanoic acid (1-oxo-1,3-dihydroisobenzofuran-5-yl)amide; 4-(5-fluoro-2-hydroxyphenyl)-2-hydroxy-2-[2-(4-hydroxyphenyl)ethyl]-4-methylpentanoic acid (1-oxo-1,3-dihydroisobenzofuran-5-yl)amide; 2-(2-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-2-(2-hydroxybenzyl)-4-methylpentanoic acid (1-oxo-1,3-dihydroisobenzofuran-5-yl)amide; 2-(2-bromobenzyl)-4-(5-fluoro-2-methoxyphenyl)-2-hydroxy-4-methylpentanoic acid (1-oxo-1,3-dihydroisobenzofuran-5-yl)amide; 2-(2-bromobenzyl)-4-(5-fluoro-2-hydroxyphenyl)-2-hydroxy-4-methylpentanoic acid (1-oxo-1,3-dihydroisobenzofuran-5-yl)amide; 2-(5-fluoro-2-methoxybenzyl)-2-hydroxy-4-methyl-4-phenylpentanoic acid (1-oxo-1,3-dihydroisobenzofuran-5-yl)amide; 2-(5-fluoro-2-hydroxybenzyl)-2-hydroxy-4-methyl-4-phenylpentanoic acid (1-oxo-1,3-dihydroisobenzofuran-5-yl)amide; 2-(5-fluoro-2-methoxybenzyl)-4-(5-fluoro-2-methoxyphenyl)-2-hydroxy-4-methylpentanoic acid (1-oxo-1,3-dihydroisobenzofuran-5-yl)amide; 2-(5-fluoro-2-hydroxybenzyl)-4-(5-fluoro-2-hydroxyphenyl)-2-hydroxy-4-methylpentanoic acid (1-oxo-1,3-dihydroisobenzofuran-5-yl)amide; 2-(3,5-dimethoxybenzyl)-2-hydroxy-4-methyl-4-phenylpentanoic acid (1-oxo-1,3-dihydroisobenzofuran-5-yl)amide; 2-(3,5-dihydroxybenzyl)-2-hydroxy-4-methyl-4-phenylpentanoic acid (1-oxo-1,3-dihydroisobenzofuran-5-yl)- amide; 2-hydroxy-2-(2-methoxybenzyl)₄-methyl-4-phenylpentanoic acid (1-oxo-1,3-dihydroisobenzofuran-5-yl)amide; 12-hydroxy-2-(2-hydroxybenzyl)-4-methyl-4-phenylpentanoic acid (1-oxo-1,3-dihydroisobenzofuran-5-yl)amide; 2-hydroxy-2-[2-(4-hydroxyphenyl)ethyl]-4-methyl-4-phenylpentanoic acid (1-oxo-1,3-dihydroisobenzofuran-5-yl)amide; 15-[2-benzyl-4-(5-fluoro-2-methoxyphenyl)-2-hydroxy-4-methylpentylamino]-3H-isobenzofuran-1-one; 4-(5-fluoro-2-methoxyphenyl)-2-hydroxy-4-methyl-2-(1-phenylvinyl)pentanoic acid (1-oxo-1,3-dihydroisobenzofuran-5-yl)amide; 2-hydroxy-4-methyl-4-phenyl-2-pyridin-2-ylmethylpentanoic acid(1-oxo-1,3-dihydroisobenzofuran-5-yl)amide; 4-(5-fluoro-2-methoxyphenyl)-2-hydroxy-4-methyl-2-(1-phenylethyl-)pentanoic acid(1-oxo-1,3-dihydroisobenzofuran-5-yl)amide; 4-(5-fluoro-2-hydroxyphenyl)-2-hydroxy-4-methyl-2-(1-phenylethyl)pentanoic acid(1-oxo-1,3-dihydroisobenzofuran-5-yl)amide; 2-cyclopentyl-4-(5-fluoro-2-methoxyphenyl)-2-hydroxy-4-methylpentanoic acid(1-oxo-1,3-dihydroisobenzofuran-5-yl)amide; 2-cyclopentyl-4-(5-fluoro-2-hydroxyphenyl)-2-hydroxy-4-methylpentanoic acid(1-oxo-1,3-dihydroisobenzofuran-5-yl)amide; 2-cyclopentylmethyl-4-(5-fluoro-2-hydroxyphenyl)-2-hydroxy-4-methylpentanoic acid(1-oxo-1,3-dihydroisobenzofuran-5-yl)amide; and 2-benzyl-2-hydroxy-N-(1-oxo-1,3-dihydroisobenzofuran-5-yl)₄-phenyl-butyramide.

In still another embodiment, said at least a DIGRA has Formula I, wherein
(a) A is an aryl or heteroaryl group, each optionally independently substituted with one to three substituent groups, which are independently selected from the group consisting of C₁-C₅ alkyl, C₂-C₅ alkenyl, C₂-C₅ alkynyl, C₁-C₃ alkanoyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, aroyl, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaninosulfonyl, halogen, hydroxy, carboxy, cyano, trifluoromethyl, trifluoromethoxy, nitro, amino wherein the nitrogen atom is optionally independently mono- or di-substituted by C₁-C₅ alkyl or aryl, ureido wherein either nitrogen atom is optionally independently substituted with C₁-C₅ alkyl, C₁-C₅ alkylthio wherein the sulfur atom is optionally oxidized to a sulfoxide or sulfone;
(b) R' and R² are each independently hydrogen or C₁-C₅ alkyl, or R¹ and R² together with the carbon atom they are commonly attached to form a C₃-C₈ spiro cycloalkyl ring;
(c) R³ is the trifluoromethyl group;
(d) B is C₁-C₅ alkylene, C₂-C₅ alkenylene, or C₂-C₅ alkynylene, each optionally independently substituted with one to three substituent groups, wherein each substituent group of B is independently C₁-C₃ alkyl, hydroxy, halogen, amino, or oxo;
(e) D is absent;
(f) E is -NR⁶R⁷, wherein R⁶ and R⁷ are each independently hydrogen, C₁-C₈ alkyl, C₂-C₈ alkenyl, C₂-C₈ alkynyl, C₁-C₈ alkoxy, C₂-C₈ alkenyloxy, C₂-C₈ alkynyloxy, hydroxy, carbocyclyl, heterocyclyl, aryl, aryloxy, acyl, heteroaryl, carbocycle-C₁-C₈ alkyl, aryl-C₁-C₈ alkyl, aryl-C₁-C₈ haloalkyl, heterocyclyl-C,-Cg alkyl, heteroaryl-C₁-C₈ alkyl, carbocycle-C₂-C₈ alkenyl, aryl-C₂-C₈ alkenyl, heterocyclyl-C₂-C₈ alkenyl, heteroaryl-C₂-C₈ alkenyl, or C₁-C₅ alkylthio wherein the sulfur atom is oxidized to a sulfoxide or sulfone, each optionally independently substituted with one to three substituent groups, wherein each substituent group of R⁶ and R⁷ are independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaminosulfonyl, halogen, hydroxy, carboxy, cyano, oxo, trifluoromethyl, trifluoromethoxy, nitro, amino wherein the nitrogen atom is optionally independently mono- or di-substituted by C₁-C₅ alkyl, ureido wherein either nitrogen atom is optionally independently substituted with C₁-C₅ alkyl, or C₁-C₅ alkylthio wherein the sulfur atom is optionally oxidized to a sulfoxide or sulfone; and
(g) Q comprises a heteroaryl group optionally independently substituted with one to three substituent groups, wherein each substituent group of Q is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone, wherein each substituent group of Q is optionally independently substituted with one to three substituent groups selected from C₁-C₃ alkyl, C₁-C₃ alkoxy, halogen, hydroxy, oxo, cyano, amino, or trifluoromethyl.

Non-limiting examples of these compounds include 3-(5-fluoro-2-methoxyphenyl)-3-methyl-1-(pyridin-2-ylmethyl)-1-trifluoromethyl-butylamine; 3-(5-fluoro-2-methoxy-phenyl)-1-(1H-indol-2-ylmethyl)-3-methyl-1-trifluoromethyl-butylamine; 1-(2,6-dichloro-pyridin-4-ylmethyl)-3-(5-fluoro-2-methoxy-phenyl)-3-methyl-1-trifluoromethyl-butylamine; 1-(4,6-dimethyl-pyridin-2-ylmethyl)-3-(5-fluoro-2-methoxy-phenyl)-3-methyl-1-trifluoromethyl-butylamine; 1-(2-chloro-pyridin-4-ylmethyl)-3-(5-fluoro-2-methoxy-phenyl)-3-methyl-1-trifluoromethyl-butylamine; 3-(5-fluoro-2-methyl-phenyl)-3-methyl-1-(3-methyl-1H-indol-2-ylmethyl)-1-trifluoromethyl-butylamine; 3-(5-fluoro-2-methoxy-phenyl)-3-methyl-1-(3-methyl-1H-indol-2-ylmethyl)-1-trifluoromethyl-butylamine; 1-(6-fluoro-1H-indol-2-ylmethyl)-3-(5-fluoro-2-methoxy-phenyl)-3-methyl-1-trifluoromethyl-butylamine; 3-(4-fluoro-phenyl)-3-methyl-1-(3-methyl-1H-indol-2-ylmethyl)-1-trifluoro-methyl-butylamine; 3-benzofuran-7-yl-1-(2,6-dichloro-pyridin-4-ylmethyl)-3-methyl-1-trifluoromethyl-butylamine; 3-(2,3-dihydro-benzofuran-7-yl)-1-(6-fluoro-1H-indol-2-ylmethyl)-3-methyl-1-trifluoromethyl-butylamine; 3-(5-fluoro-2-methoxy-phenyl)-3-methyl-1-quinolin-4-ylmethyl-1-trifluoromethyl-butylamine; 1-(2-chloro-quinolin-4-ylmethyl)-3-(5-fluoro-2-methylphenyl)-3-methyl-1-trifluoromethyl-butylamine; 3-(4-fluoro-phenyl)-3-methyl-1-quinolin-4-ylmethyl-1-trifluoromethyl-butylamine; 7-[3-amino-3-(1H-benzolmidazol-2-ylmethyl)-4,4,4-trifluoro-1,1-dimethyl-butyl]-2,3-dihydrobenzofuran-5-carbonitrile; 1-(6-fluoro-1H-benzoimidazol-2-ylmethyl)-3-(5-fluoro-2-methyl-phenyl)-3-methyl-1-trifluoromethyl-butylamine; 2-[3-amino-3-(1H-benzoimidazol-2-ylmethyl)-4,4,4-trifluoro-1,1-dimethyl-butyl]4-fluoro-phenol; 1-(1H-benzoimidazol-2-ylmethyl)-3-(4-fluoro-phenyl)-3-methyl-1-trifluoromethyl-butylamine; 1-(1H-indol-2-ylmethyl)-3-methyl-3-pyridin-3-yl-(-trifluoromethyl-butylamine; 1-(1H-benzoimidazol-2-ylmethyl)-3-methyl-3-pyridin-4-yl-1-trifluoromethyl-butylamine; 3-methyl-1-(3-methyl-1H-indol-2-ylmethyl)-3-pyridin-3-yl-1-trifluoromethyl-butylamine; 1-(6-fluoro-1H-indol-2-ylmethyl)-3-methyl-3-pyridin-3-yl-1-trifluoromethyl-butylamine; 3-(2,3-dihydrobenzofuran-7-yl)-1-(1H-indol-2-ylmethyl)-3-methyl-1-trifluoromethyl-butylamine; [3-(5-fluoro-2-methoxy-phenyl)-3-methyl-1-quinolin-4-ylmethyl-1-trifluoromethyl-butyl]-methyl-amine; ethyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-propylamine; [3-(5-fluoro-2-methoxy-phenyl)-3-methyl-1-quinolin-4-ylmethyl-1-trifluoromethyl-butyl]-isobutylamine; butyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-dimethylamine; N-[3-(5-fluoro-2-methoxy-phenyl)-3-methyl-1-quinolin-4-ylmethyl-1-trifluoromethyl-butyl]-acetamide; N-[3-(5-fluoro-2-methoxy-phenyl)-3-methyl-1-quinolin-4-ylmethyl-1-trifluoromethyl-butyl]-formamide; N-[3-(5-fluoro-2-methoxy-phenyl)-3-methyl-1-quinolin-4-ylmethyl-1-trifluoromethyl-butyl]-methanesulfonamide; 1-(2,6-dimethyl-pyridin-4-ylmethyl)-3-(5-fluoro-2-methoxyphenyl)-3-methyl-1-trifluoromethyl-butylamine; 3-(5-fluoro-2-methoxy-phenyl)-3-methyl-1-(1H-pyrrolo[2,3-c]pyridin-2-ylmethyl)-1-trifluoromethyl-butylamine; 2-[2-amino-4-(5-fluoro-2-methoxy-phenyl)-4-methyl-2-trifluoromethyl-pentyl]-4-methyl- I H-indole-6-carbonitrile; N-[3-(5-fluoro-2-methoxy-phenyl)-3-methyl-1-quinolin-4-ylmethyl-1-trifluoromethyl-butyl]-hydroxylamine; and 2-(3-amino-4,4,4-trifluoro-1,1-dimethyl-3-quinolin-4-ylmethyl-butyl)-4-fluoro-phenol.

In yet another embodiment, said at least a DIGRA has Formula I, wherein A, B, D, E, R¹, R², R⁶, and R⁷ have the meanings disclosed immediately above, and R³ is C₁-C₈ alkyl, C₂-C₈ alkenyl, C₂-C₈ alkynyl, carbocycle, heterocyclyl, aryl, heteroaryl, carbocycle-C₁-C₈ alkyl, carboxy, alkoxycarbonyl, aryl-C₁-C₈ alkyl, aryl-C₁-C₈ haloalkyl, heterocyclyl-C₁-C₈ alkyl, heteroaryl-C₁-C₈ alkyl, carbocycte-C₁-C₈ alkenyl, aryl-C₂-C₈ alkenyl, heterocyclyl-C₂-C₈ alkenyl, or heteroaryl-C₂-C₈ alkenyl, each optionally independently substituted with one to three substituent groups, wherein each substituent group of R³ is independently C₁-C₅ alkyl, C₂-C₅ alkenyl, C₂-C₅ alkynyl, C₃-C₈ cycloalkyl, phenyl, C₁-C₅ alkoxy, phenoxy, C₁-C₅ alkanoyl, aroyl, C₁-C₅ alkoxycarbonyl, C₁-C₅ alkanoyloxy, aminocarbonyloxy, C₁-C₅ alkylaminocarbonyloxy, C₁-C₅ dialkylaminocarbonyloxy, aminocarbonyl, C₁-C₅ alkylaminocarbonyl, C₁-C₅ dialkylaminocarbonyl, C₁-C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, oxo, trifluoromethyl, nitro, amino wherein the nitrogen atom is optionally independently mono- or di-substituted by C₁-C₅ alkyl, ureido wherein either nitrogen atom is optionally independently substituted with C₁-C₅ alkyl, C₁-C₅ alkylthio wherein the sulfur atom is optionally oxidized to a sulfoxide or sulfone, wherein R³ cannot be trifluoromethyl.

Non-limiting examples of these compounds include 1-(2,6-dichloro-pyridin-4-ylmethyl)-3-(5-fluoro-2-methoxy-phenyl)-1,3-dimethyl-butylamine; 1-ethyl-3-(5-fluoro-2-methoxy-phenyl)-3-methyl-1-quinolin-4-ylmethyl-butylamine; 1-cyclohexylmethyl-3-(5-fluoro-2-methoxy-phenyl)-1-(1H-indol-2-ylmethyl)-3-methylbutylamine; 1-(2-chloro-quinolin-4-ylmethyl)-1-cyclopentyl-3-(5-fluoro-2-methoxyphenyl)-3-methyl-butylamine; 1-(2-chloro-pyridin-4-ylmethyl)-1-cyclopentylmethyl-3-(5-fluoro-2-methoxy-phenyl)-3-methyl-butytamine; 3-(5-fluoro-2-methoxy-phenyl)-1,3-dimethyl-1-quinolin-4-ylmethyl-butylamine; 1-cyclopropyl-3-(5-fluoro-2-methoxyphenyl)-3-methyl-1-quinolin4-ylmethyl-butylamine; 3-(5-fluoro-2-methoxy-phenyl)-1,3-dimethyl-1-(1H-pyrrolo[2,3-c]pyridin-2-ylmethyl)-butylamine; 1-cyclopropyl-3-(5-fluoro-2-methoxy-phenyl)-3-methyl-1-(1H-pyrrolo[2,3-c]-pyridin-2-ylmethyl)-butylamine; 2-[3-amino-1,1,3-trimethyl-4-(1H-pyrrolo[2,3-c]pyridin-2-yl)-butyl]-4-fluoro-phenol; 2-[2-amino-4-(5-fluoro-2-methoxy-phenyl)-2,4-dimethyl-pentyl]-4-methyl-1H-indole-6-carbonitrile.

Other compounds that can function as DIGRAs and methods for their manufacture are disclosed, for example, in U.S. Patent Application Publications 2004/0029932, 2004/0162321, 2004/0224992, 2005/0059714, 2005/0176706, 2005/0203128, 2005/0234091, 2005/0282881, 2006/0014787, 2006/0030561, and 2006/0116396.

The present invention provides an ophthalmic pharmaceutical composition for pharmacological adjunctive treatment associated with glaucoma filtration surgery.

The ophthalmic pharmaceutical composition comprises: (a) at least a DIGRA, a pharmaceutically acceptable salt thereof, or a pharmaceutically acceptable ester thereof; and (b) an antagonist to a TGF-β isoform. In one embodiment, the TGF-β isoform is selected from the group consisting of TGF-β1, TGF-β2, and combinations thereof. In another embodiment, the pharmaceutical composition further comprises a pharmaceutically acceptable carrier. Said carrier may be an ophthalmically acceptable carrier.

The concentration of a DIGRA, a pharmaceutically acceptable salt thereof, or a pharmaceutically acceptable ester thereof in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.00 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

In one embodiment, an ophthalmic composition of the present invention is in a form of a suspension or dispersion. In another embodiment, the suspension or dispersion is based on an aqueous solution. For example, a composition of the present invention can comprise sterile saline solution. In still another embodiment, micrometer-or nanometer-sized particles of a DIGRA, a pharmaceutically acceptable salt thereof, or a pharmaceutically acceptable ester thereof and an anti-inflammatory agent can be coated with a physiologically acceptable surfactant (non-limiting examples are disclosed below), then the coated particles are dispersed in a liquid medium. The coating can keep the particles in a suspension. Such a liquid medium can be selected to produce a sustained-release suspension. For example, the liquid medium can be one that is sparingly soluble in the ocular environment into which the suspension is administered. In still another embodiment, the active ingredient or ingredients are suspended or dispersed in a hydrophobic medium, such as an oil.

The DIGRA and the antagonist to at least a TGF-β isoform are present in amounts effective to reduce the risk of having a non- or poorly functioning filtering bleb following GFS, when a composition of the present invention is administered to a patient, as a pharmacological treatment associated with said GFS.

A composition of the present invention may further comprise an NSAID.

A composition of the present invention may comprise: (a) at least a DIGRA, a pharmaceutically acceptable salt thereof, or a pharmaceutically acceptable ester thereof; and (b) an NSAID.

Non-limiting examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), ε-acetamidocaproic acid, S-(5'-adenosyl)-L-methionine, 3-amino-4-hydroxybutyric acid, amixetrine, bendazac, benzydamine, α-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof.

The concentration of such an NSAID in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

A composition of the present invention may comprise: (a) at least a DIGRA, a pharmaceutically acceptable salt thereof, or a pharmaceutically acceptable ester thereof; and (b) a PPAR ligand.

The PPAR ligand may be a PPAR-binding molecule. In one embodiment, such a PPAR-binding molecule is a PPARα-, PPARδ-, or PPARγ-binding molecule. In another embodiment, such a PPAR-binding molecule is a PPARα, PPARδ, or PPARγ agonist. Such a PPAR ligand binds to and activates PPAR to modulate the expression of genes containing the appropriate peroxisome proliferator response element in its promoter region.

PPARγ agonists can inhibit the production of TNF-α and other inflammatory cytokines by human macrophages (C-Y. Jiang et al., Nature, Vol. 391, 82-86 (1998)) and T lymphocytes (A.E. Giorgini et al., Horm. Metab. Res. Vol. 31, 1-4 (1999)). Such TNF-α and other inflammatory cytokines are discussed elsewhere herein and shown to stimulate the production of, or activate a cascade involving, TGF-β isoforms. More recently, the natural PPARy agonist 15-deoxy-Δ-12,14-prostaglandin J2 (or "15-deoxy-Δ-12,14-PG J2"), has been shown to inhibit neovascularization and angiogenesis (X. Xin et al., J. Biol. Chem. Vol. 274:9116-9121 (1999)) in the rat cornea. Spiegelman et al., in U.S. Patent 6,242,196, disclose methods for inhibiting proliferation of PPARγ-responsive hyperproliferative cells by using PPARγ agonists; numerous synthetic PPARγ agonists are disclosed by Spiegelman et al., as well as methods for diagnosing PPARγ-responsive hyperproliferative cells. All documents referred to herein are incorporated by reference. PPARs are differentially expressed in diseased versus normal cells. PPARγ is expressed to different degrees in the various tissues of the eye, such as some layers of the retina and the cornea, the choriocapillaris, uveal tract, conjunctival epidermis, and intraocular muscles (see, e.g., U.S. Patent 6,316,465).

A PPARγ agonist which may be used in a composition or its use of the present invention is a thiazolidinedione, a derivative thereof, or an analog thereof. Non-limiting examples of thiazolidinedione-based PPARγ agonists include pioglitazone, troglitazone, ciglitazone, englitazone, rosiglitazone, and chemical derivatives thereof. Other PPARγ agonists include Clofibrate (ethyl 2-(4-chlorophenoxy)-2-methylpropionate), clofibric acid (2-(4-chlorophenoxy)-2-methylpropanoic acid), GW 1929 (N-(2-benzoylphenyl)-O-{2-(methyl-2-pyridinylamino)ethyl}-L-tyrosine), GW 7647 (2-{{4-{2-{{(cyclohexylamino)carbonyl}(4-cyclohexylbutyl)amino}ethyl}phenyl}thio}-2-methylpropanoic acid), and WY 14643 ({{4-chloro-6-{(2,3-dimethylphenyl)amino}-2-pyrimidinyl}thio}acetic acid). GW 1929, GW 7647, and WY 14643 are commercially available, for example, from Koma Biotechnology, Inc. (Seoul, Korea). In one embodiment, the PPARγ agonist is 15-deoxy-Δ-12, 14-PG J2.

Non-limiting examples of PPAR-α agonists include the fibrates, such as fenofibrate and gemfibrozil. A non-limiting example of PPAR-δ agonist is GW501516 (available from Axxora LLC, San Diego, California or EMD Biosciences, Inc., San Diego, California).

A composition of the present invention may further comprise a non-ionic surfactant, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween@ 80, Tween® 60, Tween® 20), poloxamers (synthetic block polymers of ethylene oxide and propylene oxide, such as those commonly known by their trade names of Pluronic®; e.g., Pluronic® F127 or Pluronic® F108)), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34^{th} ed., pp. 1411-1416 (Martindale, "The Complete Drug Reference," S. C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006); The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1, or from about 0.01 to about 0.5 weight percent).

In addition, a composition of the present invention can include additives such as buffers, diluents, carriers, adjuvants, or other excipients. Any pharmacologically acceptable buffer suitable for application to the eye may be used. Other agents may be employed in the composition for a variety of purposes. For example, buffering agents, preservatives, co-solvents, oils, humectants, emollients, stabilizers, or antioxidants may be employed. Water-soluble preservatives which may be employed include sodium bisulfite, sodium bisulfate, sodium thiosulfate, benzalkonium chloride, chlorobutanol, thimerosal, ethyl alcohol, methylparaben, polyvinyl alcohol, benzyl alcohol, and phenylethyl alcohol. These agents may be present in individual amounts of from about 0.001 to about 5% by weight (preferably, about 0.01% to about 2% by weight). Suitable water-soluble buffering agents that may be employed are sodium carbonate, sodium borate, sodium phosphate, sodium acetate, sodium bicarbonate, etc., as approved by the United States Food and Drug Administration ("US FDA") for the desired route of administration. These agents may be present in amounts sufficient to maintain a pH of the system of between about 2 and about 11. As such, the buffering agent may be as much as about 5% on a weight to weight basis of the total composition. Electrolytes such as, but not limited to, sodium chloride and potassium chloride may also be included in the formulation.

The pH of the composition may be in the range from about 4 to about 11. Alternatively, the pH of the composition may be in the range from about 5 to about 9, from about 6 to about 9, or from about 6.5 to about 8. The composition may comprise a buffer having a pH in one of said pH ranges.

The composition may have a pH of about 7. Alternatively, the composition may have a pH in a range from about 7 to about 7.5.

The composition may have a pH of about 7.4.

A composition may also comprise a viscosity-modifying compound designed to facilitate the administration of the composition into the subject or to promote the bioavailability in the subject. The viscosity-modifying compound may be chosen so that the composition is not readily dispersed after being administered into an environment of an eye. Such compounds may enhance the viscosity of the composition, and include, but are not limited to: monomeric polyols, such as, glycerol, propylene glycol, ethylene glycol; polymeric polyols, such as, polyethylene glycol; various polymers of the cellulose family, such as hydroxypropylmethyl cellulose ("HPMC"), carboxymethyl cellulose ("CMC") sodium, hydroxypropyl cellulose ("HPC"); polysaccharides, such as hyaluronic acid and its salts, chondroitin sulfate and its salts, dextrans, such as, dextran 70; water soluble proteins, such as gelatin; vinyl polymers, such as, polyvinyl alcohol, polyvinylpyrrolidone, povidone; carbomers, such as carbomer 934P, carbomer 941, carbomer 940, or carbomer 974P; and acrylic acid polymers. In general, a desired viscosity can be in the range from about 1 to about 400 centipoises ("cps") or mPa.s.

A method for preparing a composition of the present invention may comprise combining: (i) at least a DIGRA, a pharmaceutically acceptable salt thereof, or a pharmaceutically acceptable ester thereof; and (ii) a pharmaceutically acceptable carrier.

A method for preparing a composition of the present invention may comprise combining: (i) at least a DIGRA, a pharmaceutically acceptable salt thereof, or a pharmaceutically acceptable ester thereof; and (ii) an antagonist to at least a TGF-β isoform; and (iii) a pharmaceutically acceptable carrier. In one embodiment, such a carrier can be a sterile saline solution or a physiologically acceptable buffer. In another embodiment, such a carrier comprises a hydrophobic medium, such as a pharmaceutically acceptable oil. In still another embodiment, such as carrier comprises an emulsion of a hydrophobic material and water.

Physiologically acceptable buffers include, but are not limited to, a phosphate buffer or a Tris-HCl buffer (comprising tris(hydroxymethyl)aminomethane and HCl). For example, a Tris-HCl buffer having pH of 7.4 comprises 3 g/l of tris(hydroxymethyl)aminomethane and 0.76 g/l of HCl. The buffer may be 10X phosphate buffer saline ("PBS") or 5X PBS solution.

Other buffers also may be found suitable or desirable in some circumstances, such as buffers based on HEPES (N-{2-hydroxyethyl}peperazine-N'-{2-ethanesulfonic acid}) having pKₐ of 7.5 at 25 °C and pH in the range of about 6.8-8.2; BES (N,N-bis{2-hydroxyethyl)2-aminoethanesulfonic acid) having pKₐ of 7.1 at 25°C and pH in the range of about 6.4-7.8; MOPS (3-{N-morpholino}propanesulfonic acid) having pKₐ of 7.2 at 25°C and pH in the range of about 6.5-7.9; TES (N-tris(hydroxymethyl }-methyl-2-aminoethanesulfonic acid) having pKₐ of 7.4 at 25°C and pH in the range of about 6.8-8.2; MOBS (4-(N-morpholino)butanesulfonic acid) having pKₐ of 7.6 at 25°C and pH in the range of about 6.9-8.3; DIPSO (3-(N,N-bis{2-hydroxyethyl}amino)-2-hydroxypropane)) having pKₐ of 7.52 at 25°C and pH in the range of about 7-8.2; TAPSO (2-hydroxy-3{tris(hydroxymethyl)methylamino}-1-propanesulfonic acid)) having pKₐ of 7.61 at 25°C and pH in the range of about 7-8.2; TAPS ({(2-hydroxy-1,1-bis(hydroxymethyl)ethyl)amino}-1-propanesulfonic acid)) having pKₐ of 8.4 at 25°C and pH in the range of about 7.7-9.1; TABS (N-tris(hydroxymethyl)methyl-4-aminobutanesulfonic acid) having pKₐ of 8.9 at 25°C and pH in the range of about 8.2-9.6; AMPSO (N-(1,1-dimethyl-2-hydroxyethyl)-3-amino-2-hydroxypropanesulfonic acid)) having pKₐ of 9.0 at 25°C and pH in the range of about 8.3-9.7; CHES (2-cyclohexylamino)ethanesulfonic acid) having pKₐ of 9.5 at 25°C and pH in the range of about 8.6-10.0; CAPSO (3-(cyclohexylamino)-2-hydroxy-1-propanesulfonic acid) having pKₐ of 9.6 at 25°C and pH in the range of about 8.9-10.3; or CAPS (3-(cyclohexylamino)-I-propane sulfonic acid) having pKₐ of 10.4 at 25°C and pH in the range of about 9.7-11.1.

In certain embodiments, a composition of the present invention is formulated in a buffer having an acidic pH, such as from about 4 to about 6.8, or alternatively, from about 5 to about 6.8. In such embodiments, the buffer capacity of the composition desirably allows the composition to come rapidly to a physiological pH after being administered into the patient.

It should be understood that the proportions of the various components or mixtures in the following examples may be modified for the appropriate circumstances.

### EXAMPLE 1 (comparative example)

Two mixtures I and II are made separately by mixing the ingredients listed in Table 1. Five parts (by weight) of mixture I are mixed with one part (by weight) of mixture II for 15 minutes or more. The pH of the combined mixture is adjusted to 6.2-6.4 using 1 N NaOH or 1 N HCl to yield a composition of the present invention.

**Table 1**

| Ingredient | | Amount |
|---|---|---|
| Mixture I | | |
| | Carbopol 934P NF | 0.25 g |
| | Purified water | 99.75 g |

| Mixture II | | |
|---|---|---|
| | Propylene glycol | 5 g |
| | EDTA | 0.1 mg |
| | Compound of Formula IV HCl | 0.5 g |

Alternatively, purified water may be substituted with an oil, such as fish-liver oil, peanut oil, sesame oil, coconut oil, sunflower oil, corn oil, or olive oil to produce an oil-based formulation comprising a compound of Formula IV.

### EXAMPLE 2 (comparative example)

Two mixtures I and II are made separately by mixing the ingredients listed in Table 2. Five parts (by weight) of mixture I are mixed with two parts (by weight) of mixture II for 15 minutes or more. The pH of the combined mixture is adjusted to 6.2-6.4 using 1 N NaOH or 1 N HCl to yield a composition of the present invention.

**Table 2**

| Ingredient | | Amount |
|---|---|---|
| Mixture I | | |
| | Diclofenac | 0.3g |
| | Carbopol 934P NF | 0.25 g |
| | Purified water | 99.25 g |

| Mixture II | | |
|---|---|---|
| | Propylene glycol | 5 g |
| | EDTA | 0.1 mg |
| | Compound of Formula IV | 0.5 g |

Alternatively, purified water may be substituted with an oil, such as fish-liver oil. peanut oil, sesame oil, coconut oil, sunflower oil, corn oil, or olive oil to produce an oil-based formulation comprising a compound of Formula IV.

### EXAMPLE 3

Two mixtures I and II are made separately by mixing the ingredients listed in Table 3. Five parts (by weight) of mixture I are mixed with two parts (by weight) of mixture II for 15 minutes or more. The pH of the combined mixture is adjusted to 6.2-6.4 using 1 N NaOH or 1 N HCl to yield a composition of the present invention.

**Table 3**

| Ingredient | | Amount |
|---|---|---|
| Mixture I | | |
| | TGF-β antibody ab300838 (from Abcam, Inc.) | 0.2g |
| | Carbopol 934P NF | 0.25 g |
| | Purified water | 99.35 g |

| Mixture II | | |
|---|---|---|
| | Propylene glycol | 3 g |
| | Compound of Formula II | 0.25 g |
| | EDTA | 0.1 mg |

### EXAMPLE 4:

Two mixtures I and II are made separately by mixing the ingredients listed in Table 4. Five parts (by weight) of mixture I are mixed with one part (by weight) of mixture II for 15 minutes or more. The pH of the combined mixture is adjusted to 6.2-6.4 using 1 N NaOH or 1 N HCl to yield a composition of the present invention.

**Table 4**

| Ingredient | | Amount |
|---|---|---|
| Mixture I | | |
| | CAT-152 TGF-β antibody (disclosed in A.L. Mead, et al., *supra*) | 0.3g |
| | Carbopol 934P NF | 0.25 g |
| | Olive oil | 99.15 g |

| Mixture II | | |
|---|---|---|
| | Propylene glycol | 7 g |
| | Glycerin | 3 g |
| | Compound of Formula III | 1 g |
| | HAP (30%) | 0.5 mg |
| | Alexidine 2HCl | 1-2 ppm |

| | | |
|---|---|---|
| Note: "HAP" denotes hydroxyalkyl phosphonates, such as those known under the trade name Dequest®. | | |

### EXAMPLE 5: (comparative example)

The ingredients listed in Table 5 are mixed together for at least 15 minutes. The pH of the mixture is adjusted to 6.2-6.4 using 1 N NaOH or 1 N HCl to yield a composition of the present invention.

**Table 5**

| Ingredient | Amount (% by weight, except where "ppm" is indicated) |
|---|---|
| Povidone | 1 |
| HAP (30%) | 0.05 |
| Glycerin | 3 |
| Propylene glycol | 3 |
| Compound of Formula IV | 0.5 |
| Tyloxapol | 0.25 |
| BAK | 10-100 ppm |
| Purified water | q.s. to 100 |

| | |
|---|---|
| Note: "BAK" denotes benzalkonium chloride. | |

EXAMPLE 6:

The ingredients listed in Table 6 are mixed together for at least 15 minutes. The pH of the mixture is adjusted to 6.2-6.4 using 1 N NaOH or 1 N HCl to yield a composition of the present invention.

**Table 6**

| Ingredient | Amount (% by weight, except where "ppm" is indicated) |
|---|---|
| Povidone | 1.5 |
| HAP (30%) | 0.05 |
| Glycerin | 3 |
| Propylene glycol | 3 |
| Compound of Formula IV | 0.75 |
| TGF-β antibody ab 18679 (from Abcam, Inc., Cambridge, Massachusetts) | 0.1 |
| Tyloxapol | 0.25 |
| Alexidine 2HCl | 1-2 ppm |
| Purified water | q.s. to 100 |

### EXAMPLE 7: (comparative example)

The ingredients listed in Table 7 are mixed together for at least 15 minutes. The pH of the mixture is adjusted to 6.2-6.4 using 1 N NaOH or 1 N HCl to yield a composition of the present invention.

**Table 7**

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.25 |
| Ketorolac | 0.3 |
| Tyloxapol (a surfactant) | 0.25 |
| Alexidine 2HCl | 1-2 ppm |
| Sunflower oil | q.s. to 100 |

### EXAMPLE 8:

The ingredients listed in Table 8 are mixed together for at least 15 minutes. The pH of the mixture is adjusted to 6.2-6.4 using 1 N NaOH or 1 N HCl to yield a composition of the present invention.

**Table 8**

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.3 |
| TGF-β antibody ab49574 (from Abcam, Inc., Cambridge, Massachusetts) | 0.2 |
| Diclofenac sodium | 0.3 |
| Tyloxapol (a surfactant) | 0.25 |
| Alexidine 2HCl | 1-2 ppm |
| Purified water | q.s. to 100 |

### EXAMPLE 9:

The ingredients listed in Table 9 are mixed together for at least 15 minutes. The pH of the mixture is adjusted to 6.2-6.4 using 1 N NaOH or 1 N HCl to yield a composition of the present invention.

**Table 9**

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.5 |
| sTGFRI | 0.2 |
| sTGFRII | 0.2 |
| Tyloxapol (a surfactant) | 0.25 |
| Alexidine 2HCl | 1-2 ppm |
| Corn oil | q.s. to 100 |

A DIGRA, a pharmaceutically acceptable salt thereof, or a pharmaceutically acceptable ester thereof may be incorporated into a formulation for topical administration, systemic administration, periocular injection, or subconjunctival injection. An antagonist to TGF-β may be included in the formulation. A formulation can desirably comprise a carrier that provides a sustained-release of the active ingredients, such as for a period longer than about 1 week (or longer than about 1, 2, 3, 4, 5, or 6 months). In certain embodiments, the sustained-release formulation desirably comprises a carrier that is insoluble or only sparingly soluble in the ocular environment. Such a carrier can be an oil-based liquid, emulsion, gel, or semisolid. Non-limiting examples of oil-based liquids include castor oil, peanut oil, olive oil, coconut oil, sesame oil, cottonseed oil, corn oil, sunflower oil, fish-liver oil, arachis oil, and liquid paraffin.

In one embodiment, a compound or composition of the present invention can be injected subconjunctivally after GFS to control or prevent the progression of glaucoma, using a fine-gauge needle, such as 25-30 gauge. Typically, an amount from about 25 µl to about 100 µl of a composition comprising a DIGRA, a pharmaceutically acceptable salt thereof, or a pharmaceutically acceptable ester thereof is administered into a patient. A concentration of such DIGRA, or pharmaceutically acceptable salt thereof is selected from the ranges disclosed above.

A DIGRA, a pharmaceutically acceptable salt thereof, or a pharmaceutically acceptable ester thereof may be incorporated into an ophthalmic device or system that comprises a biodegradable material, and the device is injected or implanted into the vicinity of the filtering bleb to provide a long-term (e.g., longer than about I week, or longer than about 1, 2, 3, 4, 5, or 6 months) control or prevention of progression of glaucoma. Such a device or system may be injected or implanted by a skilled physician in the subject's subtenon space or periocular tissue. Such control or prevention may be achieved by ensuring a long-term functioning filtering bleb.

The use of a DIGRA, a pharmaceutically acceptable salt thereof, or a pharmaceutically acceptable ester thereof, for the preparation of a medicament for controlling or preventing progression of glaucoma, wherein the administration of the medicament may comprise: (a) providing a composition comprising a DIGRA, a pharmaceutically acceptable salt thereof, or a pharmaceutically acceptable ester thereof; and (b) administering to a subject an effective amount of the composition at a frequency sufficient to ensure a functioning filtering bleb, thereby controlling or preventing the progression of glaucoma.

The use of a DIGRA, a pharmaceutically acceptable salt thereof, or a pharmaceutically acceptable ester thereof, for the preparation of a medicament for controlling or preventing progression of glaucoma in a subject, wherein the administration of the medicament may comprise: (a) performing a GFS procedure on the subject; (b) providing a composition comprising a DIGRA, a pharmaceutically acceptable salt thereof, or a pharmaceutically acceptable ester thereof; and (c) administering to a subject an effective amount of the composition at a frequency sufficient to ensure a functioning filtering bleb, thereby controlling or preventing the progression of glaucoma.

In one embodiment, the DIGRA is selected from among those disclosed above.

In still another embodiment, the present invention provides the use of a DIGRA, a pharmaceutically acceptable salt thereof, or a pharmaceutically acceptable ester thereof, for the preparation of a medicament for lowering IOP in a subject. The administration of the medicament may comprise: (a) performing a GFS procedure on the subject; (b) providing a composition comprising a DIGRA, a pharmaceutically acceptable salt thereof, or a pharmaceutically acceptable ester thereof; and (c) administering to a subject an effective amount of the composition at a frequency sufficient to ensure a functioning filtering bleb, thereby lowering said IOP.

In another embodiment, the composition for use in any of the foregoing method further comprises an antagonist to TGF-β. Such an antagonist to TGF-β is selected from those disclosed above. The concentrations of the DIGRA, a pharmaceutically acceptable salt thereof, a pharmaceutically acceptable ester thereof, and the antagonist to TGF-β are selected to be in the ranges disclosed above.

A composition of the present invention may be administered topically or periocularly. A composition of the present invention may be incorporated into an ophthalmic implant system or device, and the implant system or device is surgically implanted periocularly in, or in the subtenon space of, the patient for the sustained or long-term release of the active ingredient or ingredients. A typical implant system or device suitable for use in a method of the present invention comprises a biodegradable matrix with the active ingredient or ingredients impregnated or dispersed therein. Non-limiting examples of ophthalmic implant systems or devices for the sustained-release of an active ingredient are disclosed in U.S. Patents 5,378,475; 5,773,019; 5,902,598; 6,001,386; 6,051,576; and 6,726,918;

A composition of the present invention may be administered topically once a day, several (e.g., twice, three, four, or more) times a day, once a week, once a month. The composition may be implanted in the patient and may be replaced at a frequency of, for example, once a year, twice a year, four times a year, or at a suitable frequency that is determined to be appropriate for controlling or preventing the progression of glaucoma.

### COMBINATION THERAPY

A composition or the use of a DIGRA, a pharmaceutically acceptable salt thereof, or a pharmaceutically acceptable ester thereof, for the preparation of a medicament of the present invention can be used with other therapeutic and adjuvant or prophylactic agents commonly used to reduce, treat, or prevent (a) an increase of intraocular pressure, (b) a loss of retinal ganglion cells, or (c) both, thus providing an enhanced overall treatment or enhancing the effects of the other therapeutic agents, prophylactic agents, and adjunctive agents used to treat and manage the different types of glaucoma. Therapeutic agents used to treat narrow angle or acute congestive glaucoma include, for example, physostigmine salicylate and pilocarpine nitrate. Adjunctive therapy used in the management of narrow angle glaucoma includes, for example, the intravenous administration of a carbonic anhydrase inhibitor such as acetozolamide to reduce the secretion of aqueous humor, or of an osmotic agent such as mannitol or glycerin to induce intraocular dehydration. Therapeutic agents used to manage wide angle or chronic simple glaucoma and secondary glaucoma include, for example, prostaglandin analogs, such as Xalatan® and Lumigan®, β-adrenergic antagonists such as timolol maleate, α-adrenergic agonists, such as brimonidine and apraclonidine, muscarinic cholinergic agents (such as pilocarpine or carbachol), and carbonic anhydrase inhibitors, such as Dorzolamide (Trusopt® or Cosopt®) or brizolamide (Azopt®). Other therapeutic agents used to manage glaucoma include the inhibitors of acetylcholinesterase such as Echothiophate (phospholine iodide).

High doses may be required for some currently used therapeutic agents to achieve levels to effectuate the target response, but may often be associated with a greater frequency of dose-related adverse effects. Thus, combined use of the compounds or compositions of the present invention, following GFS, with agents commonly used to treat glaucoma allows the use of relatively lower doses of such other agents, resulting in a lower frequency of potential adverse side effects associated with long-term administration of such therapeutic agents. Thus, another indication of the compounds or compositions in this invention is to reduce adverse side effects of prior-art drugs used to treat glaucoma, such as the development of cataracts with long-acting anticholinesterase agents including demecarium, echothiophate, and isoflurophate.

### COMPARISON OF SIDE EFFECTS OF GLUCOCORTICOIDS AND DIGRAS

Side effects of glucocorticoids and DIGRAs may be compared in their use as adjunctive therapy following GFS.

A level of at least an adverse side effect may be determined in vivo or in vitro. For example, a level of said at least an adverse side effect is determined in vitro by performing a cell culture and determining the level of a biomarker associated with said side effect. Such biomarkers can include proteins (e.g., enzymes), lipids, sugars, and derivatives thereof that participate in, or are the products of, the biochemical cascade resulting in the adverse side effect. Representative in vitro testing methods are further disclosed hereinbelow.

In another embodiment, a level of said at least an adverse side effect is determined in vivo at about one day after said glucocorticoid or DIGRA (or a prodrug thereof, a pharmaceutically acceptable salt thereof, or a pharmaceutically acceptable ester thereof) is first administered to, and are present in, said subject. In another embodiment, a level of said at least an adverse side effect is determined about 14 days after said composition is first administered to, and are present in, said subject. In still another embodiment, a level of said at least an adverse side effect is determined about 30 days after said composition is first administered to, and are present in, said subject. Alternatively, a level of said at least an adverse side effect is determined about 2, 3, 4, 5, or 6 months after said compounds or compositions are first administered to, and are present in, said subject.

Said glucocorticoid used to treat said exemplary inflammation may be administered to said subject at a dose and a frequency sufficient to produce a beneficial effect on said inflammation equivalent to a compound or composition of the present invention after about the same elapsed time.

One of the most frequent undesirable actions of a glucocorticoid therapy (such as anti-inflammation therapy) is steroid diabetes. The reason for this undesirable condition is the stimulation of gluconeogenesis in the liver by the induction of the transcription of hepatic enzymes involved in gluconeogenesis and metabolism of free amino acids that are produced from the degradation of proteins (catabolic action of glucocorticoids). A key enzyme of the catabolic metabolism in the liver is the tyrosine aminotransferase ("TAT"). The activity of this enzyme can be determined photometrically from cell cultures of treated rat hepatoma cells. Thus, the gluconeogenesis by a glucocorticoid can be compared to that of a DIGRA by measuring the activity of this enzyme. For example, in one procedure, the cells are treated for 24 hours with the test substance (a DIGRA or glucocorticoid), and then the TAT activity is measured. The TAT activities for the selected DIGRA and glucocorticoid are then compared. Other hepatic enzymes can be used in place of TAT, such as phosphoenolpyruvate carboxykinase, glucose-6-phosphatase, or fructose-2,6-biphosphatase. Alternatively, the levels of blood glucose in an animal model may be measured directly and compared for individual subjects that are treated with a glucocorticoid for a selected condition and those that are treated with a DIGRA for the same condition.

Another undesirable result of glucocorticoid therapy is GC-induced cataract. The cataractogenic potential of a compound or composition may be determined by quantifying the effect of the compound or composition on the flux of potassium ions through the membrane of lens cells (such as mammalian lens epithelial cells) in vitro. Such an ion flux may be determined by, for example, electrophysiological techniques or ion-flux imaging techniques (such as with the use of fluorescent dyes). An exemplary in-vitro method for determining the cataractogenic potential of a compound or composition is disclosed in U.S. Patent Application Publication 2004/0219512.

Still another undesirable result of glucocorticoid therapy is hypertension. Blood pressure of similarly matched subjects treated with glucocorticoid and DIGRA following GFS may be measured directly and compared.

### TESTING 1: Effect of BOL-303242-X (Compound Having Formula IV) on Inhibiting IL-1β-Induced Cytokine Expression in Human Corneal Epithelial Cells

### 1. BACKGROUND/RATIONALE:

Levels of cytokines associated with immune cells are direct indications of activity of these cells in an inflammatory condition. Reduced levels of these cytokines indicate a positive therapeutic effect on inflammation of a test compound. This study was designed to determine the effect of BOL-303242-X on IL-1ß -induced cytokine production in human corneal epithelial cells ("HCECs").

### 2. PURPOSE

To determine the effects of BOL-303242-X on IL-1ß-stimulated cytokine expression in primary human corneal epithelial cells using a 30-cytokine Luminex kit. Dexamethasone was used as a control.

### 3. EXPERIMENTAL DESIGN

Primary HCECs were seeded in 24-well plates. After 24 h, cells were treated with vehicle, IL-1ß, IL-1ß + dexamethasone, or IL-1ß + BOL-303242-X in basic EpiLife medium for 18 h (Table T-1-1). Each treatment was performed in triplicate. Media were collected and used for determination of cytokine content using a 30-cytokine Luminex kit. Cell viability was determined by alamarBlue assay (LP06013).

| Group* | Day 1 | Day 2: cells were treated with the test agents in basic EpiLife medium for 18 h | Day 3 |
|---|---|---|---|
| 1 | Cells were seeded in 24-well plates (5 X 10⁵/well in 0.5 ml medium) in EpiLife medium | Control (0. 1% DMSO) | Media for Luminex assays; cells for cell viability assay |
| 2 | | 10 ng/ml IL-1ß | |
| 3 | | 10 ng/ml IL-1ß + I nM dexamethasone | |
| 4 | | 10 ng/ml IL-1ß + 10 nM dexamethasone | |
| 5 | | 10 ng/ml IL-1ß + 100 nM dexamethasone | |
| 6 | | 10 ng/ml IL-1ß + 1 µM dexamethasone | |
| 7 | | 10 ng/ml IL-1ß + 10 µM dexamethasone | |
| 8 | | 10 ng/ml IL-1ß + 1 nM BOL-303242-X | |
| 9 | | 10 ng/ml IL-1ß + 10 nM BOL-303242-X | |
| 10 | | 10 ng/ml IL-1ß + 100 nM BOL-303242-X | |
| 11 | | 10 ng/ml IL-1ß + 1 µM BOL-303242-X | |
| 12 | | 10 ng/ml IL-1ß + 10 µM BOL-303242-X | |

| | | | |
|---|---|---|---|
| *triplicate wells per group Dexamethasone: Lot Number: 016K14521 Parent MW: 392.46 Parent:Total MW Ratio = 1.0 BOL-303242-X: Lot Number: 6286 Parent MW: 462.48 Parent:Total MW Ratio = 1.0 | | | |

### 4. DATA ANALYSIS

Median fluorescence intensity (MFI) was used to obtain the concentration of each cytokines in pg/ml based on the standard curve of each cytokine assayed by Luminex. The linear range of the standard curve for each cytokine was used for determination of cytokine concentration. Duplicate values for each sample were averaged. Data were expressed as mean ± SD. Statistical analysis was performed using one-way ANOVA-Dunnett's test, and P < 0.05 was considered statistically significant.

### 5. RESULTS

No statistically significant effect on cellular metabolic activity (as measured by alamarBlue assay) was observed with the various treatments.

Substantial amounts of 16 out of 30 cytokines tested were detected in this study and 13 out of 14 cytokines detected were stimulated by 10 ng/ml IL-1ß (Table T-1-1). IL-1β was excluded from analysis because it was the stimulus. IL-1ra was excluded because the MFI was not within the standard range.

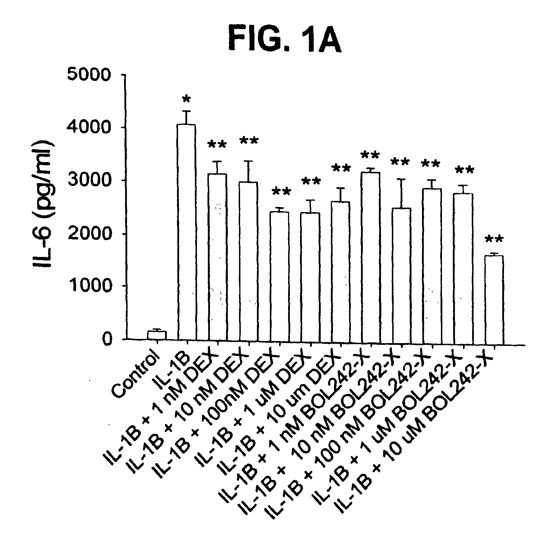

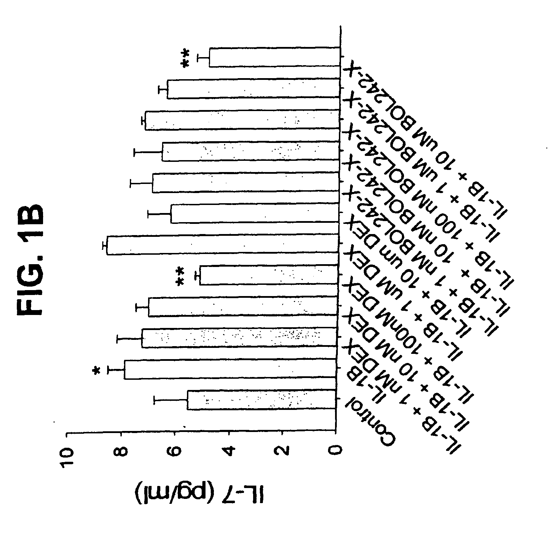

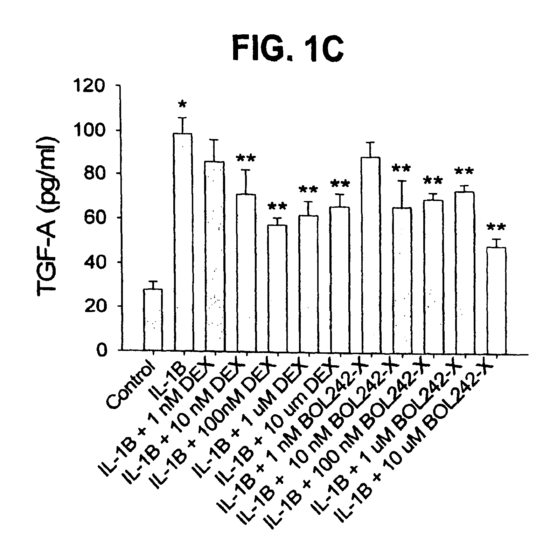

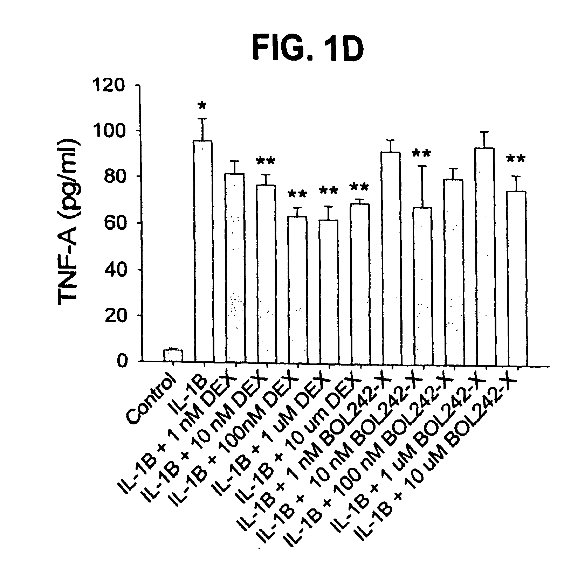

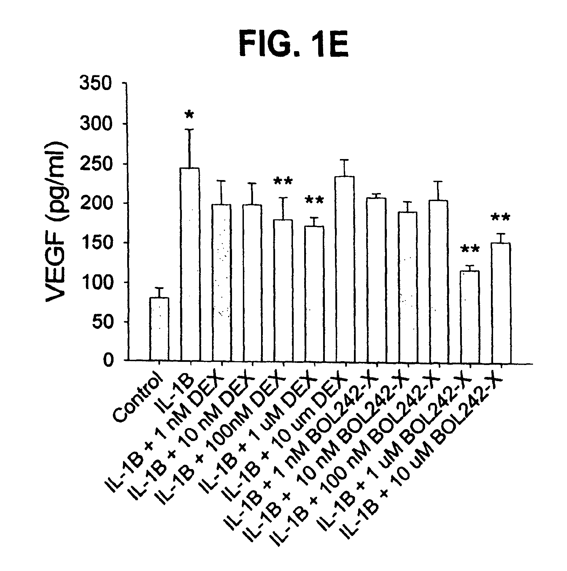

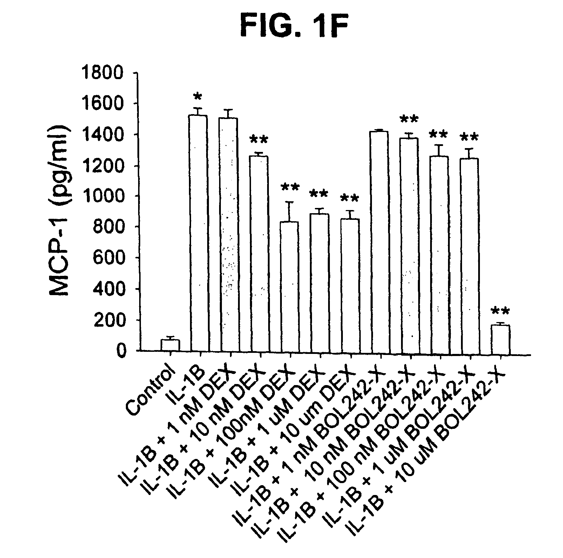

Dexamethasone and BOL-303242-X significantly inhibited IL-1ß-stimulated cytokine production with comparable potency on 6 cytokines (IL-6, IL-7, MCP-1, TGF-α, TNF-α and VEGF), and a significant inhibitory effect was observed at 1 nM on IL-6 and at 10 nM on MCP-1, TGF-α and TNF-α (Table T-1-1 and Figures 1A-1F). As discussed above, these cytokines can induce the production of TGF-β by, for example, immune cells, leading to excessive matrix deposition and ultimately a non- or poorly functioning filtering bleb after GFS. The present data indicate that BOL-303242-X can be an effective and improved alternative to GCs that have been used as pharmacological adjunctive therapy associated with GFS.

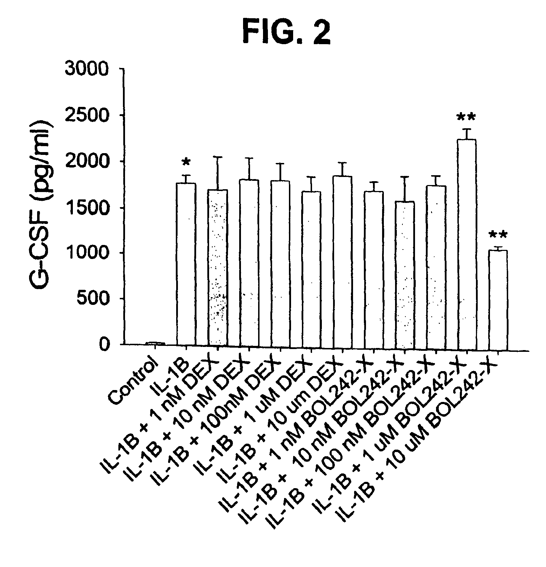

BOL-303242-X also significantly inhibited IL-1ß-stimulated G-CSF production with better potency compared to dexamethasone, and a significant inhibitory effect was observed at 10 µg/ml by BOL-303242-X while no significant effect was observed by dexamethasone on this cytokine (Fig. 2).

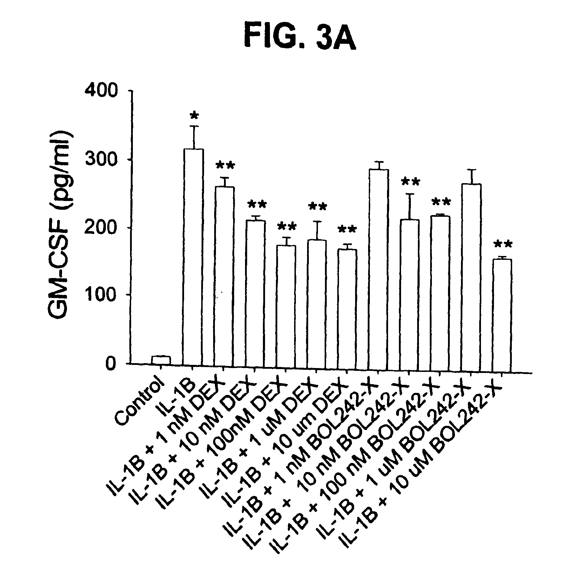

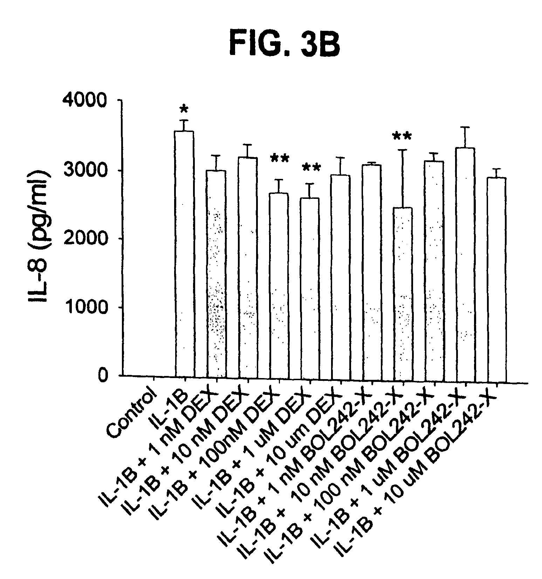

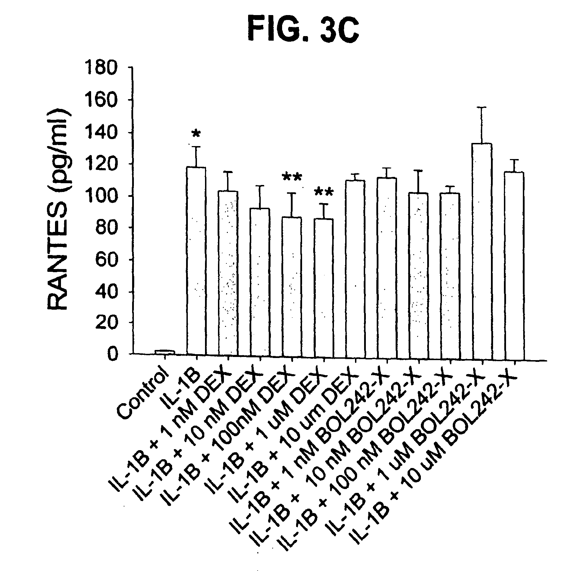

BOL-303242-X also significantly inhibited IL-1ß-stimulated cytokine production with less potency compared to dexamethasone on 3 cytokines (GM-CSF, IL-8, and RANTES). A significant inhibitory effect was observed at 1 nM by dexamethasone and at 10 nM by BOL-303242-X on GM-CSF. A significant inhibitory effect was observed at 1 µM by dexamethasone on RANTES while no significant effect was observed by BOL-303242-X on this cytokine (Figures 3A-3C).

### 6. CONCLUSION

BOL-303242-X and dexamethasone have comparable potency for inhibition of IL-1ß-stimulated cytokine production in HCECs for the cases of IL-6, IL-7, TGF-α, TNF-α, VGEF, and MCP-1. BOL-303242-X is more potent than dexamethasone in inhibiting IL-1ß-stimulated production of G-CSF in HCECs. BOL-303242-X is somewhat less potent than dexamethasone in inhibiting IL-1ß-stimulated production of GM-CSF, IL-8, and RANTES in HCECs.

**Table T-1-1**

| Inhibition of IL-1β stimulated cytokine production by dexamethasone and BOL-303242-X in primary human corneal epithelial cells | | | | | | | | | | | |
|---|---|---|---|---|---|---|---|---|---|---|---|
| Cytokines detected * | Stimulated by IL-1β (10 ng/ml) | Inhibited by dexamethasone (µM) | | | | | Inhibited by BOL-303242-X (µM) | | | | |
| | | 0.0 01 | 0.0 1 | 0.1 | 1 | 10 | 0.0 01 | 0.0 1 | 0.1 | 1 | 10 |
| G-CSF | X | | | | | | | | | | X |
| GM-CSF | X | X | X | X | X | X | | X | X | | X |
| IL-1α | X | | | | | | | | | | |
| IL-6 | X | X | X | X | X | X | X | X | X | X | X |
| IL-7 | | | | X | | | | | | | X |
| IL-8 | X | | | X | X | | | X | | | |
| IP-10 | X | | | | | | | | | | |
| MCP-1 | X | | X | X | X | X | | X | X | X | X |
| MIP-1α | | | | | | | | | | | |
| MIP-1β | X | | | | | | | | | | |
| RANTES | X | | | | X | X | | | | | |
| TGF-α | X | | X | X | X | X | | X | X | X | X |
| TNF-α | X | | X | X | X | X | | X | | | X |
| VEGF | X | | | X | X | | | | | X | X |

| | | | | | | | | | | | |
|---|---|---|---|---|---|---|---|---|---|---|---|
| Notes: (*) EGF, Eotaxin, Fractalkine, IFNγ, IL-10, IL-12p40, IL-12p70, IL-13, IL15, IL-17, IL-2, IL-4, IL-5, sCD40L were not detected. IL-1β was excluded from analysis because it was the stimulus. IL-1ra was excluded because the MFI was out of range of the standards. | | | | | | | | | | | |

### TESTING 2: Evaluation Of The Effect Of Topical BOL-303242-X, Administered Unilaterally Four Times Daily, On The Intraocular Pressure in New Zealand White Rabbits For 33 Days

### INTRODUCTION

The objective of this study was to evaluate the effect of topical BOL-303242-X on the IOP in New Zealand White rabbits when administered to right eyes four times daily for 33 days. Dosing was discontinued after 31 days due to high mortality rates and limited supply of test articles.

### MATERIALS AND METHODS

### Test Articles

Three test articles were identified as follows:
10 mg/g BOL-303242-X (compound having Formula IV) Ophthalmic Suspension (Lot No. 2676-MLC-270)
5 mg/g BOL-303242-X Ophthalmic Suspension (Lot No. 2676-MLC-270)
1 mg/g BOL-303242-X Ophthalmic Suspension (Lot No. 2676-MLC-270)

A negative control (balanced salt solution (BSS), B. Braun Medical Inc., Lot No. J6N011, exp. 10/08), and a positive control (0.1 % dexamethasone ophthalmic suspension (Maxidex^{®}, Alcon Laboratories, Inc., Lot No. 114619F, exp. 01/09)) were also provided. The formulations were provided in ready-to-use form and stored at room temperature. The suspensions were shaken before dose administrations to re-suspend them.

### Test System

### Animals

Seventy-five female New Zealand White rabbits were obtained from The Rabbit Source (Ramona, CA). Animals were 6-8 weeks old at the time of IOP-training initiation, and they weighed 1.38-2.05 kg at randomization. The protocol specified that animals would weigh at least 1.5-2.5 kg; this deviation had no effect on the outcome of the study. Animals were identified by ear tags and cage cards.

### Animal Husbandry

Upon arrival, animals were examined to ensure that they were healthy and quarantined for 10 days before placement on study. At the end of the quarantine period, animals were again examined for general health parameters and for any anatomical ophthalmic abnormalities. Quarantine was conducted according to internal operating procedure.

Animals were housed in individual, hanging, stainless steel cages. Housing and sanitation were performed according to internal operating procedure.

Animals were provided Teklad Certified Global High Fiber Rabbit Diet. Diet certification and analysis were provided by the vendor, Harlan Teklad. No analyses outside those provided by the manufacturer were performed. Animals were provided tap water *ad libitum*. No contaminants were known to exist in the water and no additional analyses outside those provided by the local water district and as specified in internal operating procedure were performed.

Environmental parameters were monitored according to internal operating procedure. The study room temperature was 65-72°F with 58-77% relative humidity Pre-Treatment Examinations

Prior to placement on study, each animal underwent a pre-treatment ophthalmic examination (slit lamp and indirect ophthalmoscopy). Observations were scored according to the McDonald Shadduck system and recorded using a standardized data collection sheet. Acceptance criteria for placement on study were as follows: Scores of ≤ 1 for conjunctival congestion and swelling; scores of 0 for all other observation variables.

### IOP Conditioning and Pre-Selection

Seventy-five rabbits underwent two weeks of IOP training to condition them for IOP measurement. IOP was determined for both eyes of each animal using a Medtronic Solan, Model 30 classic pneumatonometer. Proparacaine hydrochloride 0.5% (1 drop) was delivered to each eye prior to IOP measurement. A two-point diurnal curve was established: IOP was recorded on Monday, Wednesday, and Friday of each week, at 8 a.m. and 12 p.m., with a ± 1 hour range for each of these times. The time of the measurements was recorded.

At the end of the two weeks of conditioning, 50 rabbits were selected for topical dosing based on the consistency of their IOP measurements at each time point. The selected rabbits continued to have their IOPs measured for one additional week.

### Randomization

Prior to dosing, 50 animals were weighed and randomly assigned to five treatment groups. Treatment groups are described in Table T-2-1. Animals were randomized to treatment groups according to a modified Latin square.

### Topical Dosing Procedure

On Days 1-31, animals received daily topical doses of the appropriate test article into the right eye. Animals were dosed four times per day, with doses administered 2 hours apart. Doses were administered using a calibrated 50-µL pipette. The eyelids were held close for 10 seconds immediately following dosing. The time of each dose administration was recorded.

The protocol indicated that animals would be dosed four times daily for 33 days. Per decision of the Sponsor and Study Director, dosing was discontinued after 31 days due to high mortality rates and limited supply of test articles. This deviation had no adverse effect on the outcome of the study.

### Mortal ity/Morbidity

Animals were observed for mortality/morbidity twice daily. Animals determined to be moribund were euthanized with an intravenous injection of commercial euthanasia solution.

### Body Weights

Animals were weighed at randomization.

### IOP Measurements

IOP was determined for both eyes of each animal on Days 3, 5, 10, 12, 16, 18, 22, 24, 26, 30, and 32. IOP was evaluated with a Medtronic Solan, Model 30 classic pneumatonometer. Proparacaine hydrochloride 0.5% (1 drop) was delivered to each eye prior to IOP measurement. IOP was measured on Monday, Wednesday, and Friday of each week. A two-point diurnal curve was established: IOP was recorded at 8 a.m. and 12 p.m. on Day 3, and at 8 a.m. and 2 p.m. on later days, with a ± 1 hour range for each of these times. The time of the measurements was recorded.

### Ophthalmic Observations

Ophthalmic examinations (slit lamp) were performed prior to the first dosing on Days 5, 12, 22, 26, and 33. Ocular findings were scored according to the McDonald Shadduck system and recorded using a standardized data collection sheet.

### Study Completion

Following completion of final ophthalmic observations (Day 33), remaining animals were returned to the vivarium.

### Statistical Analysis

Descriptive statistics were prepared for IOP data of each treatment group (left and right eyes separately) at each measurement interval. The statistics included the number of observations ("N"), mean, standard deviation ("STD"), and standard error ("SEM"). Statistical analyses were conducted on IOP results using Statistical Analysis Systems (SAS Institute, Inc., Cary, NC, V8.0). Parameters were evaluated using analysis of variance/GLM Procedure followed by Tukey's Standardized Range Test (Tukey, 1985) for post hoc comparisons of group means. The level of significance was set at a probability of p < 0.05 for all statistical procedures. Group IOP means were compared at each interval, with left and right eyes compared separately.

IOP data for the following six animals were excluded from group statistics: Group A, Nos. 3081, 3037, 3068, and 3011; Group C, No. 3034; and Group E, No. 3084. The excluded Group A animals showed no IOP response to dexamethasone dosing, and the excluded Group C and Group E animals had outlying IOP data.

### Animal Welfare Statement

This study was performed to develop a hypertensive model of intraocular pressure in New Zealand White rabbits. Alternatives to performing this study were explored; however, to properly develop the model, a whole-body test system was required. This study complied with all internal animal welfare policies and was approved by the Institutional Animal Care and Use Committee.

### RESULTS

### Mortality

Mortality data are presented in Table T-2-2. Ten rabbits died or were euthanized between Days 11 and 33, as follows: Six of ten rabbits dosed with dexamethasone, one of ten rabbits dosed with 10 mg/g BOL-303242-X (0.5 mg/dose), two of ten rabbits dosed with 5 mg/g BOL-303242-X (0.25 mg/dose), and one of ten rabbits dosed with 1 mg/g BOL-303242-X (0.05 mg/dose). Seven rabbits were noted to have diarrhea, often described as severe and hemorrhagic, prior to death or euthanasia. No signs of poor health were noted for two rabbits that were found dead. Further information on observed mortality is shown in the following table.

| Group | Rabbit No. | Treatment (4 x Daily) | Day of Death⁽¹⁾ | Recorded Notes |
|---|---|---|---|---|
| A | 3011 | 0.1% Dexamethasone (0.05 mg/dose) | 23 | Euthanized due to severe profuse hemorrhagic diarrhea. Noted to be malnourished and anorexic. |
| A | 3016 | 0.1% Dexamethasone (0.05 mg/dose) | 27 | Found dead. No rigor mortis present. |
| A | 3037 | 0. 1% Dexamethasone (0.05 mg/dose) | 25 | Euthanized due to severe hemorrhagic diarrhea. Noted to be dehydrated, lethargic, and cachectic. |
| A | 3038 | 0.1% Dexamethasone (0.05 mg/dose) | 13 | Euthanized due to severe hemorrhagic diarrhea. |
| A | 3068 | 0.1% Dexamethasone (0.05 mg/dose) | 25 | Euthanized due to severe hemorrhagic diarrhea. Noted to be dehydrated, lethargic, and cachectic. |
| A | 3086 | 0.1% Dexamethasone (0.05 mg/dose) | 27 | Euthanized. Very sick/poor health; left (untreated) eye protruding. |
| B | 3008 | 10 mg/g BOL-303242-X (0.5 mg/dose) | 11 | Found dead. Noted on Day 9 to have significant diarrhea and a yellowish discharge in the dosed eye. |
| C | 3028 | 5 mg/g BOL-303242-X (0.25 mg/dose) | 17 | Euthanized due to severe diarrhea. |
| C | 3074 | 5 mg/g BOL-303242-X (0.25 mg/dose) | 33 | Euthanized prior to final ocular examination due to a respiratory infection. Diarrhea noted on Day 26. |
| D | 3010 | 1 mg/g BOL-303242-X (0.05 mg/dose) | 29 | Found dead. |

| | | | | |
|---|---|---|---|---|
| ⁽¹⁾ Day euthanized or found dead. | | | | |

Remaining rabbits survived until study completion (Day 33). One surviving rabbit dosed with 10 mg/g BOL-303242-X (0.5 mg/dose) was noted to have diarrhea on Day 18 (Group B, No. 3048).

### Ophthalmic Observations

Slit-lamp ophthalmic observations are presented in Table T-2-3. A key to the ophthalmic observation scores is presented in Table T-2-4. Eyes appeared normal at most observations. Mild conjunctival congestion (score = 1) was seen sporadically, mostly in treated right eyes, with no consistent association with test or control article. The only other findings were a small area of corneal pigmentation in an untreated left eye (Group A, No. 3086), a pinpoint corneal scar in a 10 mg/g BOL-303242-X-dosed right eye (Group B, No. 3083), and a subconjunctival hemorrhage in a 1 mg/g BOL-303242-X-dosed right eye (Group D, No. 3043). The observed corneal lesions might be related to the pneumotonometry procedure.

### Intraocular Pressure Measurements

Descriptive statistics for IOP data are presented in Table T-2-5 (left eyes, a.m.), Table T-2-6 (right eyes, p.m.), Table T-2-7 (left eyes, p.m.) and Table T-2-8 (right eyes, p.m.).

Mean IOP varied throughout the study for all groups; the variations were similar for left and right eyes within each group. For all groups (including the BSS dose group), mean IOP reached a maximum between Days 5 and 10 for both left and right eyes, a.m. and p.m. readings. Diurnal changes in IOP from a.m. to p.m. were not evident during the study, possibly due to daily feeding of rabbits prior to p.m. measurements.

For the dexamethasone group (Group A), mean IOP of both left and right eyes increased sharply after treatment began. This increase was not seen in the mean IOPs of the BOL-303242-X groups (Groups B-D) at any point of the study. On several days, the mean IOP in one or both eyes of the dexamethasone group (Group A) was significantly higher (p < 0.05) than the mean IOP in the corresponding eyes of other groups. This difference was more common in the a.m. than the p.m., and it occurred at more timepoints for the untreated left eyes than the treated right eyes. Mean IOP of BSS-dosed right eyes (Group E) was generally lower than mean IOP of BOL-303242-X-dosed right eyes (Groups B-D) in the a.m. but not in the p.m. No statistically significant (p < 0.05) differences in mean IOP were seen between the BSS group and BOL-303242-X groups.

### CONCLUSIONS

The objective of this study was to evaluate the effect of topical BOL-303242-X on the intraocular pressure (IOP) in New Zealand White rabbits when administered to right eyes four times daily for 33 days. In conclusion, unilateral topical instillation of BOL-303242-X suspension (0.05, 0.25, or 0.5 mg/dose), dexamethasone suspension (0.05 mg/dose), or balanced salt solution in rabbit eyes four times daily up to 31 days was associated with sporadic mild conjunctival congestion. Dosing with dexamethasone up to 31 days was associated with a higher mortality rate (6 deaths per 10 rabbits) than dosing with BOL-303242-X up to 31 days (per dose level, 1-2 deaths per 10 rabbits). Daily dosing with the BOL-303242-X suspensions did not increase IOP when compared to daily dosing with dexamethasone.

**Table T-2-1**

| Treatment Groups | | | | | | |
|---|---|---|---|---|---|---|
| Group | No. | Treatment (4 x Daily) | Dose Location (Right Eye) | Dose Volume | Drug Dose Level | Scheduled Study Completion⁽¹⁾ |
| A | 10 | 0.1% Dexamethasone (Maxidex^{®}) | Topical | 50 µL | 0.05 mg/dose | Day 33 |
| B | 10 | 10 mg/g BOL-303242-X | Topical | 50 µL | 0.5 mg/dose | Day 33 |
| C | 10 | 5 mg/g BOL-303242-X | Topical | 50 µL | 0.25 mg/dose | Day 33 |
| D | 10 | 1 mg/g BOL-303242-X | Topical | 50 µL | 0.05 mg/dose | Day 33 |
| E | 10 | Balanced Salt Solution | Topical | 50 µL | N/A | Day 33 |

| | | | | | | |
|---|---|---|---|---|---|---|
| N/A = Not Applicable. (1) Dosing was performed daily through Day 31. Final ophthalmic examinations were performed on Day 33. | | | | | | |

**Table T-2-2**

| Mortality | | | | | | | |
|---|---|---|---|---|---|---|---|
| Group | No. | Treatment (4 x Daily) | Dose Location (Right Eye) | Dose Volume | Drug Dose Level | Scheduled Study Completion (1) | Mortality⁽²⁾ |
| A | 10 | 0.1% Dexamethasone (Maxidex^{®}) | Topical | 50 µL | 0.05 mg/dose | Day 33 | 6/10⁽³⁾ |
| B | 10 | 10 mg/g BOL-303242-X | Topical | 50 µL | 0.5 mg/dose | Day 33 | 1/10⁽⁴⁾ |
| C | 10 | 5 mg/g BOL-303242-X | Topical | 50 µL | 0.25 mg/dose | Day 33 | 2/10⁽⁵⁾ |
| D | 10 | 1 mg/g BOL-303242-X | Topical | 50 µL | 0.05 mg/dose | Day 33 | 1/10⁽⁶⁾ |
| E | 10 | Balanced Salt Solution | Topical | 50 µL | N/A | Day 33 | 0/10 |

| | | | | | | | |
|---|---|---|---|---|---|---|---|
| N/A = Not Applicable. (1) Dosing was performed daily through Day 31. Final ophthalmic examinations were performed on Day 33. (2) Mortality is expressed as the number of animals found dead or euthanized prior to study completion/number of animals in group. (3) One Group A rabbit was found dead on Day 27. Five Group A rabbits were euthanized between Days 13 and 27 due to severe diarrhea. (4) One Group B rabbit was found dead on Day 11; it was observed to have diarrhea on Day 10. (5) One Group C rabbit was euthanized on Day 17 due to severe diarrhea. The other was euthanized on Day 33 prior to final ophthalmic examinations due to a respiratory infection. (6) One Group D rabbit was found dead on Day 29. | | | | | | | |

**Table T-2-3**

| Ophthalmic Observations (Slit-Lamp) | | | | | | |
|---|---|---|---|---|---|---|
| Group | Animal No. | Treatment (4 x Daily) | Eye | Dav | Ophthalmic Observation⁽¹⁾ | Score |
| A | 3016 | Untreated | Left | 5, 12, 22, 26 | AN | N/A |
| | | 0.1% Dexamethasone | Right | 5 | Conjunctival Congestion | 1 |
| | | | | 12, 22, 26 | AN | N/A |
| A | 3081 | Untreated | Left | 5, 12, 22, 26, 33 | AN | N/A |
| | | 0.1% Dexamethasone | Right | 22 | Conjunctival Congestion | I |
| | | | | 5, 12, 26, 33 | AN | N/A |
| A | 3086 | Untreated | Left | 26 | Cornea | 1⁽²⁾ |
| | | | | 5, 12, 22 | AN | N/A |
| | | 0.1% Dexamethasone | Right | 5, 12, 22, 26 | AN | N/A |
| A | 3037 | Untreated | Left | 5, 12, 22 | AN | N/A |
| | | 0.1% Dexamethasone | Right | 5, 12, 22 | AN | N/A |
| A | 3006 | Untreated | Left | 5, 12, 22, 26, 33 | AN | N/A |
| | | 0.1% Dexamethasone | Right | 5, 12, 22, 26, 33 | AN | N/A |
| A | 3068 | Untreated | Left | 5, 12, 22 | AN | N/A |
| | | 0.1% Dexamethasone | Right | 5, 12, 22 | AN | N/A |
| A | 3033 | Untreated | Left | 5, 12, 22, 26, 33 | AN | N/A |
| | | 0.1% Dexamethasone | Right | 5, 12, 22, 26, 33 | AN | N/A |
| A | 3029 | Untreated | Left | 5, 12, 22, 26, 33 | AN | N/A |
| | | 0.1% Dexamethasone | Right | 5, 12, 22, 26, 33 | AN | N/A |
| A | 3011 | Untreated | Left | 5, 12, 22 | AN | N/A |
| | | 0.1 % Dexamethasone | Right | 5, 12, 22 | AN | N/A |
| A | 3038 | Untreated | Left | 5, 12 | AN | N/A |
| | | 0.1% Dexamethasone | Right | 5, 12 | AN | N/A |
| | | | | | | |

| Group | Animal No. | Topical Treatment | Eye | Day | Ophthalmic Observation⁽¹⁾ | Score |
|---|---|---|---|---|---|---|
| B | 3083 | Untreated | Left | 5, 12, 22, 26, 33 | AN | N/A |
| | | 10 mg/g BOL-303242-X | Right | 5 | Cornea | 1⁽²⁾ |
| | | | | 5 | Surface area of cornea involvement | 1 |
| | | | | 12,22,26,33 | AN | N/A |
| B | 3008 | Untreated | Left | 5 | AN | N/A |
| | | 10 mg/g BOL-303242-X | Right | 5 | AN | N/A |
| B | 3017 | Untreated | Left | 5, 12, 22, 26, 33 | AN | N/A |
| | | 10 mg/g BOL-303242-X | Right | 5, 12 | Conjunctival Congestion | 1 |
| | | | | 22, 26, 33 | AN | N/A |
| B | 3048 | Untreated | Left | 5, 12, 22, 26, 33 | AN | N/A |
| | | 10 mg/g BOL-303242-X | Right | 5, 12, 22, 26, 33 | AN | N/A |
| B | 3003 | Untreated | Left | 5, 12, 22, 26, 33 | AN | N/A |
| | | 10 mg/g BOL-303242-X | Right | 12 | Conjunctival Congestion | 1 |
| | | | | 5, 22, 26, 33 | AN | N/A |
| B | 3042 | Untreated | Left | 5, 12, 22, 26, 33 | AN | N/A |
| | | 10 mg/g BOL-303242-X | Right | 26 | Conjunctival Congestion | I |
| | | | | 5, 12, 22, 33 | AN | N/A |
| B | 3023 | Untreated | Left | 5, 12, 22, 26, 33 | AN | N/A |
| | | 10 mg/g BOL-303242-X | Right | 5, 12, 22, 26, 33 | AN | N/A |
| B | 3004 | Untreated | Left | 5, 12, 22, 26, 33 | AN | N/A |
| | | 10 mg/g BOL-303242-X | Right | 5, 12, 22, 26, 33 | AN | N/A |
| B | 3049 | Untreated | Left | 5, 12,22,26,33 | AN | N/A |
| | | 10 mg/g BOL-303242-X | Right | 5, 12, 22, 26, 33 | AN | N/A |
| B | 3026 | Untreated | Left | 5, 12, 22, 26, 33 | AN | N/A |
| | | 10 mg/g BOL-303242-X | Right | 5, 12, 22, 26, 33 | AN | N/A |
| | | | | | | |
| C | 3028 | Untreated | Left | 5, 12 | AN | N/A |
| | | 5 mg/g BOL-303242-X | Right | 5, 12 | AN | N/A |
| C | 3064 | Untreated | Left | 5, 12, 22, 26, 33 | AN | N/A |
| | | 5 mg/g BOL-303242-X | Right | 5 | Conjunctival congestion | 1 |
| | | | | 12, 22, 26, 33 | AN | N/A |
| C | 3031 | Untreated | Left | 5, 12, 22, 26, 33 | AN | N/A |
| | | 5 mg/g BOL-303242-X | Right | 22 | Conjunctival congestion | 1 |
| | | | | 5, 12, 26, 33 | AN | N/A |
| C | 3032 | Untreated | Left | 5, 12, 22, 26, 33 | AN | N/A |
| | | 5 mg/g BOL-303242-X | Right | 5, 12, 22, 26, 33 | AN | N/A |
| C | 3041 | Untreated | Left | 5, 12, 22, 26, 33 | AN | N/A |
| | | 5 mg/g BOL-303242-X | Right | 5, 12, 22, 26, 33 | AN | N/A |
| C | 3034 | Untreated | Left | 5, 12, 22, 26, 33 | AN | N/A |
| | | 5 mg/g BOL-303242-X | Right | 5, 12, 22, 26, 33 | AN | N/A |
| C | 3035 | Untreated | Left | 5, 12, 22, 26, 33 | AN | N/A |
| | | 5 mg/g BOL-303242-X | Right | 22, 26 | Conjunctival congestion | 1 |
| | | | | 5, 12, 33 | AN | N/A |
| C | 3046 | Untreated | Left | 5, 12, 22, 26, 33 | AN | N/A |
| | | 5 mg/g BOL-303242-X | Right | 5, 12, 22, 26, 33 | AN | N/A |
| C | 3058 | Untreated | Left | 5, 12, 22, 26, 33 | AN | N/A |
| | | 5 mg/g BOL-303242-X | Right | 5, 12, 22, 26, 33 | AN | N/A |
| C | 3074 | Untreated | Left | 5, 12, 22, 26 | AN | N/A |
| | | 5 mg/g BOL-303242-X | Right | 26 | Conjunctival congestion | 1 |
| | | | | 5, 12, 22 | AN | N/A |
| | | | | | | |

| Table T-2-3 (continued) | | | | | | |
|---|---|---|---|---|---|---|
| Ophthalmic Observations (Slit-Lamp) | | | | | | |
| Group | Animal No. | Topical Treatment | Eye | Day | Ophthalmic Observation⁽¹⁾ | Score |
| D | 3010 | Untreated | Left | 5, 12, 22, 26 | AN | N/A |
| | | 1 mg/g BOL-303242-X | Right | 5, 12, 22, 26 | AN | N/A |
| D | 3039 | Untreated | Left | 5, 12, 22, 26, 33 | AN | N/A |
| | | 1 mg/g BOL-303242-X | Right | 5, 12, 22, 26, 33 | AN | N/A |
| D | 3043 | Untreated | Left | 5, 12,22,26,33 | AN⁽²⁾ | N/A |
| | | 1 mg/g BOL-303242-X | Right | 5, 12, 22, 26, 33 | AN | N/A |
| D | 3044 | Untreated | Left | 5, 12, 22, 26, 33 | AN | N/A |
| | | 1 mg/g BOL-303242-X | Right | 5, 12, 22, 26, 33 | AN | N/A |
| D | 3027 | Untreated | Left | 5, 12, 22, 26, 33 | AN | N/A |
| | | 1 mg/g BOL-303242-X | Right | 5, 12, 22, 26, 33 | AN | N/A |
| D | 3072 | Untreated | Left | 5, 12, 22, 26, 33 | AN | N/A |
| | | 1 mg/g BOL-303242-X | Right | 5, 12, 22, 26, 33 | AN | N/A |
| D | 3040 | Untreated | Left | 5, 12, 22, 26, 33 | AN | N/A |
| | | 1 mg/g BOL-303242-X | Right | 22 | Conjunctival congestion | 1 |
| | | | | 5, 12, 26, 33 | AN | N/A |
| D | 3020 | Untreated | Left | 5, 12, 22, 26, 33 | AN | N/A |
| | | 1 mg/g BOL-303242-X | Right | 5, 12, 22, 26, 33 | AN | N/A |
| D | 3063 | Untreated | Left | 5, 12, 22, 26, 33 | AN | N/A |
| | | 1 mg/g BOL-303242-X | Right | 5, 12, 22, 26, 33 | AN | N/A |
| D | 3077 | Untreated | Left | 5, 12, 22, 26, 33 | AN | N/A |
| | | 1 mg/g BOL-303242-X | Right | 5, 12, 22, 26, 33 | AN | N/A |
| | | | | | | |

| Ophthalmic Observations (Slit-Lamp) | | | | | | |
|---|---|---|---|---|---|---|
| Group | Animal No. | Topical Treatment | Eye | Day | Ophthalmic Observation⁽¹⁾ | Score |
| E | 3002 | Untreated | Left | 5, 12, 22, 26, 33 | AN | N/A |
| | | Balanced Salt Solution | Right | 5, 12, 22, 26, 33 | AN | N/A |
| E | 3084 | Untreated | Left | 5, 12, 22, 26, 33 | AN | N/A |
| | | Balanced Salt Solution | Right | 5, 12, 22, 26, 33 | AN | N/A |
| E | 3057 | Untreated | Left | 5, 12, 22, 26, 33 | AN | N/A |
| | | Balanced Salt Solution | Right | 12, 22, 26 | Conjunctival Congestion | 1 |
| | | | | 5, 33 | AN | N/A |
| E | 3087 | Untreated | Left | 5, 12, 22, 26, 33 | AN | N/A |
| | | Balanced Salt Solution | Right | 5, 12, 22, 26, 33 | AN | N/A |
| E | 3018 | Untreated | Left | 5, 12, 22, 26, 33 | AN | N/A |
| | | Balanced Salt Solution | Right | 26 | Conjunctival Congestion | 1 |
| | | | | 5, 12, 22, 33 | AN | N/A |
| E | 3090 | Untreated | Left | 5, 12, 22, 26, 33 | AN | N/A |
| | | Balanced Salt Solution | Right | 5, 12, 22, 26, 33 | AN | N/A |
| E | 3047 | Untreated | Left | 5, 12, 22, 26, 33 | AN | N/A |
| | | Balanced Salt Solution | Right | 5, 12, 22, 26, 33 | AN | N/A |
| E | 3070 | Untreated | Left | 26 | Conjunctival Congestion | 1 |
| | | | | 5, 12, 22, 33 | AN | N/A |
| | | Balanced Salt Solution | Right | 5, 12, 22, 26, 33 | AN | N/A |
| E | 3019 | Untreated | Left | 5, 12, 22, 26, 33 | AN | N/A |
| | | Balanced Salt Solution | Right | 5, 12, 22, 26, 33 | AN | N/A |
| E | 3007 | Untreated | Left | 5, 12, 22, 26, 33 | AN | N/A |
| | | Balanced Salt Solution | Right | 5, 12, 22, 26, 33 | AN | N/A |
| | | | | | | |

| | | | | | | |
|---|---|---|---|---|---|---|
| AN = Appeared normal. N/A = Not Applicable. See Table T-2-4 for key to ophthalmic observation scores. (1) Observations were made prior to the first dose of the day. (2) Day 12: Subconjunctival hemorrhage observed. | | | | | | |

**Table T-2-4**

| Key to Ophthalmic Observation Scoring System⁽¹⁾ | |
|---|---|
| CONJUNCTIVAL CONGESTION | |
| 1 = | A flushed, reddish color predominantly confined to the palpebral conjunctiva with some perilimbal injection but primarily confined to the lower and upper parts of the eye from the 4:00 to 7:00 and 11:00 to 1:00 positions. |

| CORNEA | |
|---|---|
| 1 = | Some loss of transparency. Only the epithelium and/or the anterior half of the stoma are involved. The underlying structures are clearly visible although some cloudiness may be readily apparent. |

| SURFACE AREA OF CORNEA INVOLVEMENT | |
|---|---|
| 1 = | 1-25% area of stromal cloudiness. |

| | |
|---|---|
| Note (1): *Dermatoxicology*, F.N. Mazulli and H.I. Maibach, 1997, "Eye Irritation," T.O. McDonald and J.A. Shaduck (pages 579-582). | |

**Table T-2-5**

| Descriptive Statistics for Intraocular Pressure in Untreated Left Eyes (A.M. Readings) | | | | | | |
|---|---|---|---|---|---|---|
| Intraocular Pressure (mmHg) | | | | | | |
| Day | Statistic | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study | MEAN | 24.4 | 23.8 | 24.2 | 23.9 | 23.4 |
| (5/9/07) | SEM | 0.7 | 0.6 | 0.4 | 0.4 | 0.5 |
| | STD | 2.1 | 1.8 | 1.2 | 1.3 | 1.5 |
| | N | 10 | 10 | 10 | 10 | 10 |
| 3 | MEAN | 24.3 | 23.3 | 23.8 | 23.5 | 22.7 |
| | SEM | 0.5 | 0.4 | 0.4 | 0.6 | 0.4 |
| | STD | 1.2 | 1.2 | 1.1 | 1.8 | 1.3 |
| | N | 6 | 10 | 9 | 10 | 9 |
| 5 | MEAN | 24.3 | 23.4 | 24.4 | 24.4 | 24.1 |
| | SEM | 0.8 | 0.6 | 0.6 | 0.5 | 0.4 |
| | STD | 2.0 | 1.9 | 1.7 | 1.5 | 1.3 |
| | N | 6 | 10 | 9 | 10 | 9 |
| 10 | MEAN | 26.9^{a} | 24.0^{a} | 24.6 | 24.5 | 25.4 |
| | SEM | 0.5 | 0.8 | 0.6 | 0.4 | 0.7 |
| | STD | 1.2 | 2.4 | 1.9 | 1.2 | 2.1 |
| | N | 6 | 10 | 9 | 10 | 9 |
| 12 | MEAN | 26.2^{a} | 23.8 | 23.8 | 22.2^{a} | 23.7 |
| | SEM | 0.6 | 0.7 | 0.7 | 0.7 | 0.7 |
| | STD | 1.5 | 2.0 | 2.2 | 2.3 | 2.0 |
| | | N 6 | 9 | 9 | 10 | 9 |
| 16 | MEAN | 25.0^{a,b} | 22.9 | 23.4 | 21.6^{a} | 20.3^{b} |
| | SEM | 1.0 | 0.7 | 0.6 | 1.1 | 0.6 |
| | STD | 2.2 | 2.1 | 1.7 | 3.4 | 1.9 |
| | | N 5 | 9 | 9 | 10 | 9 |

| | | Intraocular Pressure (mmHg) | | | | |
|---|---|---|---|---|---|---|
| Day | Statistic | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| 18 | MEAN | 24.2^{a} | 21.2^{a} | 21.9 | 23.3 | 22.3 |
| | SEM | 0.4 | 0.5 | 0.6 | 0.4 | 0.6 |
| | STD | 1.0 | 1.6 | 1.7 | 1.4 | 1.9 |
| | N | 5 | 9 | 8 | 10 | 9 |
| 22 | MEAN | 25.0^{a,b,c} | 21.8^{a} | 21.6^{b} | 22.4 | 22.0^{c} |
| | SEM | 0.5 | 0.6 | 1.1 | 0.3 | 0.5 |
| | STD | 1.2 | 1.8 | 3.0 | 1.0 | 1.6 |
| | N | 5 | 9 | 8 | 10 | 9 |
| 24 | MEAN | 23.6^{a} | 20.2^{a} | 22.1 | 22.4 | 20.8 |
| | SEM | 0.9 | 0.6 | 0.6 | 0.8 | 0.7 |
| | STD | 2.1 | 1.8 | 1.7 | 2.5 | 2.1 |
| | N | 5 | 9 | 8 | 10 | 9 |
| 26 | MEAN | 23.7 | 21.7 | 21.7 | 22.9 | 20.5 |
| | SEM | 1.0 | 0.7 | 1.1 | 0.6 | 0.6 |
| | STD | 2.2 | 2.0 | 3.0 | 2.0 | 1.7 |
| | N | 5 | 9 | 8 | 10 | 9 |
| 30 | MEAN | 24.0 | 22.7 | 22.6 | 23.4 | 22.7 |
| | SEM | 1.0 | 0.6 | 1.2 | 0.8 | 0.5 |
| | STD | 1.7 | 1.7 | 3.4 | 2.4 | 1.5 |
| | N | 3 | 9 | 8 | 9 | 9 |
| 32 | MEAN | 25.5 | 22.9 | 23.1 | 24.1 | 22.3 |
| | SEM | 0.8 | 0.5 | 0.7 | 0.6 | 0.5 |
| | STD | 1.3 | 1.6 | 2.1 | 1.8 | 1.5 |
| | N | 3 | 9 | 8 | 9 | 9 |

| Descriptive Statistics for Intraocular Pressure in Treated Right Eyes (A.M. Readings) | | | | | | |
|---|---|---|---|---|---|---|
| | | Intraocular Pressure (mmHg) | | | | |
| Day | Statistic | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study | MEAN | 24.1 | 24.0 | 24.8 | 24.4 | 24.1 |
| (5/9/07) | SEM | 0.7 | 0.5 | 0.5 | 0.6 | 0.5 |
| | STD | 2.2 | 1.7 | 1.6 | 1.9 | 1.6 |
| | N | 10 | 10 | 10 | 10 | 10 |
| 3 | MEAN | 24.3 | 22.7 | 23.7 | 23.0 | 22.1 |
| | SEM | 0.8 | 0.5 | 0.4 | 0.6 | 0.4 |
| | STD | 2.0 | 1.5 | 1.3 | 2.0 | 1.3 |
| | N | 6 | 10 | 9 | 10 | 9 |
| 5 | MEAN | 24.7 | 23.8 | 24.7 | 24.7 | 24.0 |
| | SEM | 0.8 | 0.7 | 0.7 | 0.5 | 0.5 |
| | STD | 1.9 | 2.3 | 2.1 | 1.5 | 1.5 |
| | N | 6 | 10 | 9 | 10 | 9 |
| 10 | MEAN | 26.9 | 24.5 | 25.2 | 24.8 | 25.3 |
| | SEM | 0.3 | 0.6 | 0.6 | 0.5 | 0.6 |
| | STD | 0.7 | 2.0 | 1.7 | 1.4 | 1.8 |
| | | N 6 | 10 | 9 | 10 | 9 |
| 12 | MEAN | 26.7 | 23.9 | 25.0 | 23.4 | 23.2 |
| | SEM | 0.8 | 1.1 | 0.8 | 0.8 | 0.5 |
| | STD | 1.9 | 3.4 | 2.3 | 2.6 | 1.6 |
| | N | 6 | 9 | 9 | 10 | 9 |
| 16 | MEAN | 25.8^{a,b} | 23.4 | 24.3 | 22.1^{a} | 20.7^{b} |
| | SEM | 1.4 | 0.7 | 0.6 | 1.0 | 0.9 |
| | STD | 3.2 | 2.1 | 1.7 | 3.0 | 2.8 |
| | N | 5 | 9 | 9 | 10 | 9 |
| 18 | MEAN | 24.1 | 22.3 | 23.9 | 23.7 | 21.9 |
| | SEM | 0.7 | 0.8 | 0.7 | 0.5 | 0.8 |
| | STD | 1.6 | 2.3 | 1.9 | 1.7 | 2.4 |
| | N | 5 | 9 | 8 | 10 | 9 |
| 22 | MEAN | 25.4^{a,b,c} | 22.4^{a} | 22.4^{b} | 23.2 | 21.4^{c} |
| | SEM | 0.4 | 0.6 | 0.7 | 0.4 | 0.6 |
| | STD | 0.8 | 1.9 | 1.9 | 1.4 | 1.8 |
| | N | 5 | 9 | 8 | 10 | 9 |
| 24 | MEAN | 24.3^{a} | 21.2 | 23.8 | 22.1 | 21.1^{a} |
| | SEM | 0.8 | 0.7 | 0.6 | 0.7 | 0.9 |
| | STD | 1.8 | 2.2 | 1.7 | 2.2 | 2.6 |
| | N | 5 | 9 | 8 | 10 | 9 |
| 26 | MEAN | 23.1 | 21.8 | 22.1 | 23.1 | 20.4 |
| | SEM | 0.9 | 1.0 | 1.3 | 0.8 | 0.5 |
| | STD | 1.9 | 3.0 | 3.7 | 2.4 | 1.4 |
| | N | 5 | 9 | 8 | 10 | 9 |
| 30 | MEAN | 23.5 | 22.7 | 22.9 | 24.2 | 22.1 |
| | SEM | 1.0 | 0.6 | 1.3 | 0.8 | 0.5 |
| | STD | 1.8 | 1.8 | 3.5 | 2.4 | 1.4 |
| | N | 3 | 9 | 8 | 9 | 9 |
| 32 | MEAN | 25.5 | 23.9 | 23.4 | 24.9 | 23.1 |
| | SEM | 0.6 | 0.4 | 0.9 | 0.6 | 0.5 |
| | STD | 1.0 | 1.2 | 2.5 | 1.9 | 1.4 |
| | N | 3 | 9 | 8 | 9 | 9 |

| | | | | | | |
|---|---|---|---|---|---|---|
| NOTE: Differences between means with a same superscript (a, b, or c) in the same row are statistically significant (p < 0.05). | | | | | | |

**Table T-2-7**

| Descriptive Statistics for Intraocular Pressure in Untreated Left Eyes (P.M. Readings) | | | | | | |
|---|---|---|---|---|---|---|
| | | Intraocular Pressure (mmHg) | | | | |
| Day | Statistic | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study | MEAN | 24.2 | 23.9 | 24.4 | 24.2 | 24.2 |
| (5/9/07) | SEM | 0.5 | 0.4 | 0.3 | 0.5 | 0.4 |
| | STD | 1.5 | 1.1 | 1.1 | 1.7 | 1.3 |
| | N | 10 | 10 | 10 | 10 | 10 |
| 3 | MEAN | 24.3 | 23.3 | 23.9 | 25.0 | 23.5 |
| | SEM | 0.7 | 0.4 | 0.5 | 0.4 | 0.4 |
| | STD | 1.7 | 1.2 | 1.4 | 1.3 | 1.2 |
| | N | 6 | 10 | 9 | 10 | 9 |
| 5 | MEAN | 25.6 | 25.2 | 24.8 | 24.7 | 25.1 |
| | SEM | 0.6 | 0.6 | 0.7 | 0.4 | 0.4 |
| | STD | 1.4 | 2.0 | 2.0 | 1.3 | 1.2 |
| | N | 6 | 10 | 9 | 10 | 9 |
| 10 | MEAN | 26.6 | 23.5 | 24.6 | 24.9 | 24.9 |
| | SEM | 0.6 | 1.5 | 0.4 | 0.5 | 0.4 |
| | STD | 1.4 | 4.9 | 1.1 | 1.6 | 1.3 |
| | N | 6 | 10 | 9 | 10 | 9 |
| 12 | MEAN | 22.8 | 24.1 | 23.3 | 23.7 | 24.4 |
| | SEM | 0.9 | 0.9 | 0.5 | 0.4 | 0.7 |
| | STD | 2.2 | 2.8 | 1.5 | 1.4 | 2.0 |
| | | N 6 | 9 | 9 | 10 | 9 |
| 16 | MEAN | 22.6 | 21.4 | 20.4 | 21.9 | 21.3 |
| | SEM | 0.6 | 0.4 | 0.6 | 0.4 | 0.5 |
| | STD | 1.4 | 1.2 | 1.8 | 1.3 | 1.5 |
| | N | 5 | 9 | 9 | 10 | 9 |
| 18 | MEAN | 23.6 | 22.1 | 21.9 | 22.7 | 22.0 |
| | SEM | 0.7 | 0.6 | 0.8 | 0.4 | 0.5 |
| | STD | 1.6 | 1.9 | 2.2 | 1.3 | 1.5 |
| | N | 5 | 9 | 8 | 10 | 9 |
| 22 | MEAN | 23.6 | 22.6 | 22.1 | 22.1 | 21.1 |
| | SEM | 0.4 | 0.5 | 0.8 | 0.7 | 0.8 |
| | STD | 1.0 | 1.5 | 2.2 | 2.1 | 2.4 |
| | N | 5 | 9 | 8 | 10 | 9 |
| 24 | MEAN | 25.3^{a,b} | 22.8 | 22.2^{a} | 22.9 | 22.1^{b} |
| | SEM | 0.7 | 0.8 | 0.8 | 0.5 | 0.4 |
| | STD | 1.5 | 2.3 | 2.4 | 1.6 | 1.2 |
| | N | 5 | 9 | 8 | 10 | 9 |
| 26 | MEAN | 21.9 | 21.4 | 22.3 | 22.1 | 20.9 |
| | SEM | 1.2 | 0.9 | 1.1 | 1.0 | 0.7 |
| | STD | 2.7 | 2.6 | 3.2 | 3.2 | 2.0 |
| | | N 5 | 9 | 8 | 10 | 9 |
| 30 | MEAN | 23.3 | 21.7 | 20.9 | 21.3 | 22.9 |
| | SEM | 1.1 | 0.8 | 1.1 | 0.4 | 0.7 |
| | STD | 1.9 | 2.4 | 3.0 | 1.1 | 2.0 |
| | N | 3 | 9 | 8 | 9 | 9 |
| 32 | MEAN | 25.2 | 22.6 | 21.5 | 21.9 | 22.2 |
| | SEM | 0.3 | 1.2 | 1.3 | 0.3 | 0.6 |
| | STD | 0.6 | 3.5 | 3.5 | 1.0 | 1.7 |
| | N | 3 | 9 | 8 | 9 | 9 |

| | | | | | | |
|---|---|---|---|---|---|---|
| NOTE: Differences between means with a same superscript (a or b) in the same row are statistically significant (p < 0.05). | | | | | | |

**Table T-2-8**

| Descriptive Statistics for Intraocular Pressure in Treated Right Eyes (P.M. Readings) | | | | | | |
|---|---|---|---|---|---|---|
| | | Intraocular Pressure (mmHg) | | | | |
| Day | Statistic | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study | MEAN | 23.4 | 24.0 | 24.5 | 24.2 | 24.2 |
| (5/9/07) | SEM | 0.6 | 0.4 | 0.3 | 0.5 | 0.5 |
| | STD | 1.8 | 1.2 | 0.9 | 1.7 | 1.6 |
| | N | 10 | 10 | 10 | 10 | 10 |
| 3 | MEAN | 24.1 | 23.1 | 23.6 | 24.7 | 23.2 |
| | SEM | 0.6 | 0.3 | 0.5 | 0.4 | 0.6 |
| | STD | 1.4 | 0.8 | 1.6 | 1.2 | 1.7 |
| | N | 6 | 10 | 9 | 10 | 9 |
| 5 | MEAN | 26.3 | 25.7 | 24.8 | 25.5 | 25.6 |
| | SEM | 0.5 | 0.5 | 0.6 | 0.5 | 0.6 |
| | STD | 1.2 | 1.7 | 1.9 | 1.6 | 1.8 |
| | N | 6 | 10 | 9 | 10 | 9 |
| 10 | MEAN | 26.8 | 24.3 | 25.6 | 25.3 | 24.9 |
| | SEM | 0.4 | 1.5 | 0.5 | 0.6 | 0.6 |
| | STD | 1.0 | 4.6 | 1.6 | 2.0 | 1.7 |
| | N | 6 | 10 | 9 | 10 | 9 |
| 12 | MEAN | 23.4 | 23.8 | 23.4 | 24.0 | 25.3 |
| | SEM | 0.5 | 0.8 | 0.6 | 0.5 | 0.5 |
| | STD | 1.3 | 2.5 | 1.7 | 1.5 | 1.4 |
| | N | 6 | 9 | 9 | 10 | 9 |
| 16 | MEAN | 21.5 | 21.6 | 21.4 | 22.0 | 21.3 |
| | SEM | 0.9 | 0.6 | 0.7 | 0.5 | 0.4 |
| | STD | 2.1 | 1.9 | 2.1 | 1.6 | 1.1 |
| | N | 5 | 9 | 9 | 10 | 9 |
| 18 | MEAN | 23.6 | 22.5 | 21.6 | 23.1 | 21.9 |
| | SEM | 0.8 | 0.9 | 0.9 | 0.3 | 0.5 |
| | STD | 1.8 | 2.6 | 2.6 | 0.9 | 1.5 |
| | N | 5 | 9 | 8 | 10 | 9 |
| 22 | MEAN | 23.1 | 23.1 | 22.8 | 22.5 | 21.2 |
| | SEM | 1.4 | 0.5 | 1.1 | 0.4 | 0.8 |
| | STD | 3.2 | 1.6 | 3.0 | 1.4 | 2.3 |
| | N | 5 | 9 | 8 | 10 | 9 |
| 24 | MEAN | 25.4 | 22.8 | 23.4 | 23.6 | 22.8 |
| | SEM | 0.3 | 0.8 | 0.9 | 0.6 | 0.6 |
| | STD | 0.7 | 2.5 | 2.5 | 2.0 | 1.8 |
| | N | 5 | 9 | 8 | 10 | 9 |
| 26 | MEAN | 21.2 | 20.9 | 22.2 | 22.6 | 20.8 |
| | SEM | 1.1 | 0.9 | 1.3 | 0.7 | 0.5 |
| | STD | 2.6 | 2.6 | 3.8 | 2.1 | 1.5 |
| | N | 5 | 9 | 8 | 10 | 9 |
| 30 | MEAN | 22.3 | 22.4 | 22.4 | 21.8 | 23.5 |
| | SEM | 1.1 | 1.1 | 1.0 | 0.3 | 0.5 |
| | STD | 1.9 | 3.3 | 2.7 | 1.0 | 1.5 |
| | N | 3 | 9 | 8 | 9 | 9 |
| 32 | MEAN | 24.2 | 23.3 | 22.7 | 22.9 | 22.5 |
| | SEM | 1.4 | 1.1 | 1.2 | 0.5 | 0.6 |
| | STD | 2.4 | 3.4 | 3.4 | 1.5 | 1.8 |
| | N | 3 | 9 | 8 | 9 | 9 |

### TESTING 3: Effect of BOL-303242-X on Inhibiting TGF-β Stimulated Cellular Levels of Collagen type I, α-Smooth Muscle Actin, and Tissue Transgltaminase in Human Tenon's Fibroblasts

Purpose: This study was conducted to determine the effect of BOL-303242-X on proteins (collagen type I, α-smooth muscle actin ("α-SMA"), and tissue transgltaminase) that are produced by human Tenon's fibroblasts and that are believed to be involved in scarring during post-surgical wound healing.

Methods: Human Tenon's fibroblasts were seeded in 60 mm plates in 10% FBS MEM (fetal bovine serum/Eagle's minimum essential medium) and grown until approximately 90% confluency. After the cells were starved in 0.5% FBS MEM for approximately 72 hours, the media was changed to fresh 0.5% FBS MEM. Cells were then treated with 0.1, 1,3, 5 or 10 µM BOL-303242-X after which TGF-β (5 ng/ml) was added. Cells were harvested 1, 3 or 5 days later. They were washed twice with cold 1X PBS and scraped into I x SDS sample buffer. Lysates were assayed for protein content using the DC protein assay kit from Bio-Rad. Equal amounts of protein from each sample were electrophoresed on a 7.5%Tris-glycine SDS gel. Proteins were transferred to a PVDF membrane, blocked and probed with one or more of the following antibodies: mouse monoclonal anti-α-amooth muscle actin Clone 1A4: (Sigma Aldrich, #A2547), mouse monoclonal anti-human GAPDH (6C5) (Santa Cruz Biotechnology, Inc., #sc-32233), goat anti-human collagen α1Type I (L-19) (Santa Cruz Biotechnology, Inc., #sc-8783), or mouse monoclonal anti-transglutaminase II Ab-3 (ClonesCUB7402+TG100) (NeoMarkers, # MS-300-P). Density of each band was measured densitometrically and the ratio to GADPH calculated.

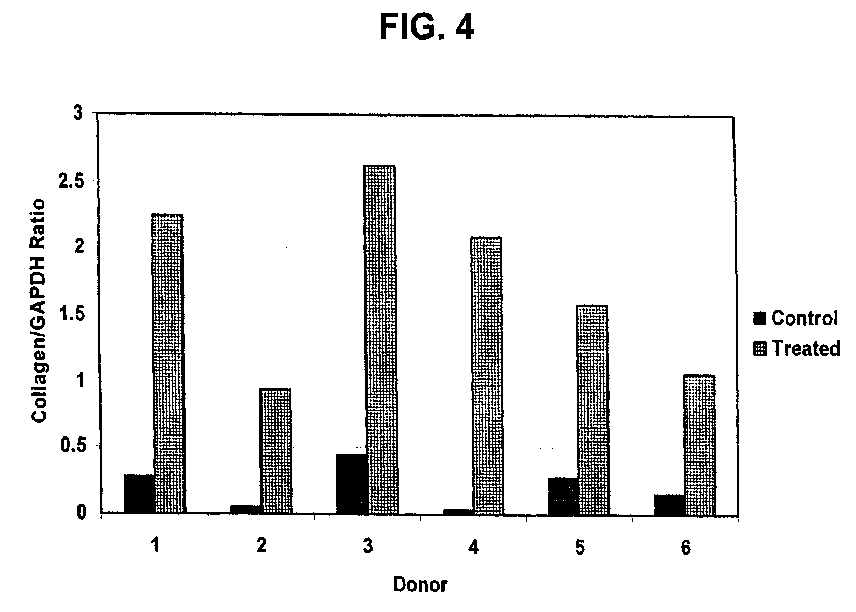

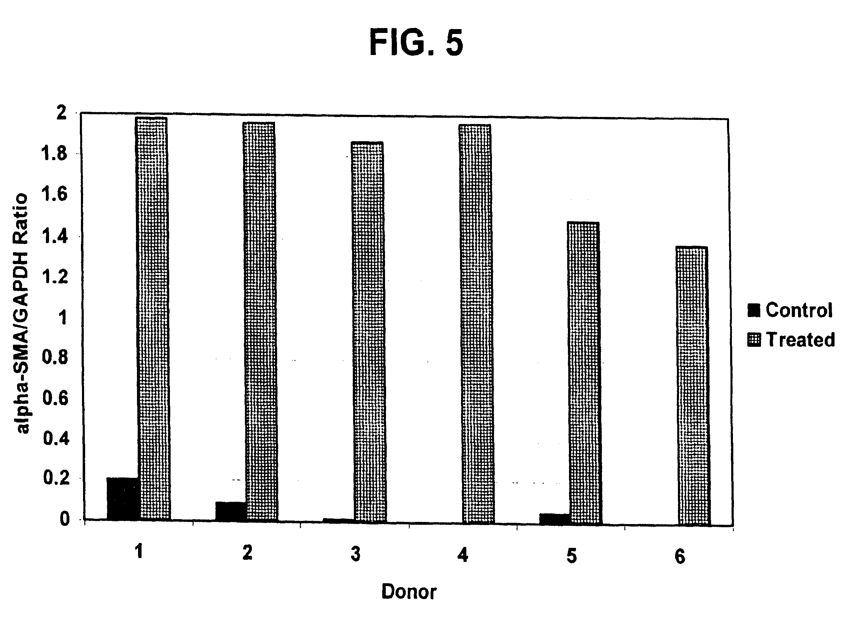

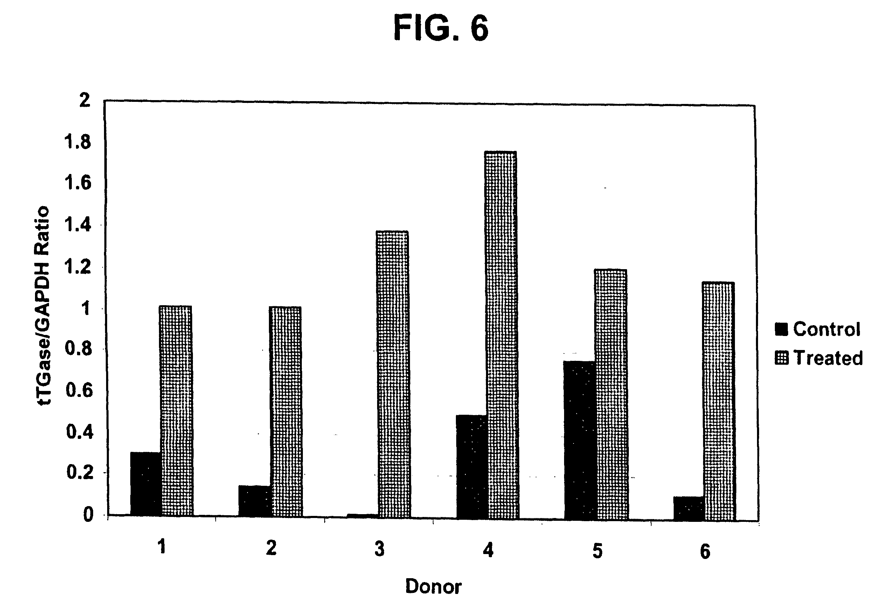

Results and Discussion: Protein levels of collagen type I (Fig. 4), α-smooth muscle actin (Fig. 5), and tissue transglutaminase (Fig. 6) were found to increase over the five-day period following addition of TGF-β compared to cells incubated without TGF-β. An inhibitory effect that appeared dose-related was observed when levels of protein at day 5 from cells treated with TGF-β were compared to levels from TGF-β treated cells incubated with increasing concentrations of BOL-303242-X (Fig. 4-6). Collagen deposition is central to wound healing and tissue remodeling. It is also known that α-SMA induces contraction of collagen gel, and thus actively participates in extracellular matrix remodeling. In addition, transglutaminase aids the irreversible cross-linking of extracellular matrix proteins, such as fibronectinand fibrin, leading to increased resistance to the outflow of the aqueous humor. Therefore, reducing the levels of collagen, α-SMA, and transglutaminase can have a positive effect on the long-term functioning of the filtering bleb.

Conclusion: BOL-303242-X was found to inhibit TGF-β-induced increases in cellular levels of collagen type I, α-SMA, and tissue transglutaminase. Thus, administration of BOL-303242-X or a DIGRA to a patient can have a positive effect on preserving the long-term functioning of the filtering bleb after GFS.

## Claims

1. A composition comprising a dissociated glucocorticoid receptor agonist ("DIGRA"), a pharmaceutically acceptable salt thereof, or a pharmaceutically acceptable ester thereof in an amount effective to reduce a risk of development of a non-functioning, poorly functioning, or failing filtering bleb created in glaucoma filtration surgery in a subject, wherein the DIGRA comprises a compound having Formula II or III 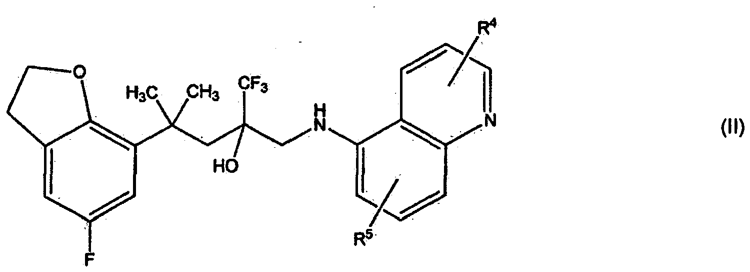 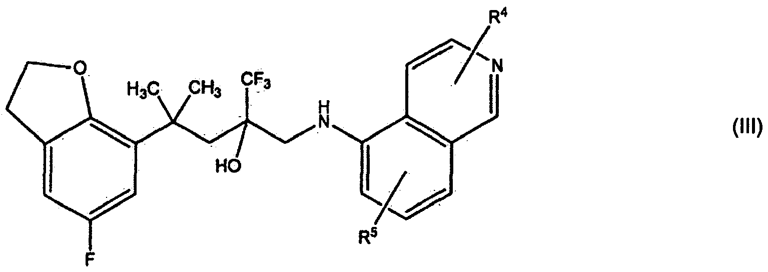 wherein R⁴ and R⁵ are independently selected from the group consisting of hydrogen, halogen, cyano, hydroxy, C₁-C₁₀ alkoxy groups, unsubstituted C₁-C₁₀ linear or branched alkyl groups, substituted C₁-C₁₀ linear or branched alkyl groups, unsubstituted C₃-C₁₀ cyclic alkyl groups, and substituted C₃-C₁₀ cyclic alkyl groups; and wherein said composition further comprises an antagonist to at least a TGF-β isoform, wherein said antagonist is selected from the group consisting of TGF-β antibodies, SB-431542, AP 12009, prolactin, sTGFRI and sTGFRII.

2. The composition according to claim 1, wherein said composition further comprises an NSAID.

3. The composition according to claim 1, wherein said glaucoma is selected from the group consisting of primary open-angle glaucoma, primary angle-closure glaucoma, secondary open-angle glaucoma, secondary angle-closure glaucoma, pigmentary glaucoma, neovascular glaucoma, pseudophakic glaucoma, malignant glaucoma, uveitic glaucoma, glaucoma due to peripheral anterior synechia, and combinations thereof.

4. The composition according to claim 1, wherein said DIGRA has Formula IV

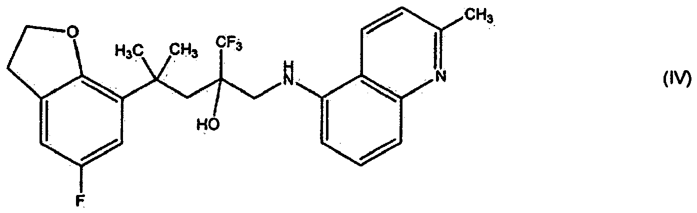

5. A DIGRA, a pharmaceutically acceptable salt thereof, or a pharmaceutically acceptable ester thereof, for use in a medicament for the pharmacological adjunctive treatment associated with glaucoma filtration surgery in a subject, wherein said DIGRA, a pharmaceutically acceptable salt thereof, or a
pharmaceutically acceptable ester thereof has the property of reducing or suppressing production of a TGF-β, and wherein said pharmacological adjunctive treatment results in a continued functioning of a filtering bleb created in said subject in said glaucoma filtering surgery and wherein the DIGRA comprises a compound having Formula II or III 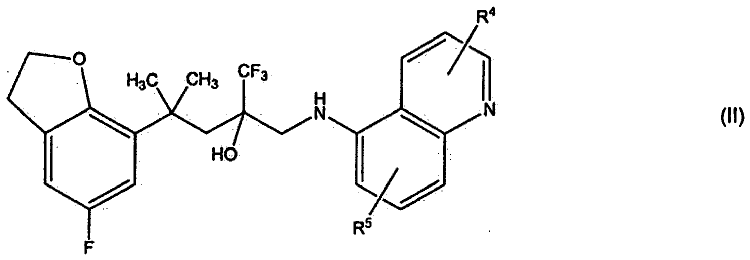 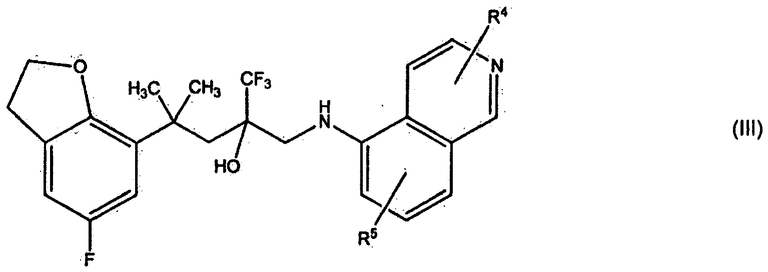 wherein R⁴ and R⁵ are independently selected from the group consisting of hydrogen, halogen, cyano, hydroxy, C₁-C₁₀ alkoxy groups, unsubstituted C₁-C₁₀ linear or branched alkyl groups, substituted C₁-C₁₀ linear or branched alkyl groups, unsubstituted C₃-C₁₀ cyclic alkyl groups, and substituted C₃-C₁₀ cyclic alkyl groups.

6. The DIGRA for use according to claim 5, wherein said production of said TGF-β is stimulated by a chemokine or cytokine selected from the group consisting of IL-1β, IL-4, IL-6, IL-9, IL-11, IL-13, IL-17, TNF-α, TGF-α, MCP-1, GM-CSF, PDGF, β-FGF, CTGF, and combinations thereof.

7. The DIGRA for use according to claim 5, wherein the DIGRA has Formula IV

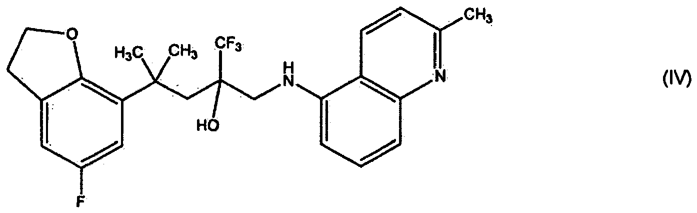

8. The DIGRA for use according to claim 5, wherein the medicament further comprises an antagonist to TGF-β.

9. The DIGRA for use according to claim 8, wherein the medicament further comprises an NSAID.

10. The DIGRA for use according to claim 5, wherein the medicament further comprises an effective amount of another therapeutic or prophylactic agent that is capable of treating, reducing, or preventing (a) an increase in intraocular pressure, (b) loss of retinal ganglion cells, or (c) both; wherein said another therapeutic or prophylactic agent is selected from the group consisting of physostigmine salicylate, pilocarpine nitrate, carbonic anhydrase inhibitors, prostaglandin analogs, β-adrenergic antagonists, muscarinic cholinergic agents, and inhibitors of acetylcholinesterase.

11. A DIGRA or a pharmaceutically acceptable salt or ester thereof, for use in the pharmacological adjunctive treatment associated with glaucoma filtration surgery in a subject, wherein said treatment provides a continued functioning filtering bleb in said subject after said filtration surgery, and wherein said DIGRA has Formula IV

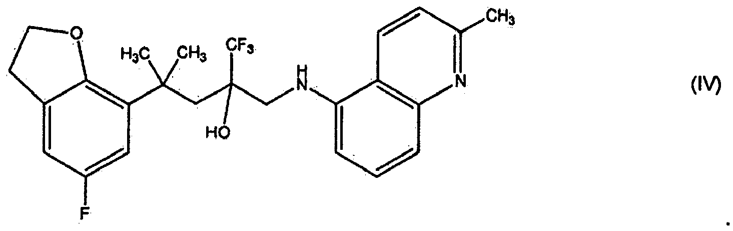

12. A DIGRA or a pharmaceutically acceptable salt or ester thereof, for use in a medicament for controlling or preventing progression of glaucoma in a subject, wherein said DIGRA has Formula IV 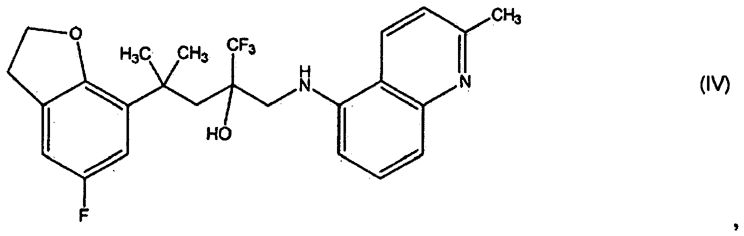 and wherein said medicament is administered in an eye of said subject, which eye underwent a glaucoma filtration surgery, and wherein said controlling or preventing said progression results from a continued functioning filtering bleb.

13. A method for manufacturing a composition, the method comprising:
(a) providing a DIGRA, a pharmaceutically acceptable salt thereof, or a pharmaceutically acceptable ester thereof;
(b) providing an antagonist to TGF-β; and
combining: (i) said DIGRA, pharmaceutically acceptable salt thereof, or pharmaceutically acceptable ester thereof; (ii) said antagonist to TGF-β; and (iii) a pharmaceutically acceptable carrier to produce said composition, wherein the DIGRA comprises a compound having Formula II or III 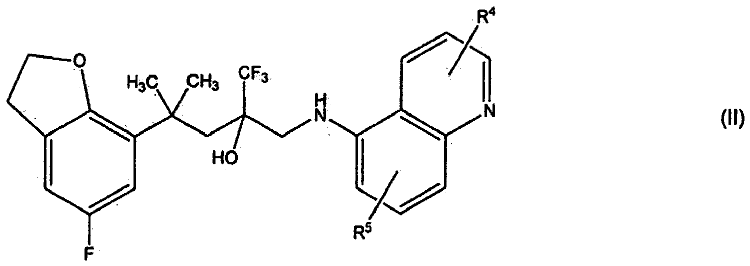 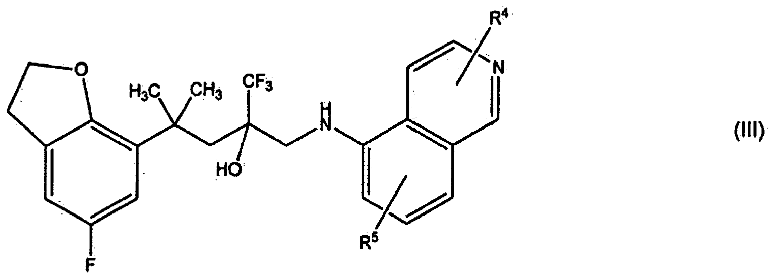 wherein R⁴ and R⁵ are independently selected from the group consisting of hydrogen, halogen, cyano, hydroxy, C₁-C₁₀ alkoxy groups, unsubstituted C₁-C₁₀ linear or branched alkyl groups, substituted C₁-C₁₀ linear or branched alkyl groups, unsubstituted C₃-C₁₀ cyclic alkyl groups, and substituted C₃-C₁₀ cyclic alkyl groups.

## Patentansprüche

1. Eine Zusammensetzung umfassend einen dissoziierten Glucocorticoid-Rezeptor-Agonisten ("DIGRA"), ein pharmazeutisch zulässiges Salz davon oder einen pharmazeutisch zulässigen Ester davon, in einer Menge, die wirksam ist, das Risiko der Entwicklung eines nicht-funktionierenden, schlecht funktionierenden oder fehlerhaften Sickerkissens, welches während einer Glaukom-Filtrationsoperation in einem Subjekt geschaffen wird, zu vermindern, wobei das DIGRA eine Verbindung mit der Formel II oder III umfasst 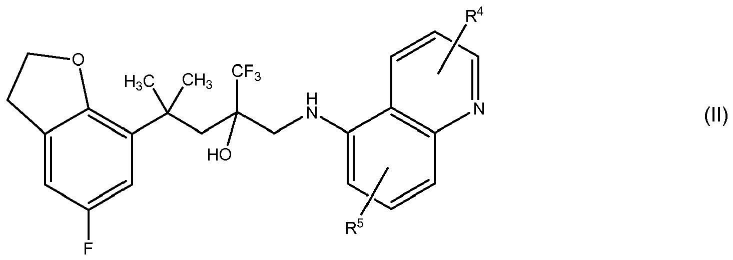 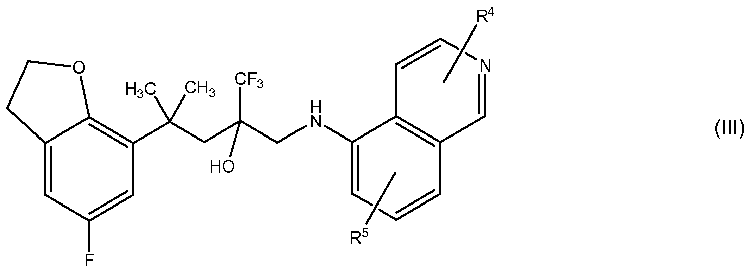 wobei R⁴ und R⁵ jeweils unabhängig voneinander ausgewählt sind aus der Gruppe bestehend aus Wasserstoff, Halogen, Cyano, Hydroxy, C₁-C₁₀ Alkoxy-Gruppen, unsubstituierten C₁-C₁₀ linearen oder verzweigten Alkyl-Gruppen, substituierten C₁-C₁₀ linearen oder verzweigten Alkyl-Gruppen, unsubstituierten C₃-C₁₀ zyklischen Alkyl-Gruppen und substituierten C₃-C₁₀ zyklischen Alkyl-Gruppen und wobei besagte Zusammensetzung weiterhin einen Antagonisten zu mindestens einer TGF-β Isoform umfasst, wobei besagter Antagonist ausgewählt ist aus der Gruppe bestehend aus TGF-β Antikörpern, SB-431542, AP 12009, Prolactin, sTGFRI und sTGFRII.

2. Die Zusammensetzung gemäß Anspruch 1, wobei besagte Zusammensetzung weiterhin einen NSAID umfasst.

3. Die Zusammensetzung gemäß Anspruch 1, wobei besagtes Glaukom ausgewählt ist aus der Gruppe bestehend aus primärem Offenwinkelglaukom, primärem Engwinkelglaukom, sekundärem Offenwinkelglaukom, sekundärem Engwinkelglaukom, Pigmentglaukom, neovaskulärem Glaukom, pseudophaken Glaukom, malignem Glaukom, uveitischem Glaukom, Glaukom aufgrund peripherer vorderer Synechie und Kombinationen davon.

4. Die Zusammensetzung gemäß Anspruch 1, wobei besagtes DIGRA die Formel IV 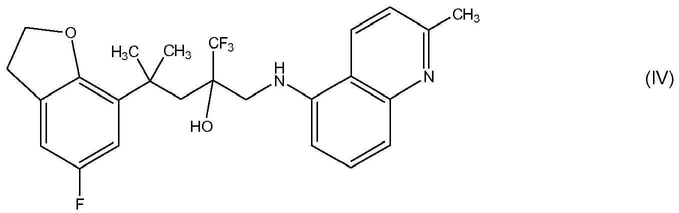 aufweist.

5. Ein DIGRA, ein pharmazeutisch zulässiges Salz davon oder ein pharmazeutisch zulässiger Ester davon, zur Verwendung in einem Medikament für die pharmakologische Begleitbehandlung die mit einer Glaukom-Filtrationsoperation in einem Subjekt einhergeht, wobei besagtes DIGRA, ein pharmazeutisch zulässiges Salz davon oder ein pharmazeutisch zulässiger Ester davon die Eigenschaft besitzt, die Produktion von TGF-β zu reduzieren oder zu unterdrücken, und wobei besagte pharmakologische Begleitbehandlung zu einem weiteren Funktionieren des Sickerkissens, welches während einer Glaukom-Filtrationsoperation in einem Subjekt geschaffen wird, führt, und wobei das DIGRA eine Verbindung mit der Formel II oder III umfasst 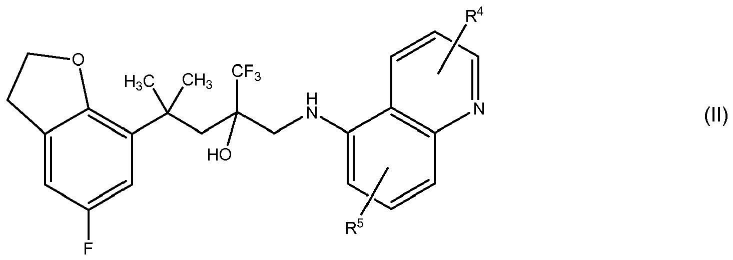 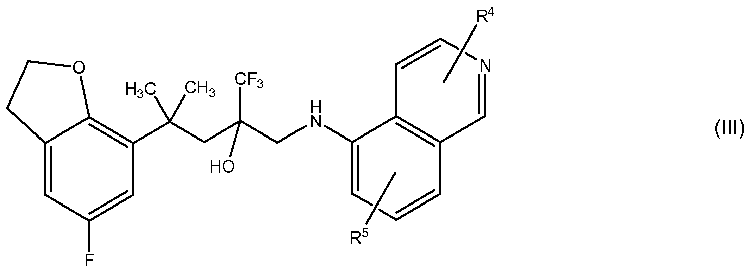 wobei R⁴ und R⁵ jeweils unabhängig voneinander ausgewählt sind aus der Gruppe bestehend aus Wasserstoff, Halogen, Cyano, Hydroxy, C₁-C₁₀ Alkoxy-Gruppen, unsubstituierten C₁-C₁₀ linearen oder verzweigten Alkyl-Gruppen, substituierten C₁-C₁₀ linearen oder verzweigten Alkyl-Gruppen, unsubstituierten C₃-C₁₀ zyklischen Alkyl-Gruppen und substituierten C₃-C₁₀ zyklischen Alkyl-Gruppen.

6. Das DIGRA zur Verwendung gemäß Anspruch 5, wobei besagte Produktion von besagtem TGF-β durch ein Chemokin oder Zytokin ausgewählt aus der Gruppe bestehend aus IL-1β, IL-4, IL-6, IL-9, IL-11, IL-13, IL-17, TNF-α, TGF-α, MCP-1, GM-CSF, PDGF, β-FGF, CTGF und Kombinationen davon stimuliert wird.

7. Das DIGRA zur Verwendung gemäß Anspruch 5, wobei das DIGRA die Formel IV 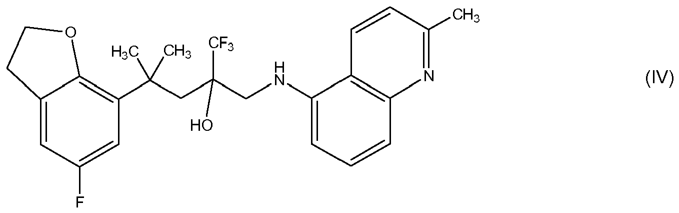 aufweist.

8. Das DIGRA zur Verwendung gemäß Anspruch 5, wobei das Medikament weiterhin einen Antagonisten zu TGF-β umfasst.

9. Das DIGRA zur Verwendung gemäß Anspruch 8, wobei das Medikament weiterhin einen NSAID umfasst.

10. Das DIGRA zur Verwendung gemäß Anspruch 5, wobei das Medikament weiterhin eine wirksame Menge eines anderen therapeutischen oder prophylaktischen Wirkstoffes umfasst, der in der Lage ist (a) einen Anstieg des Augeninnendrucks, (b) den Verlust von retinalen Ganglienzellen oder (c) beides zu behandeln, reduzieren oder zu verhindern; wobei besagter weiterer therapeutischer oder prophylaktischer Wirkstoff ausgewählt ist aus der Gruppe bestehend aus Physostigminsalicylat, Pilocarpinnitrat, kohlenstoffhaltigen Anhydrasehemmern, Prostaglandinanaloga, β-adrenergischen Antagonisten, muscarinisch-cholinergischen Wirkstoffen und Inhibitoren von Acetylcholinesterase.

11. Ein DIGRA oder ein pharmazeutisch zulässiges Salz oder Ester davon zur Verwendung bei der pharmakologischen Begleitbehandlung, die mit einer Glaukom-Filtrationsoperation in einem Subjekt einhergeht, wobei besagte Behandlung zu einem fortgesetzten Funktionieren des Sickerkissens in besagtem Subjekt nach besagter Filtrationsoperation führt, und wobei besagtes DIGRA die Formel IV 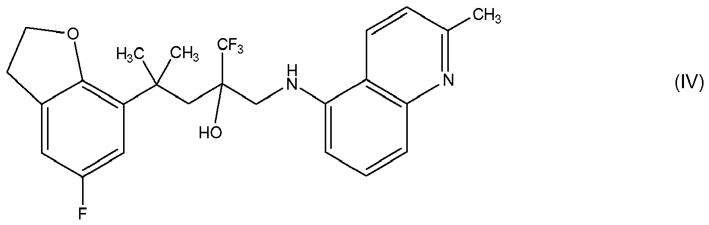 aufweist.

12. DIGRA oder ein pharmazeutisch zulässiges Salz oder Ester davon zur Verwendung in einem Medikament zur Kontrolle oder zur Verhütung von Glaukom in einem Subjekt, wobei besagtes DIGRA die Formel IV 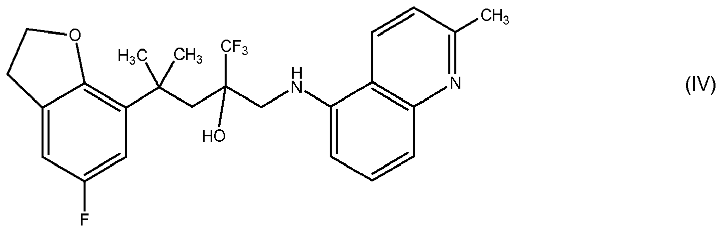 aufweist, und wobei besagtes Medikament in ein Auge eines Subjekts eingebracht wird, dessen Auge einer Glaukom-Filtrationsoperation unterzogen wurde, und wobei besagte Kontrolle oder Verhinderung besagten Fortschreitens die Folge eines fortgesetzten Funktionierens eines Sickerkissens ist.

13. Ein Verfahren zur Herstellung einer Zusammensetzung, das Verfahren umfassend:
(a) Bereitstellen eines DIGRA, eines pharmazeutisch zulässigen Salzes davon oder eines pharmazeutisch zulässigen Ester davon;
(b) Bereitstellen eines Antagonisten zu TGF-β; und
Kombinieren von: (i) besagtem DIGRA, pharmazeutisch zulässigem Salz davon oder einem pharmazeutisch zulässigem Ester davon; (ii) besagtem Antagonisten zu TGF-β; und (iii) einem pharmazeutisch wirksamen Träger zur Herstellung besagter Zusammensetzung, wobei das DIGRA eine Verbindung mit der Formel II oder III umfasst 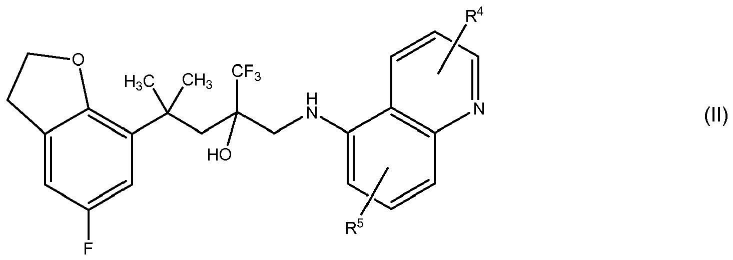 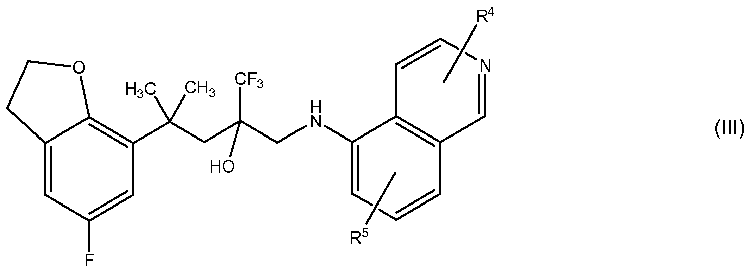 wobei R⁴ und R⁵ jeweils unabhängig voneinander ausgewählt sind aus der Gruppe bestehend aus Wasserstoff, Halogen, Cyano, Hydroxy, C₁-C₁₀ Alkoxy-Gruppen, unsubstituierten C₁-C₁₀ linearen oder verzweigten Alkyl-Gruppen, substituierten C₁-C₁₀ linearen oder verzweigten Alkyl-Gruppen, unsubstituierten C₃-C₁₀ zyklischen Alkyl-Gruppen und substituierten C₃-C₁₀ zyklischen Alkyl-Gruppen.

## Revendications

1. Composition comprenant un agoniste de récepteur de glucocorticoïde dissocié (« DIGRA »), un sel pharmaceutiquement acceptable de celui-ci ou un ester pharmaceutiquement acceptable de celui-ci en une quantité efficace pour réduire un risque de développement d'un non-fonctionnement, d'un mauvais fonctionnement ou d'une défaillance d'une bulle filtrante créée lors d'une chirurgie filtrante du glaucome chez un sujet, dans laquelle le DIGRA comprend un composé répondant à la Formule II ou III 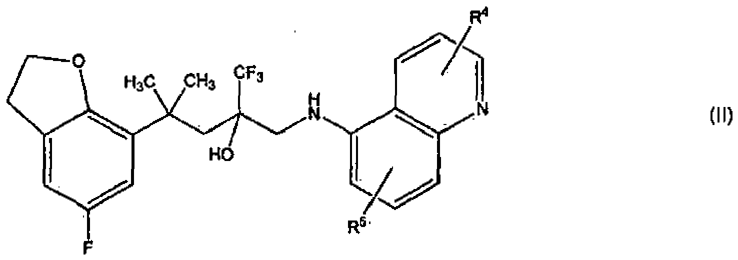 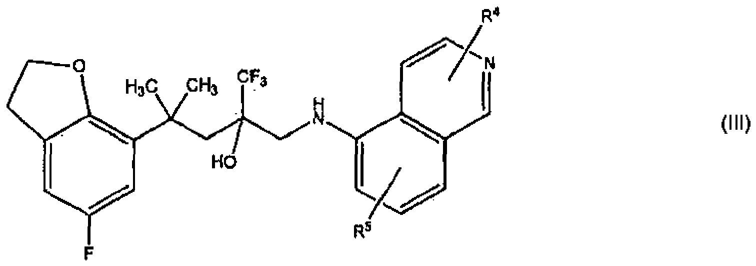 formules dans lesquelles R⁴ et R⁵ sont indépendamment choisis dans le groupe constitué de l'atome d'hydrogène, d'un atome d'halogène, d'un groupe cyano, d'un groupe hydroxy, des groupes alcoxy en C₁ à C₁₀, des groupes alkyles en C₁ à C₁₀ non substitués, linéaires ou ramifiés, des groupes alkyles en C₁ à C₁₀ substitués, linéaires ou ramifiés, des groupes alkyles cycliques en C₃ à C₁₀ non substitués et des groupes alkyles cycliques en C₃ à C₁₀ substitués ; et dans laquelle ladite composition comprend en outre un antagoniste d'au moins une isoforme de TGF-β, dans laquelle ledit antagoniste est choisi dans le groupe constitué des anticorps anti-TGF-β, du SB-431542, de l'AP 12009, de la prolactine, du sTGFRI et du sTGFRII.

2. Composition selon la revendication 1, dans laquelle ladite composition comprend en outre un AINS.

3. Composition selon la revendication 1, dans laquelle ledit glaucome est choisi dans le groupe constitué du glaucome simple primaire, du glaucome à angle fermé primaire, du glaucome simple secondaire, du glaucome à angle fermé secondaire, du glaucome pigmentaire, du glaucome néovasculaire, du glaucome du pseudophaque, du glaucome malin, du glaucome lié à une uvéite, du glaucome dû à une synéchie antérieure périphérique et de leurs combinaisons.

4. Composition selon la revendication 1, dans laquelle ledit DIGRA répond à la Formule IV

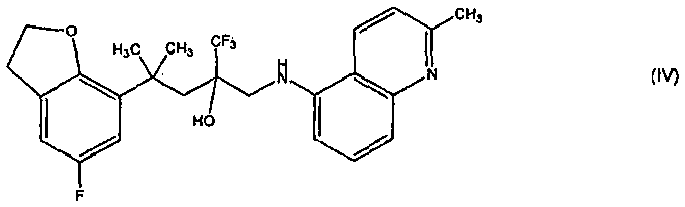

5. DIGRA, sel pharmaceutiquement acceptable de celui-ci ou ester pharmaceutiquement acceptable de celui-ci, pour l'utilisation dans un médicament destiné au traitement pharmacologique d'appoint associé à une chirurgie filtrante du glaucome chez un sujet, dans lequel ledit DIGRA, un sel pharmaceutiquement acceptable de celui-ci ou
un ester pharmaceutiquement acceptable de celui-ci a la propriété de réduire ou de supprimer la production d'un TGF-β, et dans lequel ledit traitement pharmacologique d'appoint conduit au fonctionnement continu d'une bulle filtrante créée chez ledit sujet lors de ladite chirurgie filtrante du glaucome et dans lequel le DIGRA comprend un composé répondant à la Formule II ou III 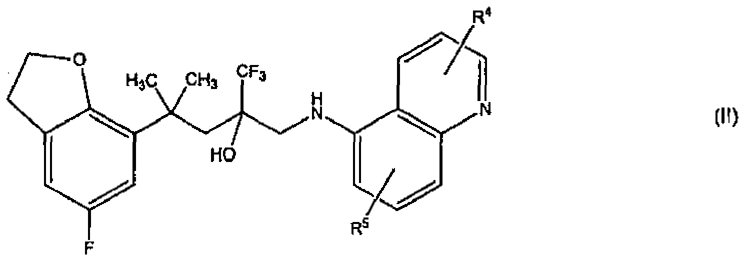 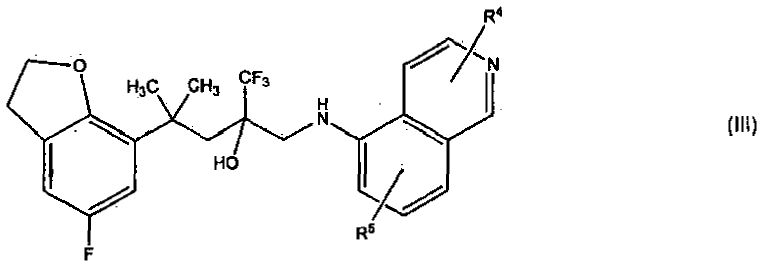 formules dans lesquelles R⁴ et R⁵ sont indépendamment choisis dans le groupe constitué de l'atome d'hydrogène, d'un atome d'halogène, d'un groupe cyano, d'un groupe hydroxy, des groupes alcoxy en C₁ à C₁₀, des groupes alkyles en C₁ à C₁₀ non substitués, linéaires ou ramifiés, des groupes alkyles en C₁ à C₁₀ substitués, linéaires ou ramifiés, des groupes alkyles cycliques en C₃ à C₁₀ non substitués et des groupes alkyles cycliques en C₃ à C₁₀ substitués.

6. DIGRA pour l'utilisation selon la revendication 5, dans lequel ladite production dudit TGF-β est stimulée par une chimiokine ou une cytokine choisie dans le groupe constitué de 1'IL-1β, de l'IL-4, de l'IL-6, de l'IL-9, de 1'IL-11, de l'IL-13, de l'IL-17, du TNF-α, du TGF-α, du MCP-1, du GM-CSF, du PDGF, du β-FGF, du CTGF et de leurs combinaisons.

7. DIGRA pour l'utilisation selon la revendication 5, dans lequel le DIGRA répond à la Formule IV

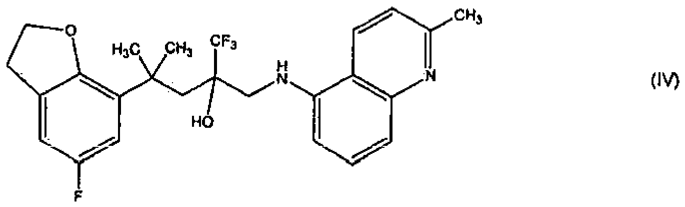

8. DIGRA pour l'utilisation selon la revendication 5, dans lequel le médicament comprend en outre un antagoniste de TGF-β.

9. DIGRA pour l'utilisation selon la revendication 8, dans lequel le médicament comprend en outre un AINS.

10. DIGRA pour l'utilisation selon la revendication 5, dans lequel le médicament comprend en outre une quantité efficace d'un autre agent thérapeutique ou prophylactique qui est capable de traiter, réduire ou prévenir (a) une augmentation de la pression intraoculaire, (b) la perte de cellules ganglionnaires de la rétine, ou (c) les deux ; dans lequel ledit autre agent thérapeutique ou prophylactique est choisi dans le groupe constitué du salicylate de physostigmine, du nitrate de pilocarpine, des inhibiteurs de l'anhydrase carbonique, des analogues de la prostaglandine, des antagonistes β-adrénergiques, des agents cholinergiques muscariniques et des inhibiteurs de l'acétylcholinestérase.

11. DIGRA ou sel ou ester pharmaceutiquement acceptable de celui-ci, pour l'utilisation dans le traitement pharmacologique d'appoint associé à une chirurgie filtrante du glaucome chez un sujet, dans lequel ledit traitement conduit au fonctionnement continu d'une bulle filtrante chez ledit sujet après ladite chirurgie filtrante, et dans lequel ledit DIGRA répond à la Formule IV

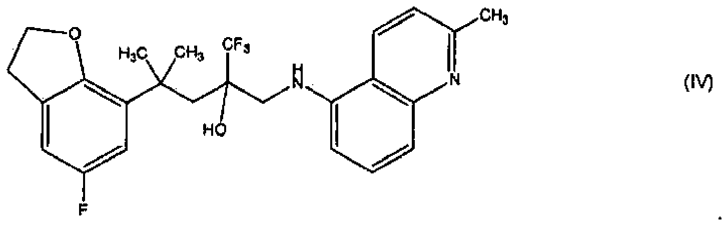

12. DIGRA ou sel ou ester pharmaceutiquement acceptable de celui-ci, pour l'utilisation dans un médicament destiné à contrôler ou à prévenir la progression d'un glaucome chez un sujet, dans lequel ledit DIGRA répond à la Formule IV 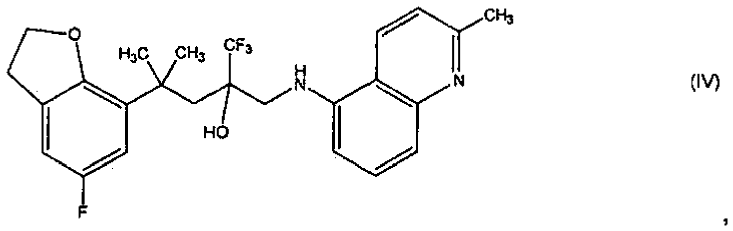 et dans lequel ledit médicament est administré dans un oeil dudit sujet, lequel oeil a subi une chirurgie filtrante du glaucome, et dans lequel ledit contrôle ou ladite prévention de ladite progression résulte du fonctionnement continu d'une bulle filtrante.

13. Procédé de fabrication d'une composition, le procédé comprenant :
(a) la fourniture d'un DIGRA, d'un sel pharmaceutiquement acceptable de celui-ci ou d'un ester pharmaceutiquement acceptable de celui-ci ;
(b) la fourniture d'un antagoniste de TGF-β; et
la combinaison : (i) dudit DIGRA, sel pharmaceutiquement acceptable de celui-ci ou ester pharmaceutiquement acceptable de celui-ci ; (ii) dudit antagoniste de TGF-β ; et (iii) d'un vecteur pharmaceutiquement acceptable pour produire ladite composition, dans lequel le DIGRA comprend un composé répondant à la Formule II ou III 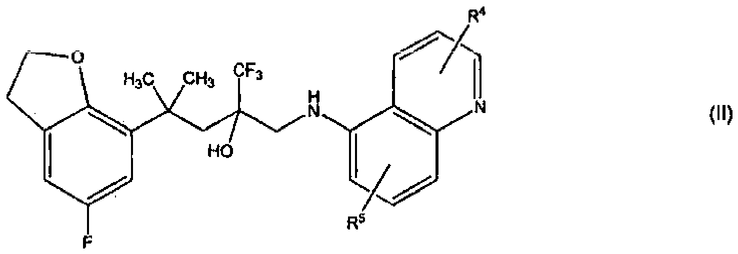 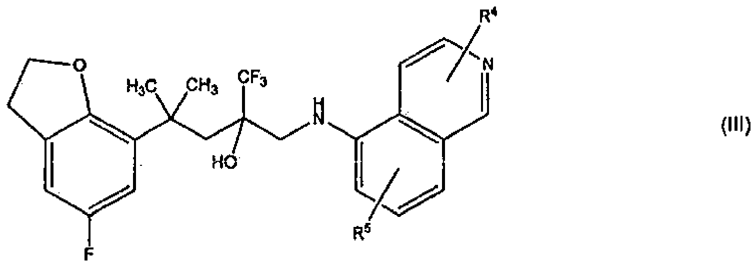 formules dans lesquelles R⁴ et R⁵ sont indépendamment choisis dans le groupe constitué de l'atome d'hydrogène, d'un atome d'halogène, d'un groupe cyano, d'un groupe hydroxy, des groupes alcoxy en C₁ à C₁₀, des groupes alkyles en C₁ à C₁₀ non substitués, linéaires ou ramifiés, des groupes alkyles en C₁ à C₁₀ substitués, linéaires ou ramifiés, des groupes alkyles cycliques en C₃ à C₁₀ non substitués et des groupes alkyles cycliques en C₃ à C₁₀ substitués.
